# EUROPÄISCHE PATENTSCHRIFT

(11) **EP 0 620 283 B1**
(45) Veröffentlichungstag und Bekanntmachung des Hinweises auf die Patenterteilung: **16.09.1998**
(21) Anmeldenummer: 94105141.9
(22) Anmeldetag: 31.03.1994
(51) Int. Cl.: C12Q 1/32, C12Q 1/26, C12Q 1/54, C07D 487/04, C07D 513/04

(54) **Verfahren zur kolorimetrischen Bestimmung eines Analyten mit einer PQQ-abhängigen Dehydrogenase**
Method for colourimetric determination of an analyte by using a PQQ-dependent dehydrogenase
Méthode pour la détermination colorimétrique d'un analyte utilisant une déhydrogénase PQQ-dépendante

(30) Priorität: 08.04.1993 DE 4311464
(43) Veröffentlichungstag der Anmeldung: 19.10.1994
(73) Patentinhaber: BOEHRINGER MANNHEIM GMBH, 68298 Mannheim (DE)
(72) Erfinder: Hoenes, Joachim, Dr., D-64673 Zwingenberg (DE); Unkrig, Volker, Dr., D-68526 Ladenburg (DE)

(56) Entgegenhaltungen:
- EP-A- 0 354 441
- EP-A- 0 441 222
- WO-A-92/07953
- DE-A- 2 147 466
- CHEMICAL ABSTRACTS, vol. 117, no. 1, 6. Juli 1992, Columbus, Ohio, US; abstract no. 3829x, IKONEN, VEIJO OLAVI ET AL 'Process and reagent combinations for the colorimetric determination of the threshold value of oxo or hydroxyl group-containing analytes, and the use of these reagent combinations' & FI-A-84 625 (IKONEN, VEIJO OLAVI;MULTANEN, VELI MATTI; PELTTARI, SIRPA MARJA)
- AGRIC. BIOL. CHEM., 52(8), 2081-2, 1988 Adachi, Osao et al 'Enzymic determination of pyrroloquinoline quinone with a quinoprotein glycerol dehydrogenase'

## Beschreibung

Die Erfindung betrifft ein Verfahren zur kolorimetrischen Bestimmung eines Analyten mittels enzymatischer Oxidation mit einer PQQ-abhängigen Dehydrogenase in Gegenwart eines Elektronenakzeptors aus der Gruppe der elektronenreichen aromatischen Nitrosoverbindungen und Bestimmung des reduzierten Elektronenakzeptors durch Farbbildung als Maß für die Menge des Analyten. Weiter betrifft die Erfindung ein Mittel zur kolorimetrischen Bestimmung eines Analyten durch enzymatische Oxidation, enthaltend eine PQQ-abhängige Dehydrogenase, eine elektronenreiche aromatische Nitrosoverbindung sowie ein farbbildendes nichtoxidatives Nachweisreagenz für die aus der elektronenreichen, aromatischen Nitrosoverbindung bei Reduktion entstehende Iminoverbindung. Weiter betrifft die Erfindung neue Nitrosoanilinverbindungen, deren Herstellung, sowie deren Verwendung zur kolorimetrischen enzymatischen Bestimmung eines Analyten.

Enzymatische Oxidationen ermöglichen in der Analytik den Nachweis und die Bestimmung von Substanzen in verschiedensten Probenmaterialien. Hierbei wirkt ein oxidierendes Enzym in Gegenwart eines die Elektronen der Oxidationsreaktion aufnehmenden Akzeptors auf ein entsprechendes Enzymsubstrat ein. Die Reduktion des Elektronenakzeptors zeigt die Anwesenheit des Enzymsubstrats an. Hierbei hat es sich bisher als besonders vorteilhaft erwiesen, wenn der reduzierte Elektronenakzeptor durch Farbbildung nachgewiesen werden kann, da dies nicht notwendigerweise nur mittels teurer Meßapparaturen möglich ist, sondern gegebenenfalls auch visuell erfolgen kann.

Bekannte Methoden zur kolorimetrischen Bestimmung von Substanzen mittels oxidierend wirkender Enzyme verwenden Oxidasen oder Dehydrogenasen. Beide Enzymgruppen gehören zur Hauptgruppe der Oxidoreduktasen (Römpps Chemielexikon, Francksche Verlagsbuchhandlung, Stuttgart, 8. Auflage, 1985, Band 4, Seite 2952; Lexikon Biochemie, Herausgeber H. D. Jakubke, Verlag Chemie, Weinheim, 2. Auflage, 1981, Seite 194), deren Mitglieder nach ihren natürlichen Elektronenakzeptoren unterschieden werden können.

Natürlicher Elektronenakzeptor für Oxidasen ist molekularer Sauerstoff (Römpps Chemielexikon, Francksche Verlagsbuchhandlung, Stuttgart, 8. Auflage, 1985, Band 4, Seite 2946). Bei Analytbestimungen wird dabei der Analyt durch eine Oxidase und O₂ oxidiert. Das entstehende H₂O₂ wird mit Hilfe von Peroxidase zur Oxidation eines Leukofarbstoffes benutzt. Stellvertretend für den Stand der Technik des Einsatzes von Oxidasen zur kolorimetrischen Bestimmung von Analyten sei A. Kunst et al. in "Methods in enzymatic analysis", Hrsg. H. U. Bergmeyer, Verlag Chemie, Weinheim, 3. Auflage, 1984, Band 6, Seite 178 - 185 zitiert. Glucose wird dort in Serum, Plasma oder in deproteinisiertem Blut durch Umsetzung mit Glucoseoxidase und Luftsauerstoff in wässriger Lösung nachgewiesen, indem das bei dieser Reaktion durch Reduktion des Sauerstoffs gebildete Wasserstoffperoxid in Gegenwart von Peroxidase reduziert wird und damit farbbildend auf ebenfalls in der Reaktionsmischung vorliegendes Phenol und 4-Aminophenazon wirkt. Das hohe Redoxpotential von H₂O₂ und die Unselektivität und Instabilität von Peroxidase führen meist zu Einschränkungen solcher Tests. Störend wirken zum Beispiel Übergangsmetallionen oder Häm oder Hämproteine, wie sie leicht in von Blut abgeleiteten Proben auftreten können, weil sie Wasserstoffperoxid zersetzen. Probeninhaltsstoffe, zum Beispiel Bilirubin oder auch Medikamente wie zum Beispiel Methyldopa,die durchaus in von Blut abgeleiteten Proben oder in Urin vorkommen, können mit Wasserstoffperoxid und Peroxidase zu einer Farbbildung und somit zu falschen Resultaten führen; sie können aber auch schon gebildeten Farbstoff wieder reduzieren und damit entfärben.

Insbesondere bei Durchführung der vorgenannten Bestimmungsmethode auf festen Trägern, in sogenannten Trockentests, hat sich der Sauerstoffbedarf der Oxidasen zusätzlich als nachteilig erwiesen. Vor allem dann, wenn viel Sauerstoff zur Oxidation hoher Konzentrationen an Enzymsubstrat gebraucht wird, kann die Diffusion von Sauerstoff aus der Luft in das Reaktionsmedium zum geschwindigkeitsbestimmenden Schritt werden und zu langen Reaktionszeiten oder insbesondere bei kinetischen Bestimmungsmethoden zu falschen Ergebnissen führen.

Dehydrogenasen können im allgemeinen in solche unterteilt werden, die für die Oxidation von Enzymsubstraten Nikotinamid-adenin-dinucleotid (NAD) oder Nikotinamid-adenin - dinucleotid -phosphat (NADP) als natürlichen unmittelbaren Elektronenakzeptor benötigen und in solche, die nicht-NAD oder -NADP-abhängig sind und die also andere Substanzen als natürliche, unmittelbare Elektronenakzeptoren bei enzymatischen Oxidationsreaktionen verwenden. Unter die Gruppe der nicht-NAD- oder -NADP-abhängigen Dehydrogenasen fallen insbesondere die PQQ- und flavin-abhängigen Dehydrogenasen.

Der Einsatz von NAD-abhängigen Dehydrogenasen für kolorimetrische Messungen ist zum Beispiel aus DE-A-2147466 bekannt. Dort wird beschrieben, daS Lactat unter Katalyse von Lactatdehydrogenase mit Nikotinamid-adenin-dinucleotid zu Pyruvat und reduziertem Nikotinamid-adenin-dinucleotid umgesetzt wird. Das gebildete NADH reagiert dann beispielsweise in Gegenwart des Enzyms Diaphorase mit Tetrazoliumsalzen unter Bildung von NAD und farbigen Formazanen, deren Konzentration photometrisch bestimmt werden kann. Anstatt Diaphorase ist auch N-Methylphenazinium-methosulfat als Reduktionskatalysator für die Übertragung der Elektronen von NADH auf das Tetrazoliumsalz genannt.

Nachteile dieses Verfahrens sind darin zu sehen, daß statt NADH auch andere eventuell in biologischen Proben, wie zum Beispiel Blut, Serum, Plasma oder Urin vorkommende, reduzierend wirkende Substanzen, wie Glutathion oder Medikamente wie Methyldopa oder Dobesylat in Gegenwart von unspezifischen Reduktionskatalysatoren, wie Diaphorase oder N-Methylphenazinium-methosulfat Tetrazoliumsalze in entsprechende Formazane überführen und so zu falsch-positiven Ergebnissen führen, obwohl sie in Abwesenheit von Reduktioskatalysatoren nicht so schnell mit Tetrazoliumsalzen reagieren würden.

Aus WO 92/07953 ist die Verwendung von nicht-NAD-abhängigen, PQQ-abhängigen Dehydrogenasen für kolorimetrische Bestimmungsverfahren bekannt, wobei die Reduktion eines künstlichen Elektronenakzeptors, wie z.B. Dichlorphenolindophenol, Phenazinmethosulfat oder Nitrotetrazolblau anhand einer Farbveränderung beobachtet wird.

Aus EP-A-O 3 54 441 ist der oxidative, enzymatische Nachweis von Analyten mittels flavinabhängiger Oxidasen oder nicht-NAD-abhängiger Dehydrogenasen, wie PQQ-abhängigen Dehydrogenasen, mit elektronenreichen aromatischen Nitrosoverbindungen bekannt. Bei dieser Nachweismethode wird die aromatische Nitrosoverbindung enzymatisch zu einem entsprechenden elektronenreichen, aromatischen Amin reduziert, das entweder mittels einer ausgefällten, schwer löslichen Heteropolysäure durch Heteropolyblaubildung nachgewiesen wird oder mit einem Kupplungsreagenz in Gegenwart eines Oxidationsmittels zu einem Farbstoff gekuppelt wird. Die blau-grauen Farbabstufungen der Heteropolyblaubildung sind für eine genaue visuelle Auswertung allerdings wenig geeignet. Zum kolorimetrischen Nachweis der im allgemeinen nicht oder nur sehr schwach gefärbten aromatischen Amine mit einem farbgebenden Kupplungsreagenz ist nachteilig, daß zusätzlich ein Oxidationsmittel benötigt wird. Da ein solches Oxidationsmittel die enzymatische Reduktion der aromatischen Nitrosoverbindung zum aromatischen Amin stören würde, muß die Nachweisreaktion in zwei getrennten Stufen erfolgen: in der ersten Stufe wird die aromatische Nitrosoverbindung enzymatisch zum aromatischen Amin reduziert und in einer davon getrennten zweiten Stufe ein Oxidationsmittel für die oxidative Kupplung des aromatischen Amins mit einem farbgebenden Kupplungsreagenz zugegeben.

Ein weiterer Nachteil dieses Nachweisverfahrens besteht darin, daß für die Reduktion jedes Äquivalentes einer aromatischen Nitrosoverbindung zu einem aromatischen Amin zwei Äquivalente des Analyten oxidiert werden müssen. Dies kann insbesondere bei geringen Analytkonzentrationen zu einer unbefriedigenden Empfindlichkeit eines solchen Nachweisverfahrens führen.

Aufgabe der vorliegenden Erfindung war es daher, die vorgenannten Nachteile des Standes der Technik zu beseitigen und ein einfacheres, weniger störanfälliges, empfindlicheres und optisch besser auswertbares Verfahren und Mittel zum oxidativen Nachweis von Analyten zur Verfügung zu stellen, das insbesondere in einer Stufe durchgeführt werden kann und über den gesamten sichtbaren Wellenlängenbereich visuell gut auswertbare Farben liefert.

Die Aufgabe wird gelöst durch die Erfindung wie sie in den Ansprüchen charakterisiert ist.

Demgemäß wurde ein Verfahren zur kolorimetrischen Bestimmung eines Analyten mittels enzymatischer Oxidation des Analyten mit einer Oxidoreduktase in Gegenwart eines direkten Elektronenakzeptors aus der Gruppe der elektronenreichen aromatischen Nitrosoverbindungen gefunden, bei dem die Bestimmung des reduzierten Elektronenakzeptors durch Farbbildung als Maß für die Menge des Analyten erfolgt, das dadurch gekennzeichnet ist, daß die elektronenreiche aromatische Nitrosoverbindung unter Oxidation des Analyten in Anwesenheit einer PQQ-abhängigen Dehydrogenase zu einer Iminoverbindung reduziert wird und diese ohne enzymatische Weiterreduktion zum aromatischen Amin durch Farbbildung nachgewiesen wird.

Insbesondere ist Gegenstand der Erfindung ein Verfahren zur kolorimetrischen Bestimmung eines Analyten mit einer Oxidoreduktase in Gegenwart eines direkten Elektronenakzeptors aus der Gruppe der elektronenreichen aromatischen Nitroso-verbindungen, bei dem die Bestimmung des reduzierten Elektronenakzeptors durch Farbbildung als Maß für die Menge des Analyten erfolgt, das dadurch gekennzeichnet ist, daß die elektronenreiche aromatische Nitrosoverbindung unter Oxidation des Analyten in Anwesenheit einer PQQ-abhängigen Dehydrogenase zu einer Iminoverbindung reduziert wird und diese statt einer enzymatischen Weiterreduktion zum aromatischen Amin durch Reaktion mit einem farbgebenden nicht-oxidativen Nachweisreagenz kolorimetrisch bestimmt wird.

Außerdem wurde ein Mittel zur kolorimetrischen Bestimmung eines Analyten durch enzymatische Oxidation des Analyten enthaltend eine PQQ-abhängige Dehydrogenase und einen direkten Elektronenakzeptor aus der Gruppe der elektronenreichen, aromatischen Nitrosoverbindungen gefunden, das dadurch gekennzeichnet ist, daß es entweder eine elektronenreiche aromatische Nitrosoverbindung enthält, die bei enzymatischer Reduktion eine farbige Iminoverbindung bildet oder daß es weiterhin ein farbgebendes nichtoxidatives Nachweisreagenz für die aus der elektronenreichen aromatischen Nitrosoverbindung durch Reduktion entstehende Iminoverbindung enthält.

Erfindungsgemäß wird unter "Analyt" eine solche Substanz verstanden, die enzymatisch oxidiert wird. In vielen Fällen wird der Analyt diejenige Substanz sein, die in der zu untersuchenden Probe direkt nachgewiesen oder quantitativ bestimmt werden soll. Beispielsweise kann Glucose direkt mit PQQ-abhängiger Glucosedehydrogenase (Glucose-dye-oxidoreduktase) oxidiert und kolorimetrisch bestimmt werden. Es ist jedoch auch möglich, daß der Analyt erst durch eine oder mehrere vorgeschaltete Reaktionen aus einer anderen Substanz gebildet wird, sodaß durch kolorimetrische Bestimmung des Analyten indirekt auf die Konzentration der Ausgangssubstanz geschlossen werden kann.

Der Analyt ist in der vorliegenden Erfindung diejenige Substanz, die als Substrat der eingesetzten PQQ-abhängigen Dehydrogenase akzeptiert wird.

PQQ-abhängige Dehydrogenasen enthalten als Cofaktor Pyrrolchinolinchinon. Eine Übersicht über solche "Chinoproteine" gibt J. A. Jongejahn et al. in "PQQ and Quinoproteins", Kluver Academic Publ. Dordrecht, Niederlande 1989. Beispiele für erfindungsgemäß einsetzbare Enzyme sind PQQ-abhängige Glucosedehydrogenase (Glucose-dye-oxidoreductase, E.C 1.1.1.50/1.1.1.91/1.1.1.97), Alkoholdehydrogenase oder Lactatdehydrogenase. In dem erfindungsgemäßen Verfahren kann insbesondere die PQQ-abhängige Glucosedehydrogenase vorteilhaft für die kolorimetrische Bestimmung von Glucose eingesetzt werden.

Als direkte Elektronenakzeptoren, die die Elektronen von dem Enzym/Cofaktorsystem Dehydrogenase/PQQ übernehmen, werden elektronenreiche aromatische Nitrosoverbindungen eingesetzt, wie sie als Elektronenakzeptoren für Oxidasen und nicht-NADH-abhängige Dehydrogenasen aus EP-A-0 354 441 und EP-A-O 441 222 bekannt sind und bei denen eine Nitrosogruppe unmittelbar an einen elektronenreichen aromatischen Kern gebunden ist. "Direkte" Elektronenakzeptoren heißt, daß die Elektronen enzymkatalysiert direkt, ohne die Notwendigkeit eines Reduktionskatalysators, vom Enzym/Cofaktorsystem übernommen werden.

Im erfindungsgemäßen Sinne als reduzierbare elektronenreiche aromatische Nitrosoverbindungen einsetzbare Verbindungen sind solche, die die Elektronen, die bei der Oxidation des der eingesetzten PQQ-abhängigen Dehydrogenase entsprechenden Substrates anfallen, von dem Enzym übernehmen und dabei Iminoverbindungen bilden. Eine entsprechende Iminoverbindung enthält eine Iminogruppe = NH, die über ihre Doppelbindung an den vormals aromatischen Kern gebunden ist und mit dessen Doppelbindungselektronen in Konjugation steht. Elektronenreiche aromatische Nitrosoverbindungen enthalten einen oder mehrere elektronenliefernde Reste oder Gruppen am oder im aromatischen Kern, die die Bildung einer Iminoverbindung begünstigen, indem sie durch Elektronenabgabe über den vormals aromatischen Kern mit der Iminogruppe in Konjugation treten.

Dies sind zum einen Substituenten, die einen +M Effekt auf den aromatischen Kern ausüben.

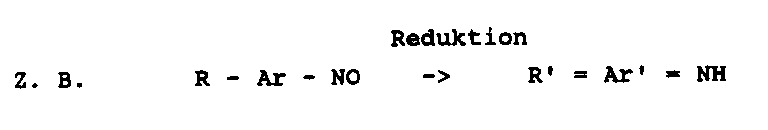

Beispiele für an einen aromatischen Kern gebundene Reste R mit +M-Effekt sind Substituenten wie Hydroxy-, Alkoxy-, Aryloxy-, Alkylthio-, Arylthio-, Amino-, Monoalkylamino-, Monoarylamino-, Dialkylamino- und Diarylaminoreste.

Im Fall von Nitrosobenzolderivaten zeigen diese Substituenten beispielsweise dann besonders ihre Wirkung, wenn sie in ortho und/oder in para-Position zur Nitrosogruppe stehen.

Gruppen mit elektronenliefernden und eine Iminstruktur begünstigenden Effekt können auch Bestandteil eines heteroaromatischen Ringsystems sein.

Beispiele sind heterocylische aromatische Nitrosoverbindungen mit einem π-Elektronenüberschuß, in denen das aromatische Ringsystem so elektronenreich ist, daß ein externer +M-Substituent für die Ausbildung einer mesomeriestabilisierten Imingruppe nach der Reduktion entbehrlich ist. Der π-Elektronenüberschuß resultiert daraus, daß mehr aromatische π-Elektronen vorhanden sind als Ringatome, über die sich die π-Elektronen verteilen können. Derartige Heterocyklen sind dem Fachmann bekannt.

Beispiele für solche aromatischen Heterocyclen sind Antipyrin, Pyrazolo-Heterocyclen und Pyrazole.

Der Elektronenreichtum der erfindungsgemäß eingesetzten elektronenreichen aromatischen Nitrosoverbindungen hat den zusätzlichen Effekt, daß diese Nitrosoverbindungen zu einem gewissen Teil über Keto-Enol-Tautomerie mit der äquivalenten Oximgrenzstruktor im Gleichgewicht stehen. $\text{R - Ar - N = O ↔ R' = Ar' = N - OH}$

Beide Tautomeriegrenzstrukturen sollen im Rahmen der Erfindung mit dem Begriff "aromatische Nitrosoverbindungen" als erfindungsgemäß einsetzbare Substanzen umfaßt sein.

Bei den vorstehend genannten +M-Substituenten sind Alkoxy-, Alkylthio-, Monoalkylamino- und Dialkylaminoreste Reste, in denen Alkyl einen Kohlenwasserstoffrest mit 1 bis 6 Kohlenstoffatomen darstellt, der seinerseits durch eine Hydroxygruppe, eine gegebenenfalls ein- oder mehrfach durch Alkyl mit 1 bis 6 Kohlenstoffatomen substituierte Aminogruppe, PO₃H₂, SO₃H oder CO₂H substituiert sein kann. Die Säurereste PO₃H₂, SO₃H und CO₂H können als solche oder in Salzform als Ammonium-, Alkali- oder Erdalkalisalze vorliegen.

Aryloxy- und Arylthioreste enthalten aromatische Reste mit 6 bis 10 Kohlenstoffatomen, wobei Phenoxy- und Phenylthioreste besonders bevorzugt sind.

Ammoniumsalze sind solche, die das Ammoniumion NH₄+ enthalten oder solche, die ein- oder mehrfach durch Alkyl-, Aryl- oder Aralkylreste substituierte Ammoniumkationen enthalten. Alkyl in Alkyl- und Aralkylresten bedeutet einen Kohlenwasserstoffrest mit 1 bis 6 Kohlenstoffatomen. Aryl in Aryl- und Aralkylresten ist ein 6 bis 10 Kohlenstoffatome zählendes aromatisches Ringsystem, wobei Phenyl bevorzugt ist. Ein bevorzugter Aralkylrest ist Benzyl.

Alkalisalze sind vorzugsweise solche des Lithiums, Natriums oder Kaliums. Erdalkalisalze sind vorzugsweise solche des Magnesiums oder Calciums.

Die im vorstehenden angegebenen Beispiele für +M-Substituenten sollen nicht als vollständige Aufzählung verstanden werden. Der Fachmann wird im Einzelfall wissen, ob ein gegebener Rest R ein +M-Substituent ist und an welcher Stelle er an einem aromatischen System diese Wirkung ausübt, oder welche aromatische Heterozyklen im Ringsystem eine entsprechende Gruppierung enthalten und insofern sollen alle diese Reste mögliche Substituenten in aromatischen Nitrosoverbindungen sein, die gemäß der vorliegenden Erfindung eingesetzt werden können.

Das aromatische Grundgerüst der einzusetzenden aromatischen Nitrosoverbindung stellt bevorzugt einen elektronenreichen aromatischen Ring von 5 - 7, besonders bevorzugt 5 - 6 Kohlenstoff- oder Heteroatomen dar, der mit einem oder zwei aromatischen oder/und alicyclischen Ringen anelliert sein kann. Für die aromatischen anellierten Ringe kommen dabei sowohl kohlenstoffaromatische Systeme als auch heteroaromatische Systeme mit je 5 - 7 bevorzugt 5 - 6 Kohlenstoff- oder Heteroatomen in Frage.

Unter alicyclischen Ringen werden gesättigte oder ungesättigte Cycloaliphaten mit 5 - 7 Kohlenstoffatomen oder Heteroatomen, vorzugsweise 5 oder 6 Kohlenstoffatomen verstanden. Unter Heteroatomen wird Stickstoff, Sauerstoff oder Schwefel verstanden.

Bevorzugte, erfindungsgemäß einsetzbare Nitrosoverbindungen sind Nitrosobenzolderivate.

Unter Nitrosobenzolderivaten wird auch Nitrosobenzol verstanden, das mit einem oder mehreren aromatischen oder/und alicyclischen Ringen anelliert ist. Als aromatische Ringe kommen hierbei sowohl kohlenstoffaromatische Systeme als auch Heteroaromaten mit je 5 - 7, bevorzugt 5 oder 6 Ringatomen in Frage. Beispiele sind anellierte Benzol- oder Naphthalinringe oder ein anellierter Pyridinring.

Besonders bevorzugt sind Nitrosobenzolderivate der allgemeinen Formel I 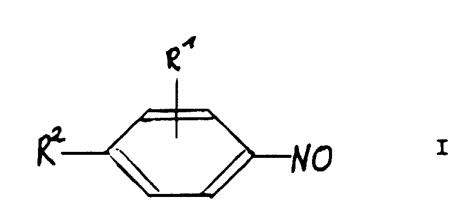
wobei R¹
Wasserstoff, Hydroxy, Alkyl gegebenenfalls substituiert durch Hydroxy COOH, PO₃H₂, oder SO₃H, Alkoxy, Alkylthio, Aryloxy, Arylthio, Halogen, oder Amino, das gegebenenfalls ein oder mehrfach durch Alkyl, gegebenenfalls substituiert durch Hydroxy, PO₃H₂, Dialkylphosphinyl, SO₃H oder CO₂H, substituiert ist, bedeutet
und R²
eine Hydroxygruppe, , Alkoxy-, , Aryloxy-, Arylthio- oder Alkylthiogruppe bedeutet, wobei der Alkylrest gegebenenfalls seinerseits durch eine Hydroxygruppe, eine Alkoxygruppe, eine gegebenenfalls ein- oder mehrfach durch Alkyl substitutierte Aminogruppe, PO₃H₂, SO₃H oder CO₂H als solche oder in Salzform als Ammonium-, Alkali- oder Erdalkalisalze substituiert ist, oder eine Aminogruppe NR³R⁴ bedeutet,
in der R³ und R⁴ gleich oder verschieden sein können, und Wasserstoff, eine Aryl- oder Alkylgruppe, die ihrerseits durch eine Hydroxy-, Alkoxy-, Hydroxyalkoxy-, eine gegebenenfalls hydroxysubstituierte Polyalkoxygruppe, PO₃H₂, SO₃H, COOH als solche oder in Salzform oder durch eine gegebenenfalls ein oder mehrfach durch Alkyl substituierte Aminogruppe substituiert sein kann, bedeutet, oder
in der R³ und R⁴ einen Alkylenrest darstellen können, der gegebenenfalls durch Sauerstoff, Schwefel oder Stickstoff unterbrochen ist, wobei Stickstoff durch einen Alkyl-, Hydroxyalkyl-, Hydroxyalkoxyalkyl-, Alkoxyhydroxyalkyl-, Alkoxycarbonylalkyl-, Dioxanylyl-alkyl- oder Polyalkoxyalkylrest substituiert ist, der seinerseits jeweils gegebenenfalls im Alkylteil durch einen Hydroxyrest substituiert sein kann, oder
wenn R¹ in ortho-Stellung zu NR³R⁴ steht, R³ oder R⁴ auch zusammen mit R¹ einen Alkylenrest darstellen kann.

Hierbei bedeutet Halogen Fluor, Chlor, Brom oder Jod. Fluor und Chlor sind besonders bevorzugt.

Alkyl in Alkyl, Alkoxy oder Alkylthio bedeutet einen Kohlenwasserstoffrest mit 1 - 6 Kohlenstoffatomen, Reste mit 1 - 3 Kohlenstoffatomen sind besonders bevorzugt. Die für Alkyl vorstehend gegebene Definition trifft auch für den Alkylteil in Hydroxyalkyl-, Dialkylaminoalkyl-, Hydroxyalkoxyalkyl-, Alkoxyalkyl-, Polyalkoxyalkyl-, Alkoxy-hydroxyalkyl- und Dioxanylyl-alkylresten zu. Ein Dioxanylyl-alkylrest ist ein Rest, bei dem ein Dioxanringsystem an einen Alkylrest gebunden ist. Vorzugsweise handelt es sich um ein 1,4-Dioxanringsystem, d. h.

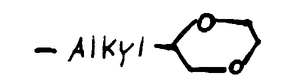

Ein Polyalkoxyalkylrest ist ein Rest
-Alkyl-(Alkoxy)ₙ -Alkoxy
mit n = 1 - 10. Bevorzugt ist n = 1 - 4. Besonders bevorzugt ist n = 1 - 3. Ein Alkylenrest ist eine geradkettige oder verzweigte - vorzugsweise geradkettige -, gesättigte oder ungesättigte - vorzugsweise gesättigte-, Kohlenwasserstoffkette aus 2 - 5, vorzugsweise 2 - 4 C-Atomen, mit zwei freien Bindungsstellen.

Aryl in Aryl- und Aralkylresten ist ein 6 bis 10 Kohlenstoffatome zählendes aromatisches Ringsystem, wobei Phenyl bevorzugt ist.

Ammoniumsalze sind solche, die das Ammoniumion NH₄+ enthalten oder solche, die ein- oder mehrfach durch Alkyl-, Aryl- oder Aralkylreste substituierte Ammoniumkationen enthalten.

Alkalisalze sind vorzugsweise solche des Lithiums, Natriums oder Kaliums. Erdalkalisalze sind vorzugsweise solche des Magnesiums oder Calciums.

Bevorzugte Reste R¹ sind Wasserstoffund Alkyl, insbesondere Wasserstoff.

Bevorzugte Reste R² sind Alkoxyreste und die Aminogruppe NR³R⁴.

In der Bedeutung eines von R¹ und R³ durch Sauerstoff; Schwefel oder Stickstoff unterbrochenen Alkylenrestes ist der durch Einbeziehung des Stickstoffatomes der allgemeinen Formel I gebildete Morpholin- baw. Thiomorpholin- bzw. Piperazinrest bevorzugt. Der Piperazinrest ist besonders bevorzugt.

In der Bedeutung eines von R¹ und R³ gebildeten Alkylenrestes ist der durch Einbeziehung des aromatischen Ringes der allgemeinen Formel I gebildete Indolin- oder 1,2,3,4-Tetrahydrochinolinrest bevorzugt.

Als Salz eines erfindungsgemäßen Nitrosoanilinderivates der allgemeinen Formel I werden insbesondere solche starker Säuren, insbesondere Mineralsäuren, wie Salzsäure, Schwefelsäure, Salpetersäure, Phosphorsäure bevorzugt. Ganz besonders bevorzugt sind Hydrochloride, das sind Salze der Salzsäure.

Bevorzugte Nitrosoverbindungen der allgemeinen Formel I sind:
N,N'- Bis-(2-hydroxyethyl)-p-nitrosoanilin
N,N'- Dimethyl -p-nitrosoanilin
N,N'- Diethyl -p-nitrosoanilin
N-Methyl-N'-(4-nitrosophenyl)-piperazin
N-(2-hydroxyethyl)-5-nitrosoindolin
2,4-Dimethoxy-nitrosobenzol
N,N'-Bis-(2-methoxyethyl)-4-nitrosoanilin
N-(4-Nitrosophenyl)-morpholin
N-(2,2-diethoxy-ethyl)N'-(4-nitrosophenyl)-piperazin
p-Nitrosophenol
3-Methoxy-4-nitrosophenol

Unter den elektronenreichen heteroaromatischen Nitrosoverbindungen, deren aromatisches Ringsystem so elektronenreich ist, daß ein externer +M-Substituent für die Nitroso-/Oxim-Tautomerie und für die Iminbildung entbehrlich ist, sind besonders mit einer Nitrosogruppe substituierte Pyrazolone, Pyrazole und insbesondere nitrososubstituierte Pyrazoloverbindungen, wie sie zum Beispiel in Ullmann's Enzyclopedia of Industrial Chemistry 5th ed., Vol A 20, Seite 72 bis 74, beschrieben sind, im erfindungsgemäßen Verfahren geeignet. Bevorzugt sind dabei die 3-Nitrosopyrazolo-Verbindungen der allgemeinen Formel II.

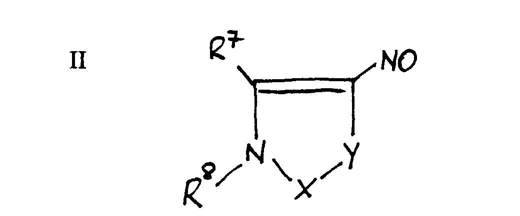

Bekannt sind diese Verbindungen zum großen Teil als Vorstufe zur präparativen Synthese der entsprechenden 3-Aminopyrazoloverbindungen aus der Europäischen Patentanmeldung EP-A-0 433 854. In der Formel II bedeutet:
- X-Y: NR⁵-CO oder N=CR⁶
- R⁵: Wasserstoff, Alkyl gegebenenfalls substituiert durch Hydroxy, Carboxy, SO₃H, PO₃H₂, Dialkylphosphinyl
- R⁶: Wasserstoff, Alkyl, Alkenyl, Alkoxy, Alkylthio, Aryl, Arylthio, Aralkyl, gegebenenfalls jeweils substituiert durch Hydroxy, Dialkylphosphinyl, Carboxy, SO₃H, PO₃H₂, ein Salz eines dieser Säurereste oder/und Alkoxycarbonyl; oder
Amino, das gegebenenfalls durch einen oder zwei gegebenenfalls einen oder mehrere Hydroxy-, Carboxy- oder/und Alkoxycarbonylreste tragende Alkylreste substituiert ist, wobei, wenn Amino durch 2 Alkylreste substituiert ist, diese Reste auch zu einem Ring geschlossen sein können, der außer durch das N-Atom der Aminogruppe gegebenenfalls auch durch Sauerstoff; Schwefel oder ein weiteres Stickstoffatom unterbrochen sein kann, oder Amino, das gegebenenfalls durch ein oder zwei Acylgruppen, Alkoxy- oder/und Aralkoxycarbonylgruppen, H₂ N-CO, Alkyl-, Aralkyl oder/und Arylcarbamoylgruppen substituiert ist; oder Wasserstoff Carboxy, Alkoxycarbonyl, Carboxamido oder Halogen, und
- R⁷: Alkyl, Thioalkyl oder Aralkyl, gegebenenfalls substituiert durch Hydroxy, Carboxy, SO₃H oder PO₃H₂ oder Amino, das gegebenenfalls durch eine oder zwei Alkylgruppen substituiert ist, die wiederum durch Hydroxy, Carboxy, SO₃H, Dialkylphosphinyl oder PO₃H₂ substituiert sein können, wobei mindestens R⁶ und/ oder R⁷ eine Aminogruppe darstellt, und
- R⁸: eine Alkyl oder Aralkylgruppe, die gegebenenfalls durch Hydroxy, Carboxy, SO₃H oder PO₃H₂ substituiert sein kann
oder wobei
- R⁷ und R⁸: zusammen eine gesättigte oder ungesättigte Kette mit 3 oder 4 Gliedern aus Stickstoffatomen oder aus Kohlenstoffatomen und gegebenenfalls einem oder mehreren Stickstoff- oder Schwefelatomen, wobei Kohlenstoffatome gegebenenfalls substituiert sind durch Alkyl, Alkoxy, Alkylthio, Hydroxy, Aralkyl, Aryl, Carboxy, Carboxamido, Alkoxycarbonyl, Cyano, Halogen, Amino, das gegebenenfalls durch einen oder zwei gegebenenfalls einen oder mehrere Hydroxy-, Carboxy- oder/und Alkoxycarbonylreste tragende Alkylreste substituiert ist, und wobei Stickstoffatome, die nicht über eine Doppelbindung gebunden sind, durch Alkyl oder Aralkyl gegebenenfalls durch Hydroxy, SO₃H, PO₃H₂, Carboxy oder Dialkylphosphinyl substituiert, substituiert sind oder zwei benachbarte Kettensubstituenten gegebenenfalls eine Alkylengruppe bilden, die ihrerseits gegebenenfalls mit Aryl substituiert oder anelliert ist
sowie gegebenenfalls entsprechende tautomere Formen und deren Salze

Hierbei bedeutet "Alkyl" - auch in Alkylthio-, Dialkyl-phosphinyl-, Alkylcarbamoyl- und Aralkylresten - einen geradkettigen oder verzweigten Alkylrest mit 1-6, vorzugsweise 1-4 C-Atomen. Beispiele sind die Methyl-, Ethyl-, Propyl-, iso-Butyl- oder tert.-Butylgruppe.

Wenn eine Aminogrupe durch 2 Alkylreste substituiert ist, können diese Reste auch so zu einem Ring geschlossen sein, daß sie insgesamt einen durch ein Stickstoffatom unterbrochenen Ring darstellen. Bevorzugt sind hierbei solche Aminogruppen, die einen insgesamt 5- oder 6-gliedrigen Ring darstellen und der seinerseits gegebenenfalls durch Sauerstoff, Schwefel oder Stickstoff unterbrochen ist. Besonders bevorzugt ist der Morpholinorest.

"Alkoxy" - auch in Alkoxy- und Aralkoxycarbonylresten - steht für einen geradkettigen oder verzweigten Alkoxyrest mit 1 - 6, vorzugsweise 1 - 4 C-Atomen. Beispiele sind die Methoxy-, Ethoxy-, Propyloxy-, iso-Butyloxy- oder tert.-Butyloxygruppe.

"Aryl" - auch in Arylcarbamoylgruppen - bezeichnet einen Kohlenstoffaromaten- oder Heteroaromatenrest, vorzugsweise einen solchen mit 6 - 10 Ring-Atomen, insbesondere die Phenyl- und die Naphthylgruppe, die zusätzlich noch durch Alkyl, Alkoxy oder/und Halogen substituiert sein können. Besonders bevorzugt ist der Phenylrest.

Ein "Aralkyl"-Rest - auch in einer Aralkylcarbamoylgruppe -bedeutet einen Rest, bei dem eine wie vorstehend definierte Alkylgruppe durch einen wie zuvor charakterisierten Arylrest substituiert ist. Bevorzugt ist die Benzylgruppe.

Ein "Aralkoxy"-Rest, beispielsweise in Aralkoxycarbonylgruppen, bezeichnet einen Rest, bei dem eine wie vorstehend definierte Alkoxygruppe durch einen wie zuvor charakterisierten Arylrest substituiert ist. Bevorzugt ist die Benzyloxygruppe.

"Halogen" steht für die Reste Fluor, Chlor, Brom und Jod. Fluor und Chlor sind bevorzugt.

Eine Acylgruppe bezeichnet einen Carbonsäurerest, der Alkyl-, Aralkyl- oder Arylreste enthalten kann. Bevorzugt sind Acetyl-, Phenylacetyl- oder Benzoylreste.

Unter einer Alkylengruppe wird eine geradkettige oder verzweigte, gesättigte oder ungesättigte Kohlenwasserstoffkette aus 3 - 5, vorzugsweise 3 oder 4 C-Atomen mit zwei freien Bindungsstellen verstanden.

Beispiele sind -CH₂-CH=CH-, 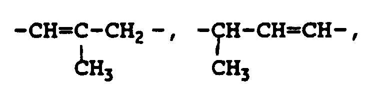 -(CH₂)₄- oder -CH=CH-CH=CH-.

Bevorzugt sind der Butadiendiylrest (-CH=CH-CH=CH-) und der Tetramethylenrest (-(CH₂)₄-).

Ein Alkenylrest ist ein geradkettiger oder verzweigter Kohlenwaserstoffrest aus 2-5 C-Atomen mit mindestens einer Doppelbindung. Bevorzugt ist beispielsweise der Vinylrest. Unter einer Dialkylphosphinylgruppe wird der Rest 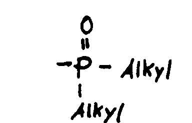 verstanden, wobei Alkyl die zuvor gegebene Bedeutung hat. Bevorzugt ist der Dimethylphosphinylrest.

Als Salze von SO₃H, PO₃H₂ und Carboxyresten können Alkali- oder Erdalkalisalze oder Ammoniumsalze eingesetzt werden. Unter Alkalisalzen werden Lithium-, Natrium-, Kalium-, Rubidium- und Cäsiumsalze verstanden, wobei Lithium-, Natrium- und Kaliumsalze, vor allem aber Natrium- und Kaliumsalze bevorzugt sind. Erdalkalisalze sind solche des Berylliums, Magnesiums, Calciums, Strontiums oder Bariums. Bevorzugt sind Magnesium- und Calciumsalze, wobei Calciumsalze besonders bevorzugt sind. Als Ammoniumsalze können solche des unsubstituierten Ammoniumions, NH₄⁺, Verwendung finden. Es ist aber auch möglich, solche Ammoniumsalze einzusetzen, bei denen das Ammoniumion durch 1 - 4 Alkyl-, Aryl- oder Aralkylreste substituiert ist. Für diese Reste gelten die zuvor gegebenen Definitionen, wobei als Alkylrest Methyl, Ethyl und n-Propyl, als Arylrest die Phenylgruppe und als Aralkylrest die Benzylgruppe besonders bevorzugt sind.

Als Carboxamidorest wird der Rest CONH₂ verstanden, aber auch solche Reste, bei denen die Aminogruppe durch ein oder zwei gegebenenfalls einen oder mehrere Hydroxy-, Carboxy- oder/und Alkoxycarbonylreste tragende Alkylreste substituiert ist.

In den erfindungsgemäß eingesetzten Nitroso-Verbindungen der allgemeinen Formel II sind solche Verbindungen bevorzugt, in denen R⁷ und R⁸ eine gesättigte oder ungesättigte Kette wie oben beschrieben bildet. Besonders bevorzugt ist dabei, wenn diese Kette ungesättigt ist und Doppelbindungselektronen und freie Stickstoffelektronenpaare der ungesättigten Kette in Konjugation zu der Doppelbindung oder zu dem Brücken-N-Atom der allgemeinen Formel II stehen, so daß ein anellierter aromatischer Ring resultiert.

Gegebenenfalls sind für eine Substanz der allgemeinen Formel II auch tautomere Formen möglich. Diese sollen ebenfalls als von der allgemeinen Formel II umfaßt gelten.

Erfindungsgemäß bevorzugt sind Nitrosoverbindungen der allgemeinen Formeln III bis XII. 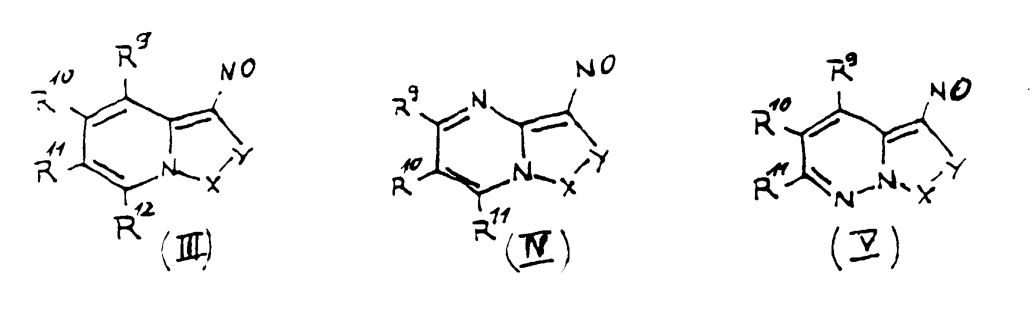 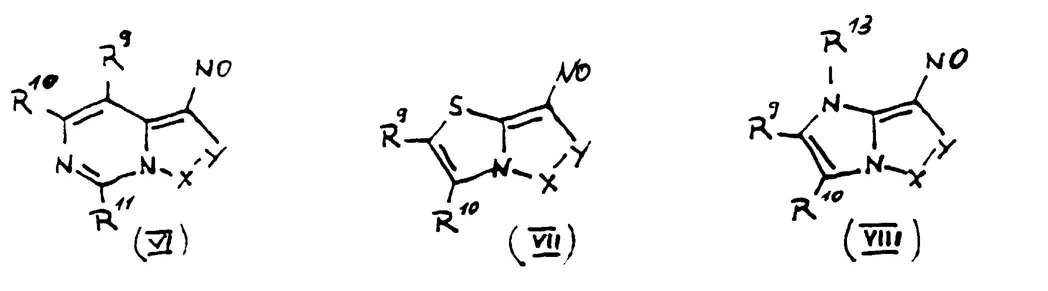 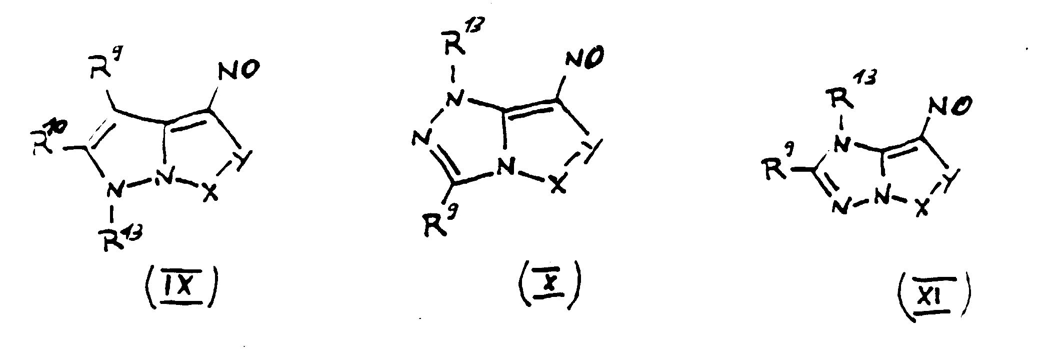 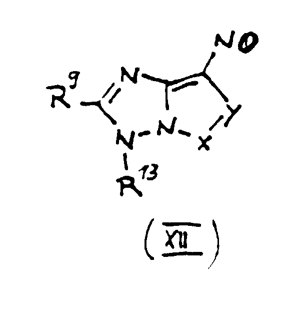 sowie gegebenenfalls entsprechende tautomere Formen und deren Salze.

Hierbei hat X-Y die gleiche Bedeutung wie zuvor beschrieben. R⁹, R¹⁰, R¹¹ und R¹², die gleich oder verschieden sind, für Wasserstoff, Hydroxy, Alkyl, Alkoxy, Alkylthio, Aralkyl, Aryl, Carboxy, Alkoxycarbonyl, Carboxamido, Cyano, Amino, das gegebenenfalls durch ein oder zwei gegebenenfalls einen oder mehrere Hydroxy-, Carboxy- oder/und Alkoxycarbonylreste tragende Alkylreste substituiert ist oder Halogen, wobei zwei benachbarte Reste gegebenenfalls eine Alkylengruppe bilden, die ihrerseits gegebenenfalls mit Aryl substituiert oder anelliert ist und R¹³ Alkyl oder Aralkyl darstellt, das gegebenenfalls durch Hydroxy, Carboxy, SO₃H, PO₃H₂ oder Dialkylphosphinyl substituiert sein kann. Die Definitionen der Reste entsprechen den für die Substanzen der allgemeinen Formel II gegebenen.

Besonders bevorzugt für die erfindungsgemäße Verwendung sind Substanzen der allgemeinen Formeln III, IV, V, VII, VIII und IX, gegebenenfalls entsprechende tautomere Formen und deren Salze. Ganz besonders bevorzugt sind solche Substanzen, in denen X-Y die Bedeutung N=CR⁶ hat, wobei R⁶ die Bedeutung haben kann wie für die allgemeine Formel II angegeben.

Als hervorragend geeignet für die erfindungsgemäße Verwendung haben sich insbesondere die Verbindungen 3-Nitroso-2-methyl-pyrazolo-[1.5a]-pyridin, 3-Nitroso-pyrazolo-[1.5a]-pyridin und 3-Nitrosopyrazolo [3.2-c]-s-triazol und deren Salze, insbesondere das Hydrochlorid erwiesen.

Wie vorstehend ausgeführt, werden für das erfindungsgemäße Verfahren aromatische Nitrosoverbindungen mit der zu untersuchenden Probe und der PQQ-abhängigen Dehydrogenase in Kontakt gebracht. In EP-A-0 354 441 und EP-A-0 441 222 wird beschrieben, daß Oxidasen und nicht-NAD-abhängige Dehydrogenasen aromatische Nitrosoverbindungen zu elektronenreichen aromatischen Aminoverbindungen reduzieren, die wiederum mit einem farbgebenden Kupplungsreagenz und einem Oxidationsmittel durch oxidative Kupplung nachgewiesen werden.

Überraschenderweise hat es sich herausgestellt, daß in Anwesenheit eines nichtoxidativen farbbildenden Nachweisreagenzes für chinoide Iminoverbindungen und einer PQQ-abhängigen Dehydrogenase, eine aromatische Nitrosoverbindung unter Analytoxidation nicht die vollständige, durch eine PQQ-abhängige Dehydrogenase katalysierte enzymatische Reduktion zum elektronenreichen aromatischen Amin durchläuft, sondern daß eine nach einer teilweisen enzymatischen Reduktion entstehende Iminozwischenstufe durch das nicht-oxidative farbbildende Nachweisreagenz abgefangen und nachgewiesen werden kann.

Noch überraschender war, daß wenn bestimmte aromatische Nitrosoverbindungen gewählt werden, die schon einen chromogenen Rest tragen, diese aromatischen Nitrosoverbindungen in Gegenwart einer PQQ-abhängigen Dehydrogenase zwar zur farbigen Iminoverbindung reduziert werden, die enzymatische Weiterreduktion zum aromatischen Amin im wesentlichen aber unterbleibt, so daß auch die farbige Iminoverbindung selbst ohne die Notwendigkeit der Reaktion mit einem farbgebenden Nachweisreagenz als Maß für die Menge des Analyten bestimmt werden kann. Beispiele für entsprechende erfindungsgemäß einsetzbare chromogene Nitrosoverbindungen werden unten gegeben.

Obwohl die Bandbreite der geeigneten elektronenreichen aromatischen Nitrosoverbindungen sehr groß ist, werden sie alle von PQQ-abhängigen Dehydrogenasen als direkte Elektronenakzeptoren akzeptiert. Essentiell scheint lediglich die Nitrosogruppe zu sein, die an einen elektronenreichen aromatischen Rest gebunden ist.

Es wird vermutet, daß die Reaktion nach folgendem Reaktionsschema am Beispiel des Nitrosoanilins dargestellt werden kann:

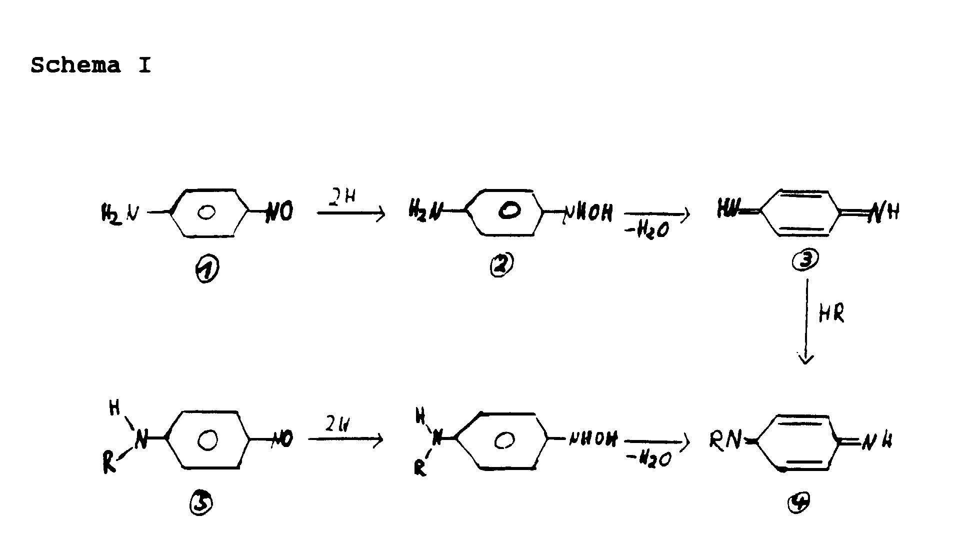

Die aromatische Nitrosoverbindung (1) wird unter Oxidation des Analyten zum aromatischen Hydroxylamin (2) reduziert, dieses spaltet spontan Wasser ab, wodurch eine chinoide Iminozwischenstufe (3) entsteht. Bevor diese weiter enzymatisch durch die PQQ-abhängige Dehydrogenase zum aromatischen Amin reduziert wird, wie es aus EP-A-03 54 441 und EP-A-0 441 222 bekannt ist, kann sie beispielsweise durch ein Kupplungsreagenz (HR) abgefangen und als farbiges Kupplungsprodukt (4) kolorimetrisch bestimmt werden oder sie enthält den chromogenen Rest des Kupplungsproduktes schon in der Ausgangsverbindung (5) und kann so nach enzymatischer Reduktion direkt als farbige Iminoverbindung (4) kolorimetrisch bestimmt werden.

Überraschend ist, daß spezifisch bei der Verwendung von PQQ-abhängigen Dehydrogenasen die Iminozwischenstufe (3) erfindungsgemäß mit einem Nachweisreagenz abgefangen oder direkt nachgewiesen werden kann, ohne daß die weitere aus EP-A-0 354 441 bekannte enzymatische Reduktion zum Amin erfolgt. Denn bei Verwendung von flavinabhängigen Oxidasen, die gemäß EP-A-0 354 441 ebenfalls aromatische Nitrosoverbindungen reduzieren, gelingt ein Nachweis der Iminozwischenstufe nur zu einem geringen Teil, während der größte Teil der Iminozwischenstufe unter Analytoxidation sehr schnell enzymatisch zum aromatischen Amin weiterreduziert wird.

Erfindungsgemäß kann der Nachweis der Iminoverbindung über ihre Eigenfarbe erfolgen.

Erfindungsgemäß kann der Nachweis der chinoiden Iminoverbindung auch mit einem farbgebenden nicht-oxidativen Reagenz erfolgen. Farbgebend heißt, daß durch Reaktion des farbbildenden Reagenzes mit der Iminoverbindung eine farbige Substanz erhalten wird, deren Absorptionsmaximum verschieden ist von dem der eingesetzten Nitrosoverbindung und des farbbildenden Reagenzes vor der Reaktion. Unter einem nicht-oxidativen farbgebenden Nachweisreagenz wird verstanden, daß das Nachweisreagenz mit einer nachzuweisenden Verbindung unter Farbbildung reagiert, ohne daß das Nachweisreagenz auf die nachzuweisende Verbindung oxidierend wirkt, (z. B. durch ein im Nachweisreagenz enthaltendes zusätzliches Oxidationsmittel).

Der Nachweis der in dem erfindungsgemäßen Verfahren entstehenden Iminoverbindung kann durch Reaktion eines farbbildenden Kupplungsreagenzes mit der Iminoverbindung erfolgen. Dabei wird eine farbige Substanz erhalten, die die Iminoverbindung als Teilstruktur enthält. Der Nachweis kann aber auch so erfolgen, daß die Iminoverbindung ein Leukofarbstoffmolekül zu einem farbigen Molekül oxidiert.

Möglich ist auch die Kombination beider Nachweisprinzipien, indem ein Kupplungsreagenz mit einem Molekül der nachzuweisenden Iminoverbindung zu einem nicht-farbigen Leukofarbstoffmolekül kuppelt, das durch ein weiteres Molekül der Iminoverbindung zu einem Farbstoff oxidiert wird.

Solch farbgebende Nachweisreagenzien für Iminoverbindungen sind in großer Vielzahl bekannt. Prinzipiell kommen beispielsweise alle Substanzen in Betracht, die mit oxidierten p-Phenylendiaminderivaten unter Farbbildung reagieren.

Beispiele für farbgebende Kupplungsnachweisreagenzien sind aromatische Verbindungen, vorzugsweise Phenol- und Naphtholverbindungen, die mit guten Abgangsgruppen, substituiert sind und damit diese Abgangsgruppen leicht und sehr schnell unter Farbbildung durch die nachzuweisende Iminoverbindung substituieren können. Bevorzugte Beispiele sind 1-Naphthol-4-sulfonsäure, 2,4,6-Tribrom-3-hydroxybenzoesäure, 2,4-Dichlor-1-naphthol, Tatrazin oder Orange 1.

In dem erfindungsgemäßen Verfahren sind zum Nachweis der Iminoverbindung auch Nachweisreagenzien einsetzbar, die nicht mit der Iminoverbindung kuppeln, sondern die unter Reduktion der Iminoverbindung zum Amin eine farbige Verbindung bilden. Häufig geschieht dies unter Dimerisierung des Leukofarbstoffes. Geeignete Leukofarbstoffe sind dem Fachmann aus Nachweisverfahren für H₂O₂ oder Peroxidase bekannt. Beispiele für solche Leukofarbstoffe sind 1-Naphthylaminosulfonsäuren, Triarylimidazole (DE-OS 2735690) Diarylimidazole (US-P-4,919,890), Aminocarbazole (DE-OS 2205733, 2338932) Oxazole, Thiazole (EP-A-0 167 973) u.a.

Geeignet sind alle Leukofarbstoffe, die aufgrund ihres Redoxpotentials in der Lage sind, Iminoverbindungen zu reduzieren.

Schließlich lassen sich die Nachweisprinzipien der Kupplung mit einem Kupplungsreagenz und der Oxidation eines Leukofarbstoffes kombinieren, indem als Kupplungskomponente für Iminoverbindungen ein Reagenz eingesetzt wird, das mit der Iminoverbindung eine farblose Leukoverbindung bildet, die erst durch ein zweites Molekül der Iminoverbindung unter dessen Reduktion zum entsprechenden aromatischen Amin zum Farbstoff oxidiert wird. Die gebräuchlichsten, aus der Farbfotografie sehr gut bekannten sogenannten "Kuppler" sind Phenole, Naphthole, Aniline, Naphthylamine sowie deren Derivate und methylenaktive Verbindungen. Eine Übersicht über diese Art von Kupplern gibt T. H. James in "The Theory of the Photographic Process" 3rd ed., Mc Millan, New York, 1966, Chapter 17 auf den Seiten 385 - 390.

Das erfindungsgemäße Verfahren wird so durchgeführt, daß die zu untersuchende Probe mit einer PQQ-abhängigen Dehydrogenase, einer oder mehreren der vorstehend beschriebenen elektronenreichen aromatischen Nitrosoverbindungen und einem Nachweisreagenz für Iminoverbindungen gleichzeitig kontaktiert wird. Enthält die Probe einen Analyten, der von der PQQ-abhängigen Dehydrogenase oxidiert wird, reagiert die aromatische Nitrosoverbindung unter enzymatischer Reduktion zur entsprechenden Iminoverbindung. Diese reagiert mit dem farbbildenden Nachweisreagenz, so daß die entstehende Farbe mit der Konzentration des Analyten in der Probe korreliert werden kann. Die Farbmessung kann direkt visuell durch Vergleichsfarben oder photometrisch geschehen.

Ein besonders einfaches Verfahren zum oxidativen Nachweis von Analyten liegt dann vor, wenn die durch Reduktion der aromatischen Nitrosoverbindung entstehende Iminoverbindung selbst farbig ist und nicht erst durch Kupplung mit einem farbgebenden Nachweisreagenz oder durch Oxidation eines Leukofarbstoffes nachgewiesen werden muß. Farbige Iminoverbindungen sind an sich wie oben beschrieben dem Fachmann aus der Farbfotographie und aus analytischen Nachweisverfahren bekannt.

Für das erfindungsgemäße Verfahren zum oxidativen Nachweis eines Analyten durch direkte Messung einer farbigen chinoiden Iminoverbindung haben sich Nitrosoanilinverbindungen der Formel XIII als direkte chromogene Elektronenakzeptoren als besonders geeignet erwiesen, die zu einem farbigen Chinondiimin der Formel XIV reduziert werden. 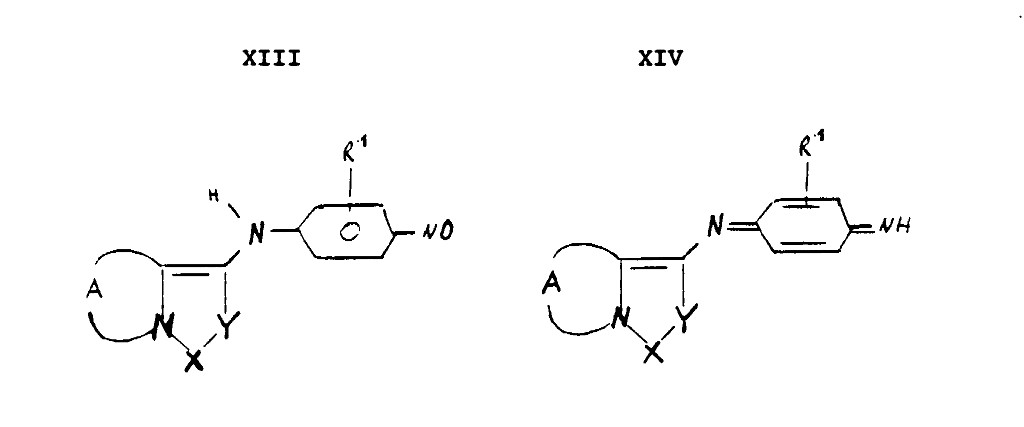
R¹ in der allgemeinen Formel XIII bedeutet Wasserstoff, Hydroxy, Halogen, Alkoxy, Alkylthio, Aryloxy oder Arylthio, Alkyl gegebenenfalls substituiert durch Hydroxy, Carboxy, PO₃H₂ oder SO₃H, Amino gegebenenfalls ein- oder mehrfach substituiert durch Alkyl, das wiederum gegebenenfalls durch Hydroxy, PO₃H₂, Dialkylphosphinyl, SO₃H oder Carboxy substituiert sein kann
X-Y bedeutet NR⁵-CO oder N=CR⁶
   wobei R⁵ und R⁶ die gleiche Bedeutung hat wie in der allgemeinen Formel II und
A bedeutet eine gesättigte oder ungesättigte Kette aus drei Gliedern mit einem Stickstoff oder Schwefelatom und zwei Kohlenstoffatomen oder zwei Stickstoffatomen und einem Kohlenstoffatom darstellt, wobei Kohlenstoffatome gegebenenfalls substituiert sind durch Alkyl, Alkoxy, Alkylthio, Hydroxy, Aralkyl, Aryl, Carboxy, Carboxamido, Alkoxycarbonyl, Cyano, Halogen, Amino, das gegebenenfalls durch einen oder zwei, gegebenenfalls einen oder mehrere Hydroxy-, Carboxy-, oder/und Alkoxycarbonylreste tragende Alkylreste substituiert ist, und wobei Stickstoffatome, die nicht über eine Doppelbindung gebunden sind, durch Wasserstoff, Alkyl, gegebenenfalls substituiert durch SO₃H, PO₃H₂, Carboxy oder Dialkylphosphinyl, oder durch Aralkyl substituiert sind oder zwei benachbarte Kettensubstituenten gegebenenfalls eine Alkylengruppe bilden, die ihrerseits gegebenenfalls mit Aryl substituiert oder anelliert ist,
sowie gegebenenfalls entsprechende tautomere Formen und deren Salze.

Die Bedeutung der einzelnen Substituenten ist dabei die gleiche wie in den Verbindungen mit der allgemeinen Formel II.

Bevorzugte Reste für R¹ sind dabei Wasserstoff und Alkyl und für X-Y die Gruppe N=CR⁶

Besonders bevorzugt bildet A mit dem benachbarten Heterocyclus einen Imidazol, Triazol- Benzimidazol-, Thiazol- oder Dihydroimidazol-Ring, dessen Kohlenstoffatome die in der allgemeinen Definition von A gegebenen Substituenten tragen können. Ganz besonders bevorzugt ist dabei der Imidazolring.

Besonders bevorzugte Verbindungen sind:
1) (2,4-Dimethyl-pyrazolo-[1.5a]-imidazol-3 -yl)-(4-nitrosophenyl)-amin
2) (4-Methyl-pyrazolo-[1.5a]-imidazol-3-yl)-(4-nitroso-phenyl)-amin
3) (4-(Dimethylphosphinylmethyl)-2-methyl-pyrazolo-[1.5a]-imidazol-3 -yl)-(4-nitrosophenylamin)
5) (5,6 Dihydro-4-dimethylphosphinylmethyl-2 methyl-pyrazolo-[1.5a]imidazol-3-yl)-(4-nitrosophenyl)-amin
6) (4-(Dimethylphosphinylmethyl-2-methyl-pyrazolo[1.5a]imidazol-3-yl)-(4-nitrosophenyl)-amin
7) (2,6 Dimethyl-4-dimethylphosphinylmethyl-pyrazolo-[3.2c]-s-triazol-3-yl)-(4-nitrosophenyl)-amin

Wird das erfindungsgemäße Verfahren so durchgeführt, daß durch Reduktion der aromatischen Nitrosoverbindung direkt eine farbige Chinondiiminverbindung der Formel XIV entsteht, so wird die zu untersuchende Probe mit einer PQQ-abhängigen Dehydrogenase und einer oder mehreren elektronenreichen aromatischen Nitrosoverbindungen der allgemeinen Formel XIII gleichzeitig kontaktiert. Enthält die Probe einen Analyten, der von PQQ-abhängigen Dehydrogenasen oxidiert wird, reagiert die elektronenreiche aromatische Nitrosoverbindung zum entsprechenden farbigen Chinondiimin. Der zusätzliche Elektronenreichtum des an den chromogenen elektronenreichen Pyrazolring ankondensierten Ringes A bewirkt, daß überraschenderweise die gebildete Chinondiiminverbindung nicht mehr weiter enzymatisch zum entsprechenden Amin reduziert wird, sondern daß die farbige Chinondiiminverbindung direkt in Form einer Lichtabsorptionsmessung gemessen und mit der Konzentration des Analyten in der Probe korreliert werden kann. Um eine schnelle enzymatische Reduktion der chromogenen aromatischen Nitrosoverbindungen zu erzielen, ist es besonders vorteilhaft, wenn diese ausreichend gut löslich sind. Besonders vorteilhaft für das erfindungsgemäße Verfahren beim Einsatz von chromogenen Nitrosoaromaten der Formel XIII ist es, wenn deren Konzentration in Lösung mindestens 10⁻³ mol/l, bevorzugt, mindestens 10⁻² mol/l, beträgt.

Eine gute Löslichkeit kann insbesondere dadurch erreicht werden, daß die chromogene Nitrosoverbindung mit hydrophilen Gruppen versehen wird.

Entsprechend ausnehmend gut im erfindungsgemäßen Verfahren geeignete chromogene Nitrosoverbindungen sind beispielsweise die vorstehend genannten Verbindungen 3,4 und 7.

Das Verfahren kann in einem sogenannten Naßtest, zum Beispiel einer Küvette oder als sogenannter Trockentest auf einem entsprechenden Reagenzträger durchgeführt werden, wobei die notwendigen Testreagenzien auf einem Testträger insbesondere einem saugfähigen oder quellbaren Material vorliegen. Solche Testträger sind zum Beispiel aus DE-A-3 247 608, EP-A-0 262 445 oder EP-A-0 256 806 bekannt.

Ein weiterer Gegenstand der Erfindung ist ein Mittel zur kolorimetrischen Bestimmung eines Analyten durch enzymatische Oxidation, wie es in den Ansprüchen gekennzeichnet ist. Ein solches Mittel enthält neben der für enzymatische Oxidation des zu bestimmenden Analyten notwendigen PQQ-abhängigen Dehydrogenase und neben mindestens einer aromatischen Nitrosoverbindung als direktem Elektronenakzeptor, der die bei der enzymatischen Oxidation des Analyten freiwerdenden Elektronen von dem PQQ/Dehydrogenase-System übernimmt, weiterhin zusätzlich ein farberzeugendes nichtoxidatives Nachweisreagenz für Iminoverbindungen.

Als PQQ-Dehydrogenasen, aromatische Nitrosoverbindungen und farbbildende Nachweisreagenzien für chinoide Iminoverbindungen werden die vorstehend für das erfindungsgemäße Verfahren beschriebenen Stoffe eingesetzt. Werden in den erfindungsgemäßen Verfahren elektronenreiche aromatische Nitrosoverbindungen der Formel XIII eingesetzt, die einen am Anilinstickstoff gebundenen chromogenen Rest schon enthalten, so enthält das Mittel vorteilhafterweise kein farberzeugendes Nachweisreagenz für Iminoverbindungen, da das bei Reduktion entstehende Chinondiimin der Formel XIV schon farbig ist.

Zur Einhaltung eines für die Durchführung des Verfahrens geeigneten pH-Wertes, der sich insbesondere nach den einzusetzenden Enzymen und nach dem Nachweisreagenz für die Iminoverbindung richtet, enthält das erfindungsgemäße Mittel ein Puffersystem. Vorteilhafterweise enthält das Mittel ein Puffersystem, das in der Testlösung einen pH-Wert zwischen 4 - 9 einstellt. Insbesondere vorteilhaft ist ein leicht saurer pH-Wert zwischen 5 und 6,5.

Das erfindungsgemäße Mittel kann in Form einer Lösung oder auf einem saugfähigen oder quellbaren Träger aufgezogen vorliegen. In Form einer Lösung enthält das Mittel vorzugsweise sämtliche für das erfindungsgemäße Verfahren benötigte Reagenzien. Als Lösungsmittel kommen bevorzugt Wasser, aber auch Mischungen mit wasserlöslichen organischen Lösungmittel, wie zum Beispiel Methanol, Ethanol, Aceton oder Dimethylformamid in Frage. Aus Haltbarkeitsgründen kann es vorteilhaft sein, die für den Test benötigten Reagenzien auf zwei oder mehreren Lösungen zu verteilen, die erst bei der eigentlichen Untersuchung vermischt werden. Zweckmäßigerweise ist aber darauf zu achten, daß die aromatische Nitrosoverbindung und das Nachweisreagenz für Iminoverbindungen vor Testbeginn in einer Lösung vorliegen. Die Konzentration der eingesetzten aromatischen Nitrosoverbindungen richtet sich nach der Konzentration des zu messenden Analyten.

Typische Konzentrationen für die im erfindungsgemäßen Verfahren zu messenden Analyte sind 10⁻⁶ -10⁻² mol/l, insbesondere 10⁻⁵ - 10⁻³ mol/l. Entsprechend sind typische Konzentrationen der eingesetzten Nitrosoverbindungen 10⁻⁴ - 10⁻¹ mol/l. Um eine ausreichend schnelle enzymatische Reduktion zu erzielen sind Konzentrationen der Nitrosoverbindungen, insbesondere der chromogenen Nitrosoverbindungen der Formel XIV, von über 10⁻³ mol/l besonders vorteilhaft. Die Konzentration der eingesetzten PQQ-abhängigen Dehydrogenase richtet sich nach deren Aktivität und der Konzentration des Analyten. Typische Werte für Enzymkonzentrationen sind 1mU/ml - 1U/ml bei Küvettentests.

Nachweisreagenzien für Iminoverbindungen werden mindestens im stöchiometrischen Verhältnis zu den eingesetzten Nitrosoverbindungen eingesetzt, bevorzugt in einem 1,5 - 2-fachen Überschuß.

Das erfindunsgemäße Mittel kann auch in Form eines Teststreifens vorliegen. Solche Teststreifen sind in verschiedensten Ausführungsarten bekannt, zum Beispiel aus EP-A-O 016 387, DE-A-3247608, EP-A-O 262 445 oder EP-A-O 256 806. In einem Teststreifen liegen die zur Durchführung des Bestimmungsverfahrens benötigten Reagenzien auf festen Trägerschichten vor. Als Trägerschichten kommen insbesondere saugfähige und/oder quellbare Materialien in Frage, die von der zu untersuchenden Probenflüssigkeit benetzt werden. Beispiele sind Gelatine, Cellulose oder Kunstfaservliese. In oder auf diesen Tragermaterialien liegen die Reagenzien in fester Form vor. Bei Aufgabe der Probenflüssigkeit auf den Teststreifen oder Eintauchen des Teststreifens in die Probenflüssigkeit, bildet sich in den Streifen ein flüssiges Milieu aus, innerhalb dessen die Nachweisreaktion abläuft. Die durch die Reaktion verursachte Farbbildung kann visuell oder photometrisch, zum Beispiel reflektionsphotometrisch ausgewertet werden.

Die bevorzugten Konzentrationen der einzelnen Reagenzien auf Teststreifen betragen:
Analyt typischerweise 10⁻⁴ bis 10⁻¹ M
Nitrosoverbindung 10⁻³ bis 1M
nichtoxidatives Nachweisreagenz 10⁻³ bis 1M
Enzym 0,1 bis 100 U pro Testfeld

Die vorliegende Erfindung bietet den Vorteil, daß keine unspezifischen Reduktionskatalysatoren wie Diaphorase oder Methylphenazinium-methosulfat zur Reduktion eines Elektronenakzeptors notwendig sind, sondern daß dessen Reduktion direkt über ein spezifisches Analyt/Enzymsystem erfolgt. Störende Nebenreaktionen können so vermieden werden. Durch den Einsatz von Nitrosoverbindungen als Elektronenakzeptoren tritt keine Limitierung des Elektronenakzeptors beispielsweise durch zu langsame Diffusion mehr auf, wie dies bei Sauerstoff als Elektronenakzeptor der Fall ist. Pro Äquivalent der nachzuweisenden Iminozwischenstufe wird nur ein Äquivalent des Analyten oxidiert. Dies bewirkt eine hohe Empfindlichkeit des Analytnachweises. Ferner werden keine Oxidationsmittel für die Reaktion mit einem farberzeugenden Reagenz benötigt. Deshalb kann auch die gesamte Nachweisreaktion ohne Störung in einer Stufe und in einer gemeinsamen Lösung durchgeführt werden. Die große Bandbreite der einzusetzenden farbbildenden Nachweisreagenzien, unter anderem solche, die aus der Farbfotografie bekannt sind, erlaubt eine nahezu freie Auswahl der Wellenlänge bei der die Messung durchgeführt werden soll und des Extinktionskoeffizienten der die Empfindlichkeit der Messung bestimmt.

Ganz besonders einfach gestaltet sich das erfindungsgemäße Verfahren, wenn direkt eine farbige Iminoverbindung gebildet und eine Reaktion mit einem farbgebenden Reagenz -nötig wird.

Ein weiterer Gegenstand der Erfindung sind neue chromogene Nitrosoanilinverbindung der Formel XIII 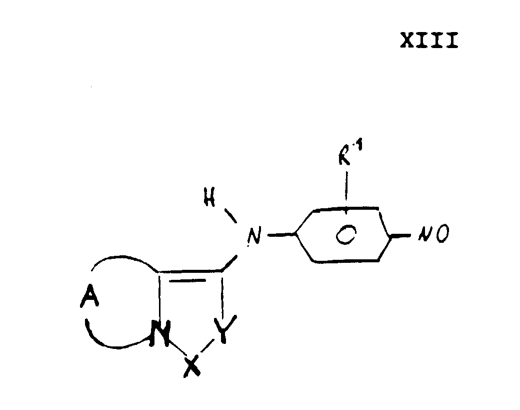 wobei
R¹
   Wasserstoff, Hydroxy, Halogen, Alkoxy oder Alkylthio, Aryloxy oder Arylthio, Alkyl gegebenenfalls substituiert durch Hydroxy, Carboxy, PO₃H₂ Alkyl, Dialkylphosphinyl oder SO₃H, Amino gegebenenfalls ein- oder mehrfach substituiert durch Alkyl, das wiederrum gegebenenfalls durch Hydroxy, PO₃H₂, SO₃H oder Carboxy substituiert sein kann, bedeutet, und NR⁵ -CO oder N=CR⁶ bedeutet
   - X-Y: NR⁵-CO oder N=CR⁶ bedeutet, wobei
R⁵ Wasserstoff, Alkyl gegebenenfalls substituiert durch Hydroxy, Carboxy, SO₃H, PO₃H₂, Dialkylphosphinyl und
R⁶ Wasserstoff, Alkyl gegebenenfalls jeweils substituiert durch Hydroxy, Dialkylphosphinyl, Carboxy, SO₃H, PO₃H₂, ein Salz eines dieser Säurereste oder/und Alkoxycarbonyl; oder
Amino, das gegebenenfalls durch einen oder zwei gegebenenfalls einen oder mehrere Hydroxy-, Carboxy- oder/und Alkoxycarbonylreste tragende Alkylreste substituiert ist, wobei, wenn Amino durch 2 Alkylreste substituiert ist, diese Reste auch zu einem Ring geschlossen sein können, der außer durch das N-Atom der Aminogruppe gegebenenfalls auch durch Sauerstoff, Schwefel oder ein weiteres Stickstoffatom unterbrochen sein kann, oder Amino, das gegebenenfalls durch ein oder zwei Acylgruppen, Alkoxy- oder/und Aralkoxycarbonylgruppen, H₂N-CO, Alkyl-, Aralkyl- oder/und carbamoylgruppen substituiert ist; oder Wasserstoff, Carboxy, Alkoxycarbonyl, Carboxamido oder Halogen und
A eine gesättigte oder ungesättigte Kette aus drei Gliedern mit einem Stickstoff oder Schwefelatom und zwei Kohlenstoffatomen oder zwei Stickstoffatomen und einem Kohlenstoffatom darstellt, wobei Kohlenstoffatome gegebenenfalls substituiert sind durch Alkyl, Alkoxy, Alkylthio, Hydroxy, Aralkyl, Aryl, Carboxy, Carboxamido, Alkoxycarbonyl, Cyano, Halogen, Amino, das gegebenenfalls durch einen oder zwei, gegebenenfalls einen oder mehrere Hydroxy-, Carboxy-, oder/und Alkoxycarbonylreste tragende Alkylreste substituiert ist, und wobei Stickstoffatome, die nicht über eine Doppelbindung gebunden sind, durch Wasserstoff, Alkyl, gegebenenfalls substituiert durch Hydroxy, SO₃H, Carboxy, PO₃H₂ oder Dialkylphosphinyl, oder durch Aralkyl substituiert sind oder zwei benachbarte Kettensubstituenten gegebenenfalls eine Alkylengruppe bilden, die ihrerseits gegebenenfalls mit Aryl substituiert oder anelliert ist,
sowie gegebenenfalls entsprechende tautomere Formen und deren Salze

Die Bedeutung der einzelnen Substituenten ist dabei die gleiche wie in der Verbindung mit der allgemeinen Formel II.

Bevorzugte Reste R₁ sind Wasserstoff und unsubstituiertes oder substituiertes Alkyl. X-y bildet bevorzugt die Gruppe N=CR⁶ wobei R⁶ bevorzugt Wasserstoff oder unsubstituiertes oder substituiertes Alkyl darstellt.

Bevorzugt bildet A mit dem benachbarten Heterocyclus einen Imidazol-, Triazol-, Benzimidazol-, Thiazol- oder Dihydroimidazol-Ring, in denen die Bedeutung der Ring-Substituenten denen der allgemeinen Formel III entspricht.

Ganz besonders bevorzugt bildet A einen Imidazolring.

Besonders bevorzugte Verbindungen sind:
1) (2,4-Dimethyl-pyrazolo-[1.5a]-imidazol-3-yl)-(4-nitrosophenyl)-amin
2) (4-Methyl-pyrazolo-[1.5 a]-imidazol-3 -yl)-(4-nitrosophenyl)-amin
3) (4-(Dimethylphosphinylmethyl)-2-methyl-pyrazolo-[1.5a]-imidazol-3yl)-(4-nitrosophenylamin)
5) (5,6 Dihydro-4-dimethylphosphinylmethyl-2 methylpyrazolo-[1.5a]-imidazol-3-yl)-(4-nitrosophenyl)-amin
6) (4-(Dimethylphosphinylmethyl-2-methyl-pyrazolo-[1.5a]imidazol-3-yl-(4-nitrosophenyl)-amin
7) (2,6 Dimethyl-4-dimethylphosphinylmethyl-pyrazolo-[3.2c]-s-triazol-3 -yl)-(4-nitrosophenyl)-amin

Die Herstellung der erfindungsgemäßen Verbindungen der Formel XIII erfolgt nach an sich bekannten Methoden, wie sie von J.T. Hays et al. im J. Org. Chem. 32, 158 (1967) beschrieben wurden. Dazu werden Ether, bevorzugt Methylether, von p-Nitrosophenolderivaten unter Protonenkatalyse mit entsprechenden 3-Amino-heterocyclen umgesetzt. Unter Substitution der Methoxygruppe entstehen sekundäre Amine der Formel XIII.

Die verwendeten Heteroarylamine sind entweder in der Literatur beschrieben oder können analog den bekannten Verfahren hergestellt werden. Insbesondere sind Aminoverbindungen, die als Grundbaustein Pyrazolo-Heterocyclen enthalten, in EP-A-O 433 854 beschrieben.

Die als Zwischenprodukte benötigten Aminoverbindungen liegen in der Regel aus Haltbarkeitsgründen als Salze von starken Säuren, z.B. Mineralsäuren vor. Zur Umsetzung mit p-Nitrosoanisolen werden bevorzugt die freien Basen der Aminoverbindungen eingesetzt, die man nach üblichen Methoden z.B. durch Auflösen der Salze in Wasser, Zugabe von Base bis zu einem pH 8-10 und Extraktion der freien Base mit einem organischen Lösungsmittel beispielsweise Essigester oder Methylenchlorid erhält. Alternativ dazu kann man, insbesondere bei schwer extrahierbaren Aminoverbindungen folgendermaßen vorgehen: Man löst die Aminoverbindung in Methanol, gibt dann eine Base z.B. NaHCO₃-Lösung, Triethylamin usw. zu, bis der pH-Wert der Methanollösung auf einem nassen pH Papier etwa den Wert 5-6 erreicht hat. Anschließend setzt man den zweiten Reaktionspartner, das p-Nitrosoanisol, zu.

### Beispiel 1

Glucose-Bestimmung mit PQQ-abhängiger Glucose-Dye-Oxidoreductase und 1-Naphthol-Sulfonsäure als Kupplungsreagenz

In einer Küvette wurden gemischt:

| | |
|---|---|
| Citratpuffer/Pyrophosphatpuffer (50mM) pH 7,5 | 0,38 ml |
| N,N- Bis (2-hydroxyethyl)-4-nitrosoanilin (5mM in Puffer) | 0,40 ml |
| 1-Naphthol-4-Sulfonsäure (10 mM in H₂O) als Kupplungsreagenz | 0,20 ml |
| Probelösung (0-40 mM Glucose) | 0,01 ml |

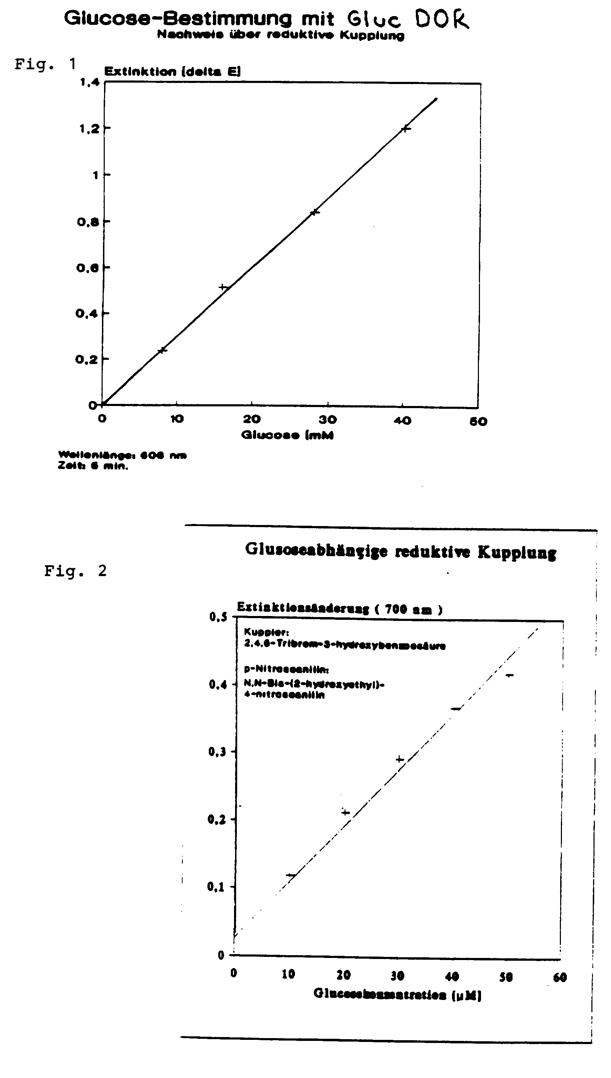

Der Test wurde durch Zugabe von 0,01 ml Enzymlösung (Glucose-Dye-Oxidoreduktase, EC 1.1.99.17, 900 U/mg, 800 U/ml) gestartet und die Extinktionsänderung bei 606 nm gemessen. Konstante Extinktionswerte wurden nach maximal 5 Minuten erreicht (Figur 1).

### Beispiel 2

Glucosenachweis mit PQQ-abhängiger Glucose-Dye-Oxidoreduktase und 2,4,6-Tribrom-3-hydroxybenzoesäure als Nachweisreagenz.

| Meßansatz (Endkonzentrationen) | |
|---|---|
| 100 mM | Citratpuffer pH 5,8 |
| 10 mM | 2,4,6-Tribrom-3-hydroxybenzoesäure |
| 1 mM | N,N-Bis-(2-hydroxyethyl)-4-nitrosoanilin |
| 10 U/ml | Glucose-Dye-Oxidoreduktase |
| 0,1 M | Natriumnitrat |
| 100-500 mM | Glucose |

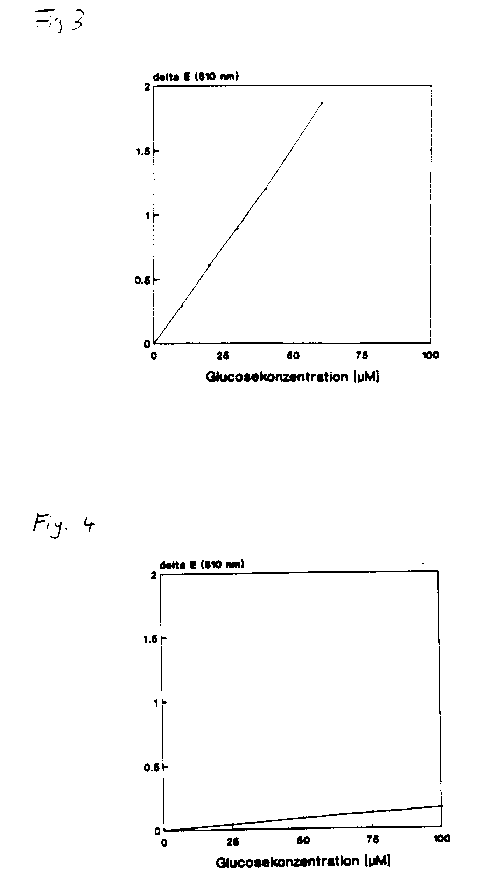

Mit zunehmender Glukosekonzentration wird eine zunehmende Bildung eines grünen Farbstoffes beobachtet (max.=700 nm, Figur 2).

### Beispiel 3

In gleicher Weise können die in Tabelle 1 aufgeführten weiteren Kupplungsreagenzien für Imine eingesetzt werden. Tabelle 1 gibt die nach der Kupplung des N,N-Bis(2-hydroxyethyl)-p-chinondiimins mit den aufgeführten Kupplern erhaltenen Absorptionsmaxima wieder.

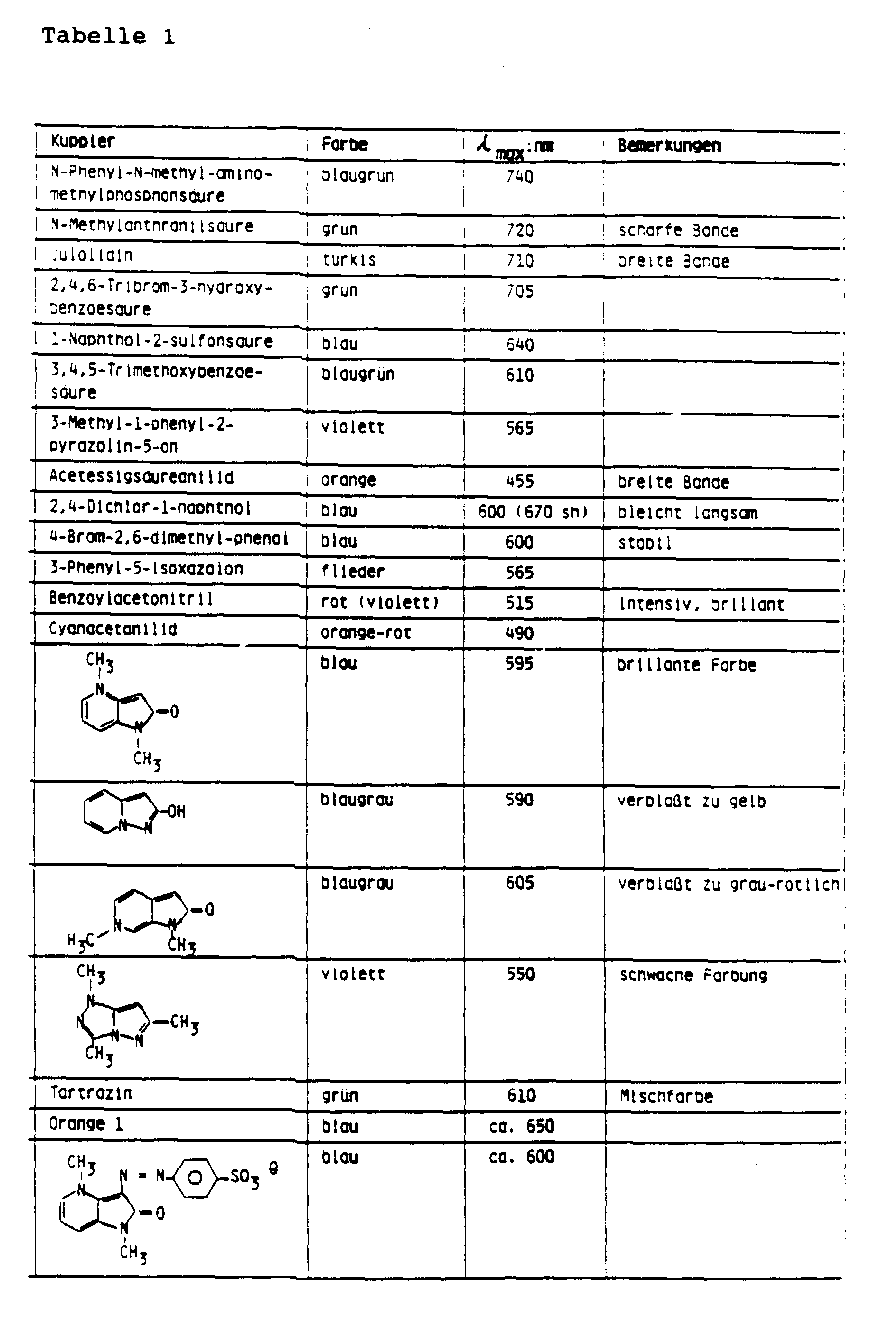

### Beispiel 4

Analog Beispiel 1 werden verschiedene in Tabelle 2 aufgeführte elektronenreiche aromatische Nitrosoverbindungen eingesetzt. Tabelle 2 gibt die nach Kupplung mit 1-Naphthol-2-Sulfonsäure oder 1-Naphthol-4-Sulfonsäure erhaltenen Farben bzw. Absorptionsmaxima an.

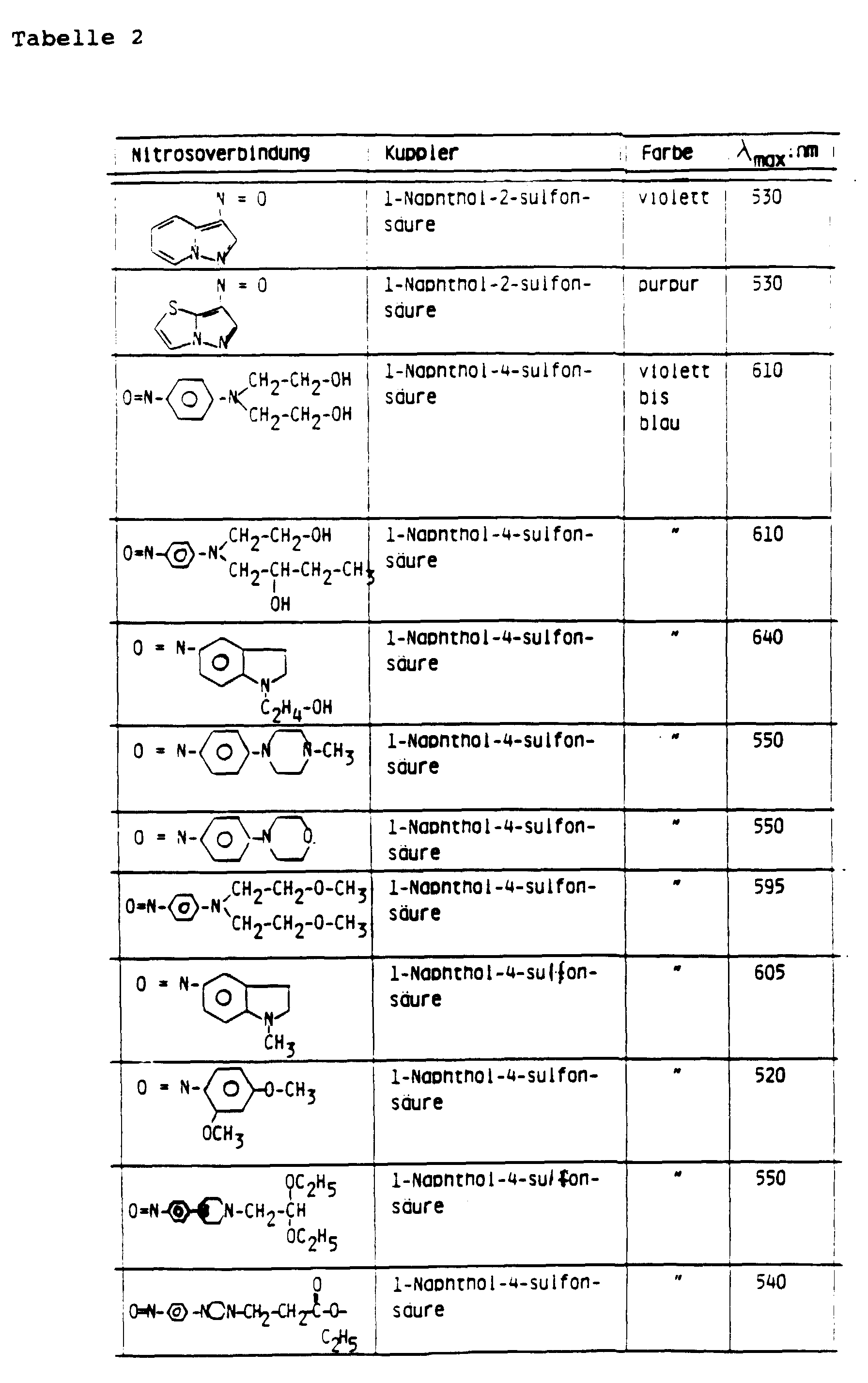

### Beispiel 5

Vergleich der Glukosebestimmung mittels Nachweis von Iminoverbindungen durch reduktive Kupplungen zwischen PQQ-abhängiger Glucose-Dye-Oxidoreduktase und Glucose-Oxidase.

In einer Küvette werden vorgelegt:

| | |
|---|---|
| 100 mM | Phosphatpuffer pH 7,5 |
| 1 mM | N,N-Bis-(2 hydroxyethyl)-4 nitrosoanilin |
| 10 mM | 1-Naphthol-4-Sulfonsäure |
| 100 U/ml | Glucose-Dye-Oxidoreductase |

Durch Zugabe von Glucose (0-60 mM) erhält man entsprechende Mengen an Farbe (Figur 3).

Wird die Glucose-Dye-Oxidoreductase durch die gleiche Menge Glucose-Oxidase ersetzt, erhält man hingegen nur eine um den Faktor 8 geringere Farbausbeute, da ein GroBteil der gebildeten Chinondiimin-Zwischenstufe von Glucoseoxidase unter Verbrauch von Glucose zum Phenylendiamin weiterreduziert wird (Figur 4).

### Beispiel 6

Glucosebestimmung mit PQQ-abhängiger Glucose-Dye-Oxidoreductase mittels reduktiver Bildung von farbigen Iminoverbindungen.

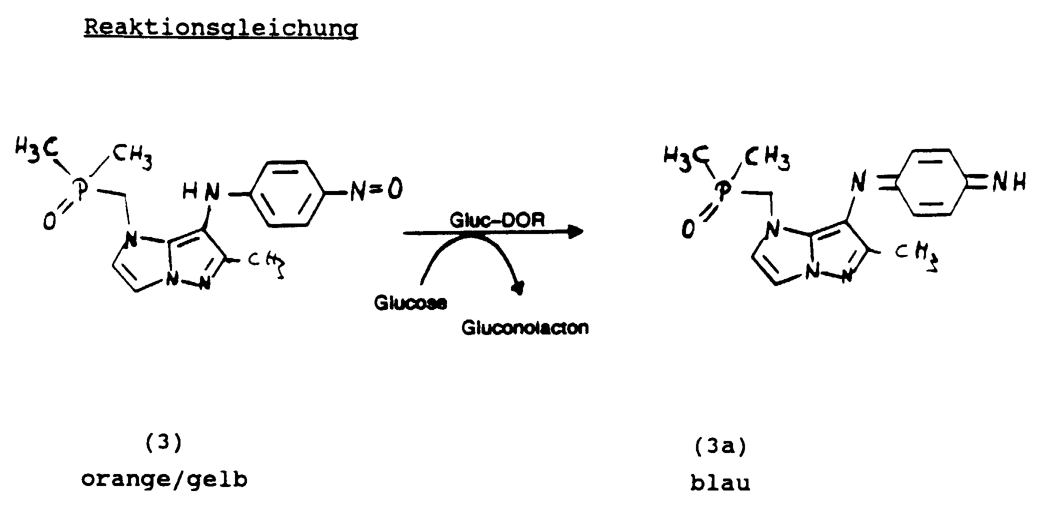

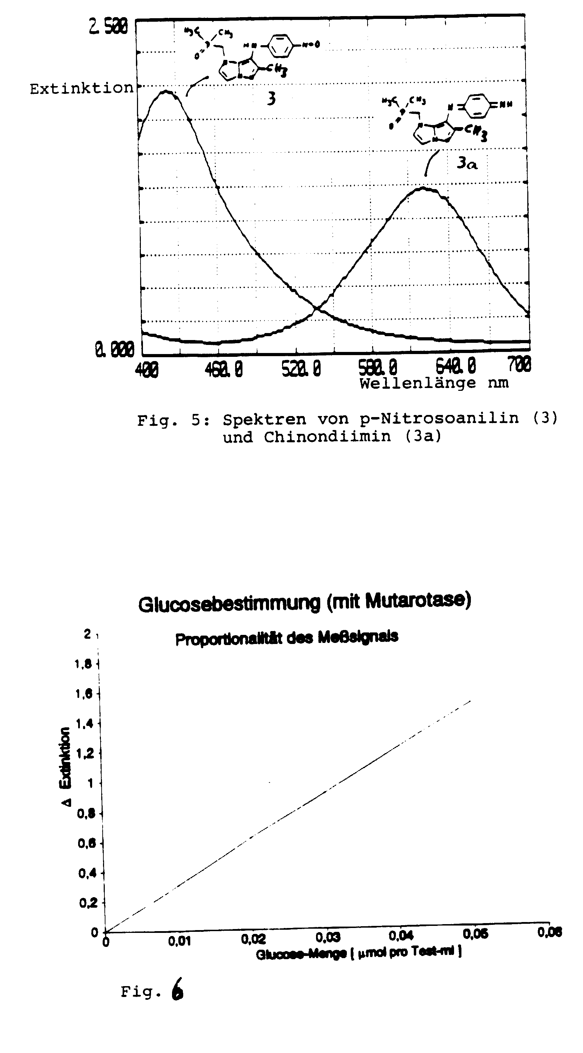

Die Spektren von (3) und (3a) zeigt Figur 5.

| Meßansatz (Endkonzentration) | |
|---|---|
| 200 mM | Citratpuffer pH 6 |
| 1 mM | p-Nitrosoanilin (Nr. 3 aus Tabelle 3) |
| 1 mM | CaCl₂ 20 U/ml Mutarotase |
| 10 U/ml | Glucose-Dye-Oxidoreductase |

Nach Zugabe verschiedener Glucosemengen zwischen 0 und 50 µM Endkonzentration wurden 5 min. später die Extinktionsänderungen gemessen (Figur 6)

Analog werden die Verbindungen aus Tabelle 3 eingesetzt. Tabelle 3 gibt die Absorptionsmaxima der eingesetzten Nitrosoverbindungen und der entstehenden farbigen Chinondiime an.

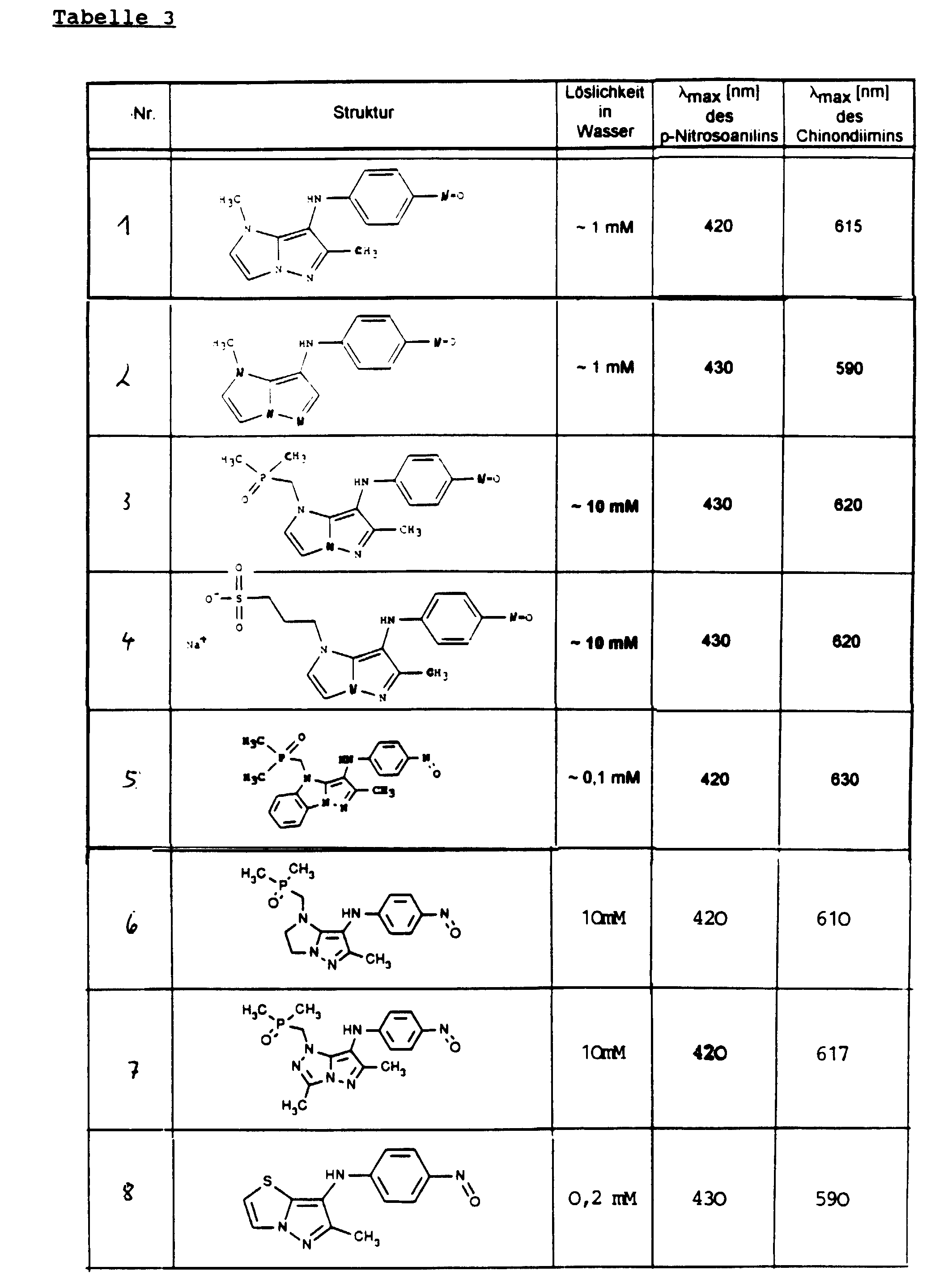

### Beispiel 7

Bestimmung der Enzymaktivität einer PQQ-abhängigen Glucose-Dye-Oxidoreductase mittels reduktiver Bildung einer farbigen Iminoverbindung aus einer aromatischen Nitrosoverbindung.

Die im Beispiel 6 beschriebene Reaktion kann auch zur Messung der Enzymaktivität einer PQQ-abhängigen Dehydrogenase benutzt werden.

| Meßansatz (Endkonzentrationen) | |
|---|---|
| 200 mM | Citratpuffer pH 5,8 |
| 1 mM | p-Nitrosoanilin (3) aus Beispiel 6 |
| 1mM | CaCl₂ |
| 30 mM | Glucose |

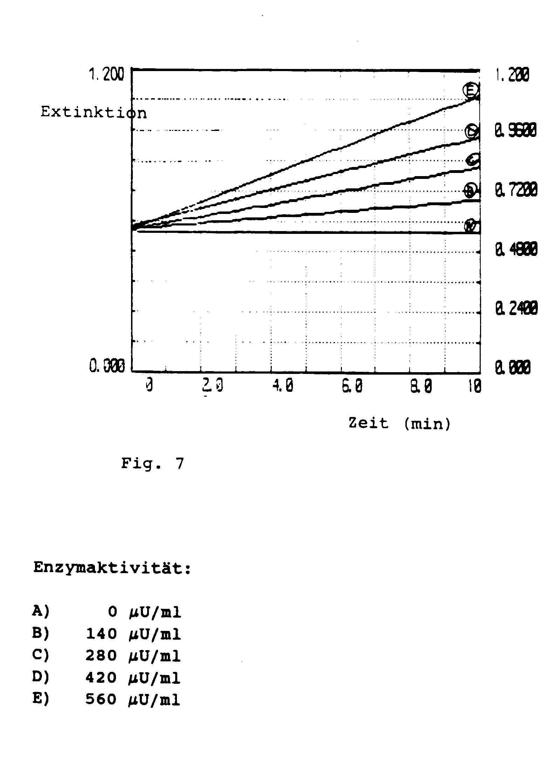

Die kinetische Messung wurde mit 0-1 mU Glucose-Dye-Oxidoreduktase gestartet und die Farbänderung zeitlich gemessen (Figur 7)

### Beispiel 8

### (4-(Dimethylphosphinylmethyl)-2-methyl-pyrazolo-[1.5a]-imidazol-3-yl)-(4-nitrosophenyl)-amin (3)

13.7 g 3-Amino-2-methyl-4-(dimethylphosphinylmethyl)-pyrazolo-[1.5a]-imidazol Dihydrochlorid werden in 350 ml Methanol gelöst. Die Lösung wird auf ca. 5 °C abgekühlt und mit konz. wässriger Natriumbikarbonatlösung versetzt, bis auf einem nassen pH-Papier ein pH-Wert von ca. 6 angezeigt wird. Dazu tropft man innerhalb von 30 min eine Lösung von 7.8 g p-Nitrosoanisol in 35 ml Methanol zu. Man rührt die Mischung 4 Stunden bei Raumtemperatur und hält den pH-Wert durch Zugabe von weiterer NaHCO₃-Lösung auf 6.

Das Reaktionsgemisch wird filtriert, mit ca. 150 ml Kieselgel vermischt und zur Trockene eingedampft. Das Kieselgel wird auf eine Kieselgelsäule gepackt und das Produkt durch Eluieren mit Toluol/Methanol 2 : 1 isoliert. Man erhält 10.3 g einer schwarzbraunen Masse, die erneut über Kieselgel mit Methylenchlorid/Methanol 12 : 1 chromatographiert wird. Man erhält 4.9 g der Titelverbindung mit dem Fp. 204 ° (unter Zersetzung).
R_{f} (Kieselgel) Toluol/Methanol 2 : 1 = 0.3
CH₂Cl₂/Methanol 12 : 1 = 0.21

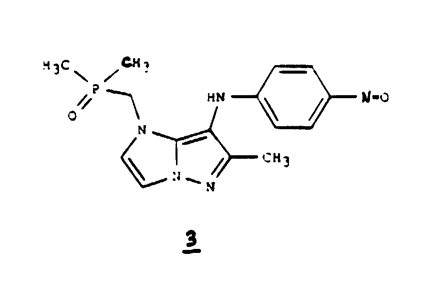

### Herstellung des Ausgangsproduktes

a) 4-Dimethylphosphinylmethyl-2-methyl-pyrazolo-[1.5a]-imidazol
   37 g 2-Methyl-pyrazolo-[1.5a]-imidazol (J. Het. Chem. 10, 441 (1973)) werden in 370 ml trockenem Dimethylformamid gelöst und mit 54.2 g Chlormethyl-dimethylphosphanoxid und 119 g Kaliumcarbonat versetzt. Die Mischung wird 10 Std. bei 115 ° (Badtemp.) gerührt. Der Niederschlag wird abgesaugt und das Filtrat eingedampft. Der Rückstand wird an Kieselgel chromatographiert (Laufmittel Essigester/Methanol 2 : 1). Man erhält insgesamt 36.6 g der Titelverbindung als Mischung von braunen Kristallen und einem braunen Öl. DC (Kieselgel, Essigester/Methanol 2 : 1) R_{f} = 0.2
b) 3-Amino-4-dimethylphosphinylmethyl-2-methyl-pyrazolo-[1.5a]-imidazol x 2 HCl
   18 g der oben erhaltenen Verbindung werden in 25 ml konz. Salzsäure und 50 ml Wasser gelöst. Dazu tropft man bei 0 ° eine Lösung von 6.2 g Natriumnitrit in 25 ml Wasser. Nach 30 min bei 0 ° macht man die Lösung durch Zugabe von Natriumbicarbonatlösung leicht alkalisch und gibt dann portionsweise 21.4 g Natriumdithionit zu. Man rührt das Gemisch noch 30 min und versetzt es mit einer Lösung von 17 g Di-tert.-butyldicarbonat in 100 ml Dioxan. Die Reaktionsmischung wird über Nacht bei Raumtemperatur gerührt, das Dioxan abdestilliert und der Rückstand mehrfach mit n-Butanol/Essigester 3 : 1 extrahiert. Der nach dem Trocknen und Eindampfen der organischen Phase verbleibende Rückstand wird in 320 ml mit HCl gesättigtem Methanol gelöst. Man rührt noch 2 Stunden, kühlt im Eisbad ab und filtriert die ausgefallenen Kristalle ab. Es ergeben sich insgesamt 18.1 g der Titelverbindung. DC (Kieselgel, Toluol/Methanol 3 : 1) R_{f} = 0.1.

### Beispiel 9

### (4-Nitrosophenyl)-2-methyl-(4-sulfopropyl-pyrazolo-[1.5a]-imidazol-3-yl)-amin (4)

Analog Beispiel 7 setzt man 3-Amino-4-sulfopropyl-pyrazolo-[1.5a]-imidazol mit p-Nitrosoanisol in Methanol um. Die Reaktionsmischung wird filtriert und das Filtrat eingedampft. Der Rückstand wird über Kieselgel mit Toluol/Methanol 2 : 1 chromatographiert. Das Produkt wird dann zur weiteren Reinigung auf eine Säule mit dem Adsorberharz HP 20SS (Fa. Mitsubishi) gegeben und mit einem Stufengradienten von Methanol/Wasser 1 : 9 bis 2 : 8 eluiert. Man vereinigt die produkthaltigen Fraktionen, engt ein, nimmt in wenig Ethanol auf und fällt das Produkt durch Zugabe von Ether aus. Man erhält das Titelprodukt in Form eines braunen Pulvers.

DC (Kieselgel, Ethanol: R_{f} = 0.6, Isopropanol/Essigsäurebutylester/Wasser 5 : 3 : 2: R_{f}-= 0.48)

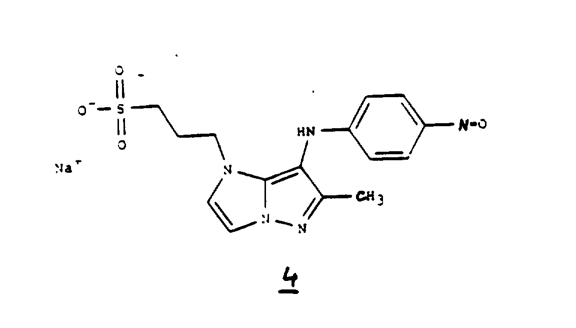

### Herstellung des Ausgangsproduktes

a) 2-Methyl-4-sulfopropyl-pyrazolo-[1.5a]-imidazol
   2-Methyl-pyrazolo-[1.5a]-imidazol wird mit Phenyldiazoniumsalz, das man in üblicher Weise durch Diazotieren von Anilin erhält, bei pH 2-5 umgesetzt. Das erhaltene 2-Methyl-3-phenylazo-pyrazolo-[1.5a]-imidazol (5.6 g) wird in 60 ml Dimethylformamid gelöst, mit 3.4 g Propansulton und 7 g Kaliumcarbonat versetzt. Man rührt das Gemisch 6 Std. bei 110 °, gibt erneut 5 g Propansulton und 7 g Kaliumcarbonat hinzu und rührt weitere 8 Stunden bei 110 °. Nach dem Abkühlen wird filtriert und der Rückstand zweimal gut mit Methanol gewaschen. Die Lösung wird eingeengt und der Rückstand chromatographisch an Kieselgel getrennt (Laufmittel Essigester/Methanol/Wasser 75 : 15 : 10).
   Ausbeute: 5.3 g gelbbraune Kristalle
   DC (Kieselgel, Essigester/Methanol/Wasser 75 : 15 : 10)
   R_{f} = 0.3
b) 3-Amino-2-methyl-4-sulfopropyl-pyrazolo-[1.5a]-imidazol
   4.3 g der oben erhaltenen Azoverbindung werden in 40 ml Eisessig gelöst und innerhalb 1 Stunde portionsweise mit 4 g Zinkpulver versetzt. Man rührt das Gemisch noch 30 min, saugt ab und dampft ein. Der Rückstand wird mit 30 ml Essigester verrieben, abgesaugt und das Filtrat verworfen. Man erhält 10.2 g eines graubraunen Pulvers, das zur Weiterverarbeitung rein genug ist.
   DC (Kieselgel, Essigester/Aceton/Eisessig/Wasser 5 : 2 : 2 :1)
   R_{f} = 0.14

### Beispiel 10

Analog zu Beispiel 8 oder 9 wurden die Verbindungen aus Tabelle 4 hergestellt. Die Synthese der Amino-Ausgangsverbindungen erfolgt auf folgende Weise:
a) Herstellung der Ausgangsverbindung zu 5 in Tabelle 4: 3-Amino-5,6-dihydro-,4-dimethylphosphinylmethyl-2-methyl-pyrazolo-[1.5a]-imidazol
   3.9 g 2-Methyl-pyrazolo-[1.5a]-imidazolin, 2.7 g Natriumacetat und 7.4 g p-Methoxybenzoldiazonium-tetrafluoroborat werden in 40 ml Eisessig gelöst und die Lösung 1 Stunde auf 40 - 50 ° erhitzt. Das Reaktionsgemisch wird eingedampft, der Rückstand in NaHCO₃-Lösung und Essigester aufgenommen. Man extrahiert mit Essigester, trocknet und dampft ein. Der Rückstand wird durch Chromatographie über Kieselgel gereinigt (Laufmittel: Essigester/Ligroin 1 : 1 - 2 : 1; Essigester; Essigester/Methanol 95 : 5). Man erhält 4.12 g der entsprechenden 3-Azoverbindung (DC: Kieselgel, Essigester/Methanol 95 : 5; R_{f} = 0.5), die mit Chlormethyl-dimethylphosphanoxid am Stickstoff des Imidazolinringes alkyliert wird. Die Überführung in die 3-Aminoverbindung erfolgt durch Reduktion der Azogruppe analog Beispiel 8b.
   Zur Isolierung und Reinigung wird die rohe Aminoverbindung in wenig Wasser gelöst, mit festem Natriumbicarbonat versetzt und eine Lösung der dreifachen molaren Menge an Diter.-butyldicarbonat in Dioxan zugegeben. Man rührt das Gemisch über Nacht, dampft ein, extrahiert zunächst mit Ether, um Nebenprodukte zu entfernen und dann 5mal mit Methylenchlorid, um das Produkt zu isolieren. Man erhält die kristalline t-Butoxycarbonylverbindung (DC: Essigester/Aceton/Eisessig/Wasser 50 : 25 : 12 : 5 : 12 : 5, R_{f} = 0.5), die zur Abspaltung der tert. Butoxycarbonylgruppe in 50 ml methanolischer Salzsäure gelöst wird. Nach 1 Stunde bei Raumtemperatur engt man auf die Hälfte ein und fällt das Produkt mit Ether aus. Man erhält die Titelverbindung als Hydrochlorid.
   DC: n-Butanol/Eisessig/Wasser 2 : 1 : 1 R_{f} = 0.3
b) Herstellung der Ausgangsverbindung zu 6 in Tabelle 4: 3-Amino-4-dimethylphosphinylmethyl-2-methyl-pyrazolo[1.5a]-benzimidazol
   Die Titelverbindung wird analog Beispiel 10 durch Umsetzung von 2-Methyl-pyrazolo-[1.5a]-benzimidazol (J. prakt. Chem. 326, 829 (1984)) mit Phenyldiazoniumsalz, N-Alkylierung mit Chlormethyl-dimethylphosphanoxid und Reduktion der Azogruppe mit Zink in Eisessig durchgeführt. Man erhält die Titelverbindung als Trihydrochlorid-Dihydrat vom Fp. 192 (Zersetzung)
   DC: Kieselgel, Isopropanol/Essigsäurebutylester/Wasser 50 : 30 : 20)
   R_{f} = 0.38
c) Herstellung der Ausgangsverbindung zu 7 in Tabelle 4: 3-Amino-2,6-dimethyl-4-dimethylphosphinyl-pyrazolo-[3.2c]-s-triazol
   6 g 2,6-Dimethyl-pyrazolo-[3.2c]-s-triazol werden in 65 ml Dimethylformamid gelöst und die Lösung mit 3.9 g Chlormethyl-dimethylphosphanoxid und 8 g Kaliumcarbonat versetzt. Man rührt das Gemisch 2 Stunden bei 100 °, saugt heiß ab und dampft das Filtrat ein. Der Rückstand wird über Kieselgel chromatographiert (Laufmittel Essigester/Methanol 4 : 1). Zur Verseifung und Decarboxylierung wird das Produkt 7 Stunden in conc. Salzsäure unter Rückfluß erhitzt. Das Reaktionsgemisch wird eingedampft. Man erhält ein hellbraunes Öl.
   DC (Kieselgel Essigester/Methanol 2 : 1)
   R_{f} = 0.37
   Die Umwandlung des oben erhaltenen Produktes in die 3-Aminoverbindung erfolgt analog dem in Beispiel 8 für die entsprechende 3-Aminopyrazolo-[1.5a]-imidazol beschriebenen Verfahren (8b). Man erhält die Titelverbindung als Hydrochlorid vom Fp. 225 ° (Zersetzung).
   DC (Kieselgel Essigester/Methanol 3 : 1)
   R_{f} = 0.2

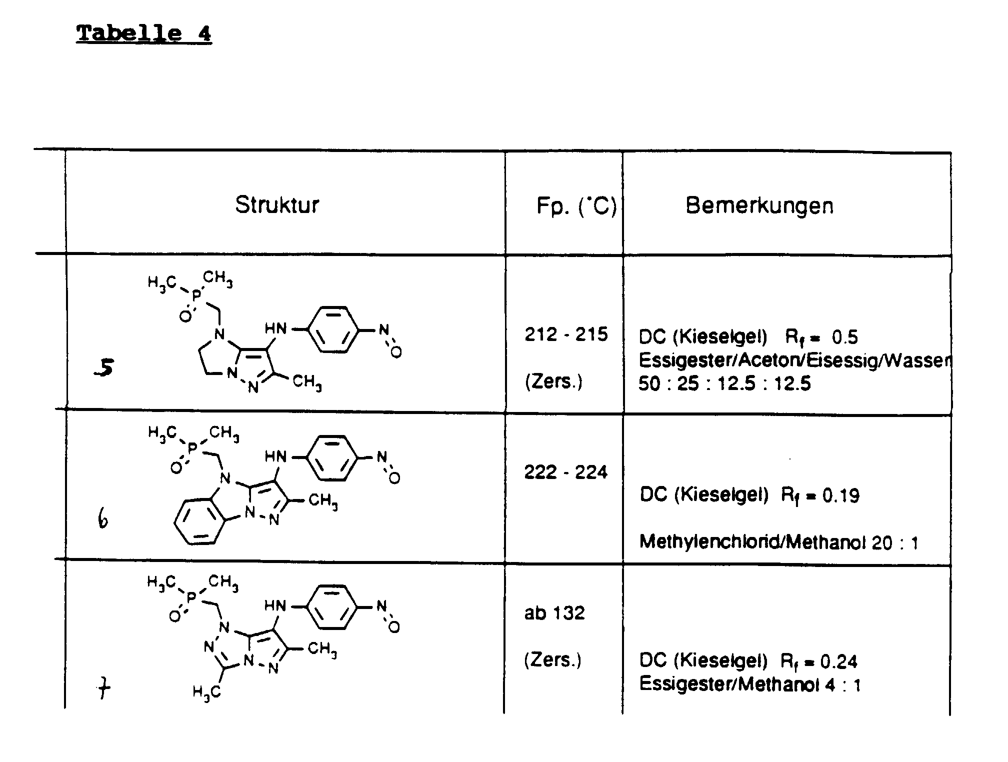

## Patentansprüche

1. Verfahren zur kolorimetrischen Bestimmung eines Analyten mittels enzymatischer Oxidation des Analyten mit einer Oxidoreduktase in Gegenwart eines direkten Elektronenakzeptors aus der Gruppe der elektronenreichen aromatischen Nitrosoverbindungen und Bestimmung des reduzierten Elektronenakzeptors durch Farbbildung als Maß fur die Menge des Analyten, wobei die elektronenreiche aromatische Nitrosoverbindung unter Oxidation des Analytenin Gegenwart einer PQQ-abhängigen Dehydrogenase zu einer Iminoverbindung reduziert wird, dadurch gekennzeichnet, daß die Iminoverbindung ohne enzymatische Weiterreduktion zum aromatischen Amin durch Farbbildung über ihre Eigenfarbe nachgewiesen wird.

2. Verfahren zur kolorimetrischen Bestimmung eines Analyten mittels enzymatischer Oxidation des Analyten mit einer Oxidoreduktase in Gegenwart eines direkten Elektronenakzeptors aus der Gruppe der elektronenreichen aromatischen Nitrosoverbindungen und Bestimmung des reduzierten Elektronenakzeptors durch Farbbildung als Maß fur die Menge des Analyten, wobei die elektronenreiche aromatische Nitrosoverbindung unter Oxidation des Analyten in Gegenwart einer PQQ-abhängigen Dehydrogenase zu einer Iminoverbindung reduziert wird, dadurch gekennzeichnet, daß die Iminoverbindung ohne enzymatische Weiterreduktion zum aromatischen Amin durch Reaktion mit einem nicht-oxidativen farbgebenden Nachweisreagenz durch Farbbildung nachgewiesen wird.

3. Verfahren gemäß einem der Ansprüche 1 oder 2, dadurch gekennzeichnet, daß als aromatische Nitrosoverbindung eine Verbindung der Formel I eingesetzt wird, 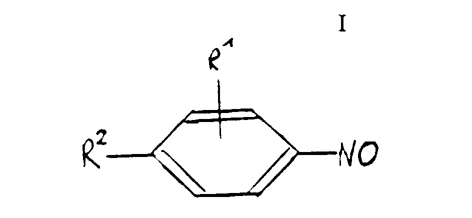 wobei R¹
Wasserstoff, Hydroxy, Alkyl, Alkyl substituiert durch Hydroxy, COOH oder PO₃H₂, SO₃H, Alkoxy, Alkylthio, Aryloxy, Arylthio, Halogen, oder Amino, Amino das ein- oder mehrfach durch Alkyl, Alkyl substituiert durch Hydroxy, PO₃H₂, Dialkylphosphinyl, SO₃H oder CO₂H, substituiert ist, bedeutet und R²
eine Hydroxygruppe, Alkylthio-, Alkoxy-, Aryloxy-, Arylthio- oder Alkylthiogruppe, wobei der Alkylrest seinerseits durch eine Hydroxygruppe, eine Alkoxygruppe, eine Aminogruppe, eine ein- oder mehrfach durch Alkyl substituierte Aminogruppe, PO₃H₂, SO₃H oder CO₂H als solche oder in Salzform als Ammonium-, Alkali- oder Erdalkalisalze substituiert ist, bedeutet oder eine Aminogruppe NR³R⁴ bedeutet,
in der R³ und R⁴ gleich oder verschieden sein können, und Wasserstoff, eine Aryl- oder Alkylgruppe, die ihrerseits durch eine Hydroxy-, Alkoxy-, Hydroxyalkoxy-, eine Polyalkoxygruppe, eine hydroxysubstituierte Polyalkoxygruppe, PO₃H₂, SO₃H, COOH als solche oder in Salzform oder durch eine Aminogruppe oder eine ein- oder mehrfach durch Alkyl substituierte Aminogruppe substituiert sein kann, bedeutet, oder
in der R³ und R⁴ einen Alkylenrest darstellen können, oder einen Alkylenrest, der durch Sauerstoff, Schwefel oder Stickstoffunterbrochen ist, wobei Stickstoff durch einen Alkyl-, Hydroxyalkyl-, Hydroxyalkoxyalkyl-, Alkoxyhydroxyalkyl-, Alkoxycarbonylalkyl-, Dioxanylylalkyl- oder Polyalkoxyalkylrest substituiert ist, der seinerseits jeweils im Alkylteil durch einen Hydroxyrest substituiert sein kann, oder
wenn R¹ in ortho-Stellung zu NR³R⁴ steht, R³ oder R⁴ auch zusammen mit R¹ einen Alkylenrest darstellen kann.

4. Verfahren gemäß einem der Ansprüche 1 oder 2, dadurch gekennzeichnet, daß als aromatische Nitrosoverbindung eine Verbindung der Formel II eingesetzt wird, 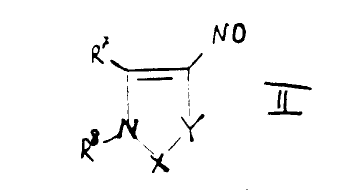 in der
X-Y NR⁵-CO oder N=CR⁶ bedeutet, wobei
R⁵ H, Alkyl, Alkyl substituiert durch Hydroxy, Carboxy, SO₃H, PO₃H₂, Dialkylphosphinyl
R⁶ H, Alkyl, Alkenyl, Alkoxy, Alkylthio, Aryl, Arylthio, Aralkyl, die jeweils durch Hydroxy, Dialkylphosphinyl, Carboxy, SO₃H, PO₃H₂, ein Salz eines dieser Säurereste oder/und Alkoxycarbonyl substituiert sein können; oder
Amino oder Amino, das durch einen oder zwei Alkylreste, die einen oder mehrere Hydroxy-, Carboxy oder/und Alkoxycarbonylreste tragen können, substituiert ist, wobei, wenn Amino durch 2 Alkylreste substituiert ist, diese Reste auch zu einem Ring geschlossen sein können, der außer durch das N-Atom der Aminogruppe auch durch Sauerstoff, Schwefel oder ein weiteres Stickstoffatom unterbrochen sein kann, oder Amino, oder Amino das durch ein oder zwei Acylgruppen, Alkoxy- oder/und Aralkoxycarbonylgruppen, H₂ N-CO, Alkyl-, Aralkyl- oder/und Arylcarbamoylgruppen substituiert ist; oder Wasserstoff, Carboxy, Alkoxycarbonyl, Carboxamido oder Halogen ist und
R⁷ Alkyl, Thioalkyl oder Aralkyl, das durch Hydroxy, Carboxy, SO₃H oder PO₃H₂ substituiert sein kann, oder Amino, oder Amino, das durch eine oder zwei Alkylgruppen substituiert ist, die wiederum durch Hydroxy, Carboxy, SO₃H, Dialkylphosphinyl oder PO₃H₂ substituiert sein können, bedeutet, wobei mindestens R⁶ und/ oder R⁷ eine Aminogruppe darstellt
R⁸ eine Alkyl- oder Aralkylgruppe darstellt, die durch Hydroxy, Carboxy, SO₃H oder PO₃H₂ substituiert sein kann
oder wobei
R⁷ und R⁸ eine gesättigte oder eine ungesättigte Kette mit 3 oder 4 Gliedern aus Stickstoffatomen oder aus Kohlenstoffatomen oder aus Kohlenstoffatomen und einem oder mehreren Stickstoff- oder Schwefelatomen darstellt, wobei Kohlenstoffatome substituiert sein können durch Alkyl, Alkoxy, Alkylthio, Hydroxy, Aralkyl, Aryl, Carboxy, Carboxamido, Alkoxycarbonyl, Cyano, Halogen, Amino, Amino, das durch einen oder zwei Alkylreste, die einen oder mehrere Hydroxy-, Carboxy- oder /und Alkoxycarbonylreste tragen können, substituiert ist, und wobei Stickstoffatome, die nicht über eine Doppelbindung gebunden sind, durch Alkyl oder Aralkyl, oder Dialkylphosphinyl substituiert sind oder zwei benachbarte Kettensubstituenten eine Alkylengruppe bilden, die ihrerseits mit Aryl substituiert oder anelliert sein kann,
sowie entsprechende tautomere Formen und deren Salze.

5. Verfahren gemäß Anspruch 4, dadurch gekennzeichnet, daß als aromatische Nitrosoverbindung eine Verbindung eingesetzt wird, in der R⁷ und R⁸ eine gesättigte oder ungesättigte Kette bilden.

6. Verfahren gemäß einem der Ansprüche 1 oder 2, dadurch gekennzeichnet, daß als aromatische Nitrosoverbindung eine Verbindung der Formel III - XII eingesetzt wird, 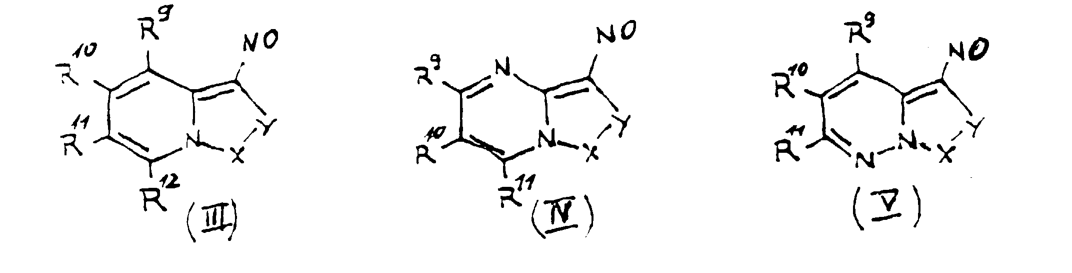 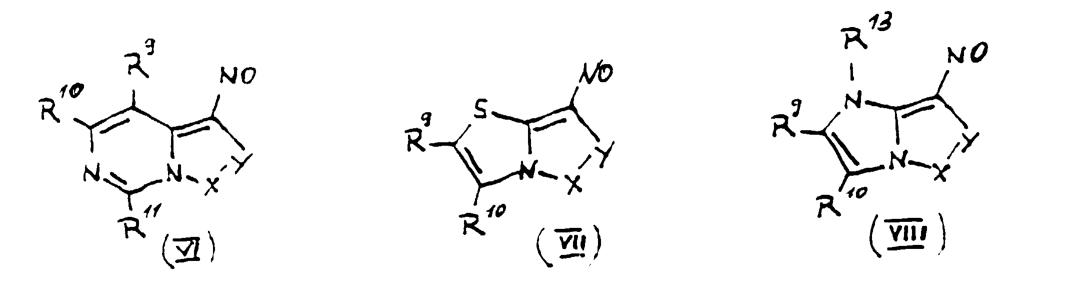 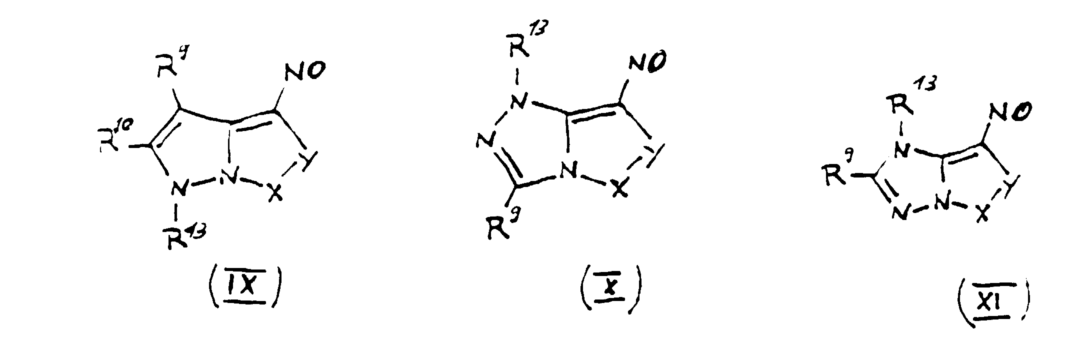 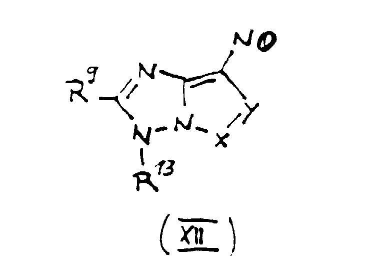
wobei R⁹, R¹⁰, R¹¹ und R¹², die gleich oder verschieden sind, für Wasserstoff, Hydroxy, Alkyl, Alkoxy, Alkylthio, Aralkyl, Aryl, Carboxy, Alkoxycarbonyl, Carboxamido, Cyano, Amino, Amino, das durch ein oder zwei Alkylreste, die einen oder mehrere Hydroxy-, Carboxy- oder/und Alkoxycarbonylreste tragen können, substituiert ist, oder Halogen stehen, wobei zwei benachbarte Reste eine Alkylengruppe bilden können, die ihrerseits mit Aryl substituiert oder anelliert sein kann
und R¹³ Alkyl oder Aralkyl darstellt, das durch Hydroxy, Carboxy, SO₃H, PO₃H₂ oder Dialkylphosphinyl substituiert sein kann.

7. Verfahren gemäß Anspruch 2, dadurch gekennzeichnet, daß das nicht-oxidative farbgebende Nachweisreagenz ein Kupplungsreagenz für Iminoverbindungen ist.

8. Verfahren gemäß Anspruch 7, dadurch gekennzeichnet, daß als Kupplungsreagenz ein Phenol-, Naphthol-, Anilin- oder ein Naphthylamiderivat eingesetzt wird.

9. Verfahren gemäß Anspruch 7, dadurch gekennzeichnet, daß als Kupplungsreagenz 2,4,6-Tribrom-3-hydroxybenzoesäure, 2,4-Dichlor-1-naphthol oder 1-Naphthol-4-sulfonsäure eingesetzt wird.

10. Verfahren gemäß Anspruch 2, dadurch gekennzeichnet, daß die Iminoverbindung durch Oxidation eines Leukofarbstoffes zu einem Farbstoff nachgewiesen wird.

11. Verfahren gemäß Anspruch 10, dadurch gekennzeichnet, daß als Leukofarbstoff Diarylimidazole, Triarylimidazole oder Naphthylamine verwendet werden.

12. Verfahren gemäß Anspruch 1, dadurch gekennzeichnet, daß eine aromatische Nitrosoanilinverbindung der allgemeinen Formel XIII 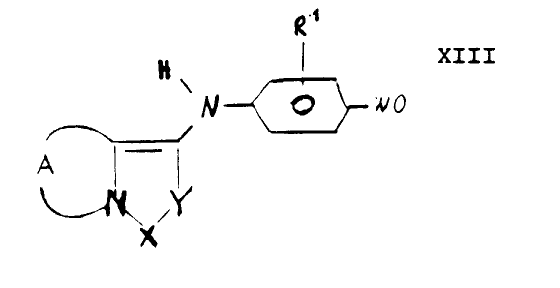 eingesetzt wird, in der
R¹ Wasserstoff, Hydroxy, Halogen, Alkoxy, Alkylthio, Aryloxy oder Arylthio, Alkyl, Alkyl substituiert durch Hydroxy, Carboxy, PO₃H₂, oder SO₃H, Amino, Amino ein- oder mehrfach substituiert durch Alkyl, das wiederum durch Hydroxy, PO₃H₂ Dialkylphosphinyl, SO₃H oder Carboxy substituiert sein kann, bedeutet und
X-Y NR⁵-C0 oder N=CR⁶ darstellt, wobei
R⁵ H, Alkyl, Alkyl substituiert durch Hydroxy, Carboxy, SO₃H, PO₃H₂, Dialkylphosphinyl und
R⁶ H, Alkyl, Alkenyl, Alkoxy, Alkylthio, Aryl, Arylthio,Aralkyl, die jeweils durch Hydroxy, Carboxy, SO₃H, PO₃H₂, ein Salz eines dieser Säurereste oder/und Alkoxycarbonyl substituiert sein können; oder Amino oder
Amino, das durch einen oder zwei Alkylreste, die einen oder mehrere Hydroxy-, Carboxy oder/und Alkoxycarbonylreste tragen können, substituiert ist, wobei wenn Amino durch 2 Alkylreste substituiert ist, diese Reste auch zu einem Ring geschlossen sein können, der außer durch das N-Atom der Aminogruppe auch durch Sauerstoff, Schwefel oder ein weiteres Stickstoffatom unterbrochen sein kann oder Amino oder Amino, das durch ein oder zwei Acylgruppen, Alkoxy- oder/und Aralkoxycarbonylgruppen, H₂ N-CO, Alkyl-, Aralkyl- oder/und Arylcarbamoylgruppen substituiert ist; oder Carboxy, Alkoxycarbonyl, Carboxamido oder Halogen ist und
A eine gesättige oder ungesättigte Kette aus drei Gliedern mit einem Stickstoff- oder Schwefelatom und zwei Kohlenstoffatomen oder zwei Stickstoffatomen und einem Kohlenstoffatom darstellt, wobei Kohlenstoffatome substituiert sein können durch Alkyl, Alkoxy, Alkylthio, Hydroxy, Aralkyl, Aryl, Carboxy, Carboxamido, Alkoxycarbonyl, Cyano, Halogen, Amino, Amino, das durch einen oder zwei Alkylreste, die einen oder mehrere Hydroxy-, Carboxy-, oder/ und Alkoxycarbonylreste tragen können, substituiert ist, und wobei Stickstoffatome, die nicht über eine Doppelbindung gebunden sind, durch Wasserstoff, Alkyl, Alkyl substituiert durch SO₃H, PO₃H₂, Carboxy oder Dialkylphosphinyl, oder durch Aralkyl substituiert sind oder zwei benachbarte Kettensubstituenten eine Alkylengruppe bilden können, die ihrerseits mit Aryl substituiert oder anelliert sein kann, sowie entsprechende tautomere Formen und deren Salze,
die enzymatisch zu einem farbigen Chinondiimin reagiert, das kolorimetrisch als Maß fur die Menge des Analyten bestimmt wird.

13. Verfahren gemäß Anspruch 12, dadurch gekennzeichnet, daß A mit dem benachbarten Heterocyclus einen Imidazol-, Triazol-, Benzimidazol-, Thiazol- oder Dihydroimidazol-Ring bildet.

14. Verfahren gemäß Anspruch 12 oder 13, dadurch gekennzeichnet, daß die aromatische Nitrosoverbindung in einer Konzentration von höher als 10⁻³ mol/l eingesetzt wird.

15. Verfahren gemäß einem der Ansprüche 1-14, dadurch gekennzeichnet, daß als PQQ-abhängige Dehydrogenase Glukose-dye-oxidoreduktase zur Bestimmung von Glukose eingesetzt wird.

16. Mittel zur kolorimetrischen Bestimmung eines Analyten durch enzymatische Oxidation des Analyten enthaltend eine PQQ-abhängige Dehydrogenase und eine elektronenreiche aromatische Nitrosoverbindung, dadurch gekennzeichnet, daß es weiterhin ein farbbildendes nicht-oxidatives Nachweisreagenz für Iminoverbindungen enthält.

17. Mittel gemäß Anspruch 16, dadurch gekennzeichnet, daß es eine Nitrosoverbindung der allgemeinen Formel I enthält, 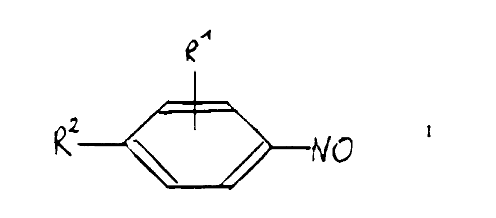
wobei R¹
Wasserstoff, Hydroxy, Alkyl, Alkyl substituiert durch Hydroxy, COOH, PO₃H₂ oder SO₃H, Alkoxy, Alkylthio, Aryloxy, Arylthio, Halogen, oder Amino, Amino, das durch Hydroxy, PO₃H₂, Dialkylphosphinyl, SO₃H oder CO₂H, substituiert ist, bedeutet
und R²
eine Hydroxygruppe, Alkoxy, Aryloxy-, Arylthio- oder Alkylthiogruppe bedeutet, wobei der Alkylrest seinerseits durch eine Hydroxygruppe, eine Alkoxygruppe, eine Aminogruppe, eine ein- oder mehrfach durch Alkyl substituierte Aminogruppe, PO₃H₂, SO₃H oder CO₂H als solche oder in Salzform als Ammonium-, Alkali- oder Erdalkalisalze substituiert sein kann, oder eine Aminogruppe NR³R⁴ bedeutet,
in der R³ und R⁴ gleich oder verschieden sein können, und Wasserstoff, eine Aryl- oder Alkylgruppe, die ihrerseits durch eine Hydroxy-, Alkoxy-, Hydroxyalkoxy-, Polyalkoxy-, eine hydroxysubstituierte Polyalkoxygruppe, PO₃H₂, SO₃H oder CO₂H als solche oder in Salzform oder durch eine Aminogruppe oder ein- oder mehrfach durch Alkyl substituierte Aminogruppe substituiert sein kann, bedeutet, oder
in der R³ und R⁴ einen Alkylenrest darstellen können, der durch Sauerstoff, Schwefel oder Stickstoffunterbrochen sein kann, wobei Stickstoff durch einen Alkyl-, Hydroxyalkyl-, Hydroxyalkoxyalkyl-, Alkoxyhydroxyalkyl-, Alkoxycarbonylalkyl-, Dioxanylyl-alkyl- oder Polyalkoxyalkylrest substituiert ist, der seinerseits jeweils im Alkylteil durch einen Hydroxyrest substituiert sein kann, oder
wenn R¹ in ortho-Stellung zu NR³R⁴ steht, R³ oder R⁴ auch zusammen mit R¹ einen Alkylenrest darstellen kann.

18. Mittel gemäß Anspruch 16, dadurch gekennzeichnet, daß es eine Nitrosoverbindung der allgemeinen Formel II enthält, 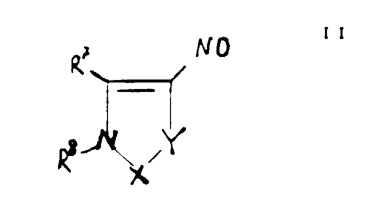 in der
X-Y NR⁵-CO oder N=CR⁶ bedeutet, wobei
R⁵ H, Alkyl, Alkyl substituiert durch Hydroxy, Carboxy, SO₃H, PO₃H₂, Dialkylphosphinyl
R⁶ H, Alkyl, Alkenyl, Alkoxy, Alkylthio, Aryl, Aralkyl, die jeweils durch Hydroxy, Dialkylphosphinyl, Carboxy, SO₃H, PO₃H₂, ein Salz eines dieser Säurereste oder/und Alkoxycarbonyl substituiert sein können; oder
Amino oder Amino, das durch einen oder zwei Alkylreste, die einen oder mehrere Hydroxy-, Carboxy- oder/und Alkoxycarbonylreste tragen können, substituiert ist, wobei, wenn Amino durch 2 Alkylreste substituiert ist, diese Reste auch zu einem Ring geschlossen sein können, der außer durch das N-Atom der Aminogruppe auch durch Sauerstoff, Schwefel oder ein weiteres Stickstoffatom unterbrochen sein kann oder Amino, oder Amino, das durch ein oder zwei Acylgruppen, Alkoxy-, oder / und Aralkyl- oder/und Aralkoxycarbonylgruppen, H₂N-CO, Alkyl-, Arylcarbamoylgruppen substituiert ist; oder Wasserstoff, Carboxy, Alkoxycarbonyl, Carboxamido oder Halogen ist und
R⁷ Alkyl, Thioalkyl oder Aralkyl, das durch Hydroxy, Carboxy, SO₃H oder PO₃H₂ substituiert sein kann, oder Amino,
oder Amino das durch eine oder zwei Alkylgruppen substituiert ist, die wiederum durch Hydroxy, Carboxy, SO₃H, Dialkylphosphinyl oder PO₃H₂ substituiert sein können, bedeutet, wobei mindestens R⁶ und/ oder R⁷ eine Aminogruppe darstellt
R⁸ eine Alkyl oder Aralkylgruppe darstellt, die durch Hydroxy, Carboxy, SO₃H oder PO₃H₂ substituiert sein kann, oder wobei
R⁷ und R⁸ eine gesättigte oder eine ungesättigte Kette mit 3 oder 4 Gliedern aus Stickstoffatomen oder aus Kohlenstoffatomen oder aus Kohlenstoffatomen und einem oder mehreren Stickstoff- oder Schwefelatomen darstellt, wobei Kohlenstoffatome substituiert sein können durch Alkyl, Alkoxy, Alkylthio, Hydroxy, Aralkyl, Aryl, Carboxy, Carboxamido, Alkoxycarbonyl, Cyano, Halogen, Amino, Amino, das durch einen oder zwei Alkylreste, die einen oder mehrere Hydroxy, Carboxy- oder / und Alkoxycarbonylreste tragen, substituiert ist, und wobei Stickstoffatome, die nicht über eine Doppelbindung gebunden sind, durch Alkyl oder Aralkyl, die substituiert sein können durch Hydroxy, SO₃H, PO₃H₂, Carboxy oder Dialkylphosphinyl, substituiert sind oder zwei benachbarte Kettensubstituenten eine Alkylengruppe bilden, die ihrerseits mit Aryl substituiert oder anelliert sein kann,
sowie entsprechende tautomere Formen und deren Salze.

19. Mittel gemäß einem der Ansprüche 16-18, dadurch gekennzeichnet, daß es als nicht-oxidatives Nachweisreagenz fur Iminoverbindungen ein chromogenes Kupplungsreagenz für Iminoverbindungen oder einen Leukofarbstoff enthält.

20. Mittel zur kolorimetrischen Bestimmung eines Analyten durch enzymatische Oxidation des Analyten enthaltend eine PQQ-abhängige Dehydrogenase und eine elektronenreiche aromatische Nitrosoverbindung, dadurch gekennzeichnet, daß die elektronenreiche aromatische Nitrosoverbindung eine Nitrosoanilinverbindung der Formel XIII ist, 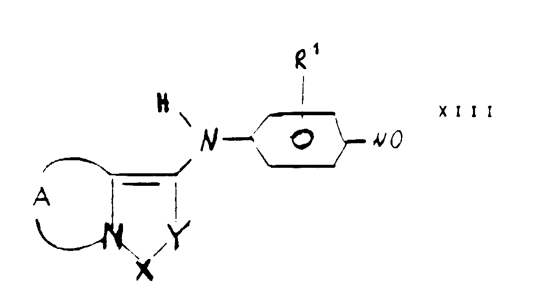 wobei
R¹ Wasserstoff, Hydroxy, Halogen, Alkoxy oder Alkylthio, Aryloxy, Arylthio, Alkyl, Alkyl substituiert durch Hydroxy, Carboxy, PO₃H₂, oder SO₃H, Amino, Amino, ein- oder mehrfach substituiert durch Alkyl, das wiederum durch Hydroxy, PO₃H₂ Dialkylphosphinyl, SO₃H oder Carboxy substituiert sein kann, bedeutet und
X-Y NR⁵-C0 oder N=CR⁶ darstellt, wobei
R⁵ H, Alkyl, Alkyl substituiert durch Hydroxy, Carboxy, SO₃H, PO₃H₂ oder Dialkylphosphinyl
R⁶ H, Alkyl, Alkenyl, Alkoxy, Alkylthio, Aryl, Arylthio, Aralkyl, die jeweils substituiert sein können durch Hydroxy, Dialkylphosphinyl, Carboxy, SO₃H, PO₃H₂, ein Salz eines dieser Säurereste oder/und Alkoxycarbonyl; oder
Amino, oder Amino, das durch einen oder zwei Alkylreste, die einen oder mehrere Hydroxy-, Carboxy- oder / und Alkoxycarbonylreste tragen können, substituiert ist, wobei, wenn Amino durch 2 Alkylreste substituiert ist, diese Reste auch zu einem Ring geschlossen sein können, der außer durch das N-Atom der Aminogruppe auch durch Sauerstoff, Schwefel oder ein weiteres Stickstoffatom unterbrochen sein kann, oder Amino, oder Amino, das durch ein oder zwei Acylgruppen, Alkoxy- oder/und Aralkoxycarbonylgruppen, H₂N-CO, Alkyl, Aralkyl oder / und Arylcarbamoylgruppen substituiert ist; oder Carboxy, Alkoxycarbonyl, Carboxamido oder Halogen ist und
A eine gesättigte oder ungesättigte Kette aus drei Gliedern mit einem Stickstoff- oder Schwefelatom und zwei Kohlenstoffatomen oder zwei Stickstoffatomen und einem Kohlenstoffatom darstellt, wobei Kohlenstoffatome substituiert sein können durch Alkyl, Alkoxy, Alkylthio, Hydroxy, Aralkyl, Aryl, Carboxy, Carboxamido, Alkoxycarbonyl, Cyano, Halogen, Amino, Amino, das durch einen oder zwei Alkylreste, die einen oder mehrere Hydroxy, Carboxy- oder / und Alkoxycarbonylreste tragen können, substituiert ist und wobei Stickstoffatome, die nicht über eine Doppelbindung gebunden sind, durch Wasserstoff, Alkyl, Alkyl, substituiert durch SO₃H, PO₃H₂, Carboxy oder Dialkylphosphinyl, oder durch Aralkyl substituiert sind oder zwei benachbarte Kettensubstituenten eine Alkylengruppe bilden, die ihrerseits eine Alkylengruppe bilden können, die ihrerseits mit Aryl substituiert oder anelliert sein kann, sowie entsprechende tautomere Formen und deren Salze.

21. Mittel gemäß Anspruch 20, dadurch gekennzeichnet, daß A mit dem benachbarten Heterocyklus einen Imidazol-, Triazol-, Benzimidazol-, Thiazol- oder Dihydroimidazol-Ring bildet.

22. Testträger enthaltend ein Mittel gemäß einem der Anspruche 16-21.

23. Nitrosoanilinverbindungen der Formel XIII, 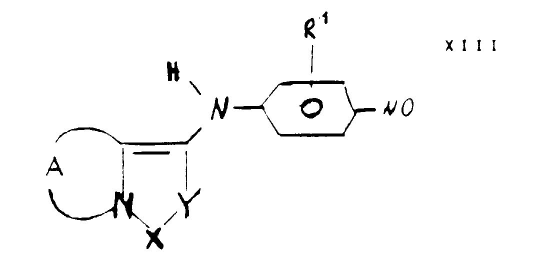 wobei
R¹ Wasserstoff, Hydroxy, Halogen, Alkoxy oder Arylthio, Alkyl, Alkyl substituiert durch Hydroxy, Carboxy, PO₃H₂, Alkyl, Dialkylphosphinyl oder SO₃H, Amino, Amino ein- oder mehrfach substituiert durch Alkyl, das wiederum durch Hydroxy, PO₃H₂, SO₃H oder Carboxy substituiert sein kann, bedeutet und
X-Y NR⁵-CO oder N=CR⁶ darstellt, wobei
R⁵ H, Alkyl, Alkyl substituiert durch Hydroxy, Carboxy, SO₃H, PO₃H₂ oder Dialkylphosphinyl
R⁶ H, Alkyl, Alkenyl, Alkoxy, Alkylthio, Aryl, Arylthio, Aralkyl, die jeweils durch Hydroxy, Dialkylphosphinyl, Carboxy, SO₃H, PO₃H₂, ein Salz eines dieser Säurereste oder/und Alkoxycarbonyl substituiert sein können; oder
Amino, oder Amino das durch einen oder zwei Alkylreste, die einen oder mehrere Hydroxy, Carboxy- oder / und Alkoxycarbonylreste tragen können, substituiert ist, wobei, wenn Amino durch 2 Alkylreste substituiert ist, diese Reste auch zu einem Ring geschlossen sein können, der außer durch das N-Atom der Aminogruppe auch durch Sauerstoff, Schwefel oder ein weiteres Stickstoffatom unterbrochen sein kann, oder Amino, oder Amino, das durch ein oder zwei Acylgruppen, Alkoxy- oder/und Aralkoxycarbonylgruppen, H₂N-CO, Alkyl-, Aralkyl-
oder/ und Arylcarbamoylgruppen substituiert ist; oder Wasserstoff, Carboxy, Alkoxycarbonyl, Carboxamido oder Halogen ist und
A eine gesättigte oder ungesättigte Kette aus drei Gliedern mit einem Stickstoff- oder Schwefelatom und zwei Kohlenstoffatomen oder zwei Stickstoffatomen und einem Kohlenstoffatom darstellt, wobei Kohlenstoffatome substituiert sein können durch Alkyl, Alkoxy, Alkylthio, Hydroxy, Aralkyl, Aryl, Carboxy, Carboxamido, Alkoxycarbonyl, Cyano, Halogen, Amino, Amino, das durch einen oder zwei Alkylreste, die einen oder mehrere Hydroxy, Carboxy- oder / und Alkoxycarbonylreste tragen können, substituiert ist, und wobei Stickstoffatome, die nicht über eine Doppelbindung gebunden sind, durch Wasserstoff, Alkyl, Alkyl substituiert durch SO₃H, PO₃H₂, Carboxyl, Dialkylphosphinyl oder Aralkyl substituiert sind oder zwei benachbarte Kettensubstituenten eine Alkylengruppe bilden, die ihrerseits mit Aryl substituiert oder anelliert sein kann, sowie entsprechende tautomere Formen und deren Salze.

24. Nitrosoanilinverbindungen gemäß Anspruch 23, dadurch gekennzeichnet, daß A mit dem benachbarten Heterocyklus einen Imidazol-, Triazol-, Benzimidazol-, Thiazol- oder Dihydroimidazol-Ring bildet.

25. Verwendung der Nitrosoanilinverbindungen gemäß einem der Ansprüche 23 oder 24 zur kolorimetrischen enzymatischen Bestimmung eines Analyten.

## Claims

1. Method for the colorimetric determination of an analyte by means of enzymatic oxidation of the analyte with an oxidoreductase in the presence of a direct electron acceptor from the group of the electron-rich aromatic nitroso compounds and determination of the reduced electron acceptor by colour formation as a measure of the amount of analyte, in which the electron-rich aromatic nitroso compound is reduced with concomitant oxidation of the analyte in the presence of a PQQ-dependent dehydrogenase to an imino compound wherein the imino compound is detected by colour formation via its intrinsic colour without further enzymatic reduction to an aromatic amine.

2. Method for the colorimetric determination of an analyte by means of enzymatic oxidation of the analyte with an oxidoreductase in the presence of a direct electron acceptor from the group of the electron-rich aromatic nitroso compounds and determination of the reduced electron acceptor by colour formation as a measure of the amount of analyte, in which the electron-rich aromatic nitroso compound is reduced with concomitant oxidation of the analyte in the presence of a PQQ-dependent dehydrogenase to an imino compound wherein the imino compound is detected by colour formation without further enzymatic reduction to an aromatic amine by reaction with a non-oxidative chromogenic detection reagent.

3. Method as claimed in one of the claims 1 or 2, wherein a compound of formula I is used as the aromatic nitroso compound 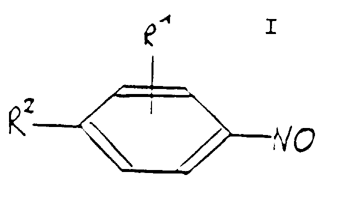
in which R¹
denotes hydrogen, hydroxy, alkyl, alkyl substituted by hydroxy, COOH or PO₃H₂, SO₃H, alkoxy, alkylthio, aryloxy, arylthio, halogen, or amino, amino which is substituted once or several times by alkyl, alkyl substituted by hydroxy, PO₃H₂, dialkylphosphinyl, SO₃H or CO₂H,
and R²
denotes a hydroxy group, alkylthio, alkoxy, aryloxy, arylthio or alkylthio group in which the alkyl residue is in turn substituted by a hydroxy group, an alkoxy group, an amino group, an amino group substituted once or several times by alkyl, PO₃H₂, SO₃H or CO₂H as such or in the form of a salt such as ammonium, alkali or alkaline earth salts, or it denotes an amino group NR³R⁴,
in which R³ and R⁴ can be the same or different and denote hydrogen, an aryl or alkyl group which in turn can be substituted by a hydroxy, alkoxy, hydroxyalkoxy, a polyalkoxy group, a hydroxy-substituted polyalkoxy group, PO₃H₂, SO₃H, COOH as such or in the form of a salt or by an amino group or an amino group which is substituted once or several times by alkyl, or
in which R³ and R⁴ can represent an alkylene residue or an alkylene residue which is interrupted if desired by oxygen, sulphur or nitrogen wherein nitrogen is substituted by an alkyl, hydroxyalkyl, hydroxyalkoxyalkyl, alkoxyhydroxyalkyl, alkoxycarbonylalkyl, dioxanylylalkyl or polyalkoxyalkyl residue each of which can in turn be substituted by a hydroxy residue in the alkyl moiety, or
if R¹ is in the ortho position in relation to NR³R⁴, R³ or R⁴ also together with R¹ can represent an alkylene residue.

4. Method as claimed in one of the claims 1 or 2, wherein a compound of formula II is used as the aromatic nitroso compound. 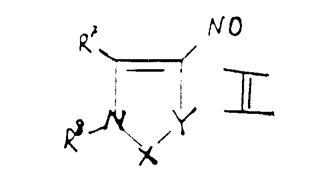 in which
X-Y denotes NR⁵-CO or N=CR⁶, wherein
R⁵ denotes H, alkyl, alkyl substituted by hydroxy, carboxy, SO₃H, PO₃H₂, dialkylphosphinyl
R⁶ denotes H, alkyl, alkenyl, alkoxy, alkylthio, aryl, arylthio, aralkyl, each of which can be substituted by hydroxy, dialkylphosphinyl, carboxy, SO₃H, PO₃H₂, a salt of one of these acid residues or/and alkoxycarbonyl; or
amino or amino which is substituted by one or two alkyl residues which can carry one or several hydroxy, carboxy, or/and alkoxycarbonyl residues, wherein if the amino is substituted by 2 alkyl residues, these residues can also be linked to form a ring which apart from the N atom of the amino group can also be interrupted by oxygen, sulphur or a further nitrogen atom, or amino, or amino which is substituted by one or two acyl groups, alkoxy or/and aralkoxycarbonyl groups, H₂N-CO, alkyl, aralkyl, or/and arylcarbamoyl groups; or hydrogen, carboxy, alkoxycarbonyl, carboxamido or halogen and
R⁷ represents alkyl, thioalkyl or aralkyl which can be substituted by hydroxy, carboxy, SO₃H or PO₃H₂ or amino, or amino which is substituted by one or two alkyl groups which in turn can be substituted by hydroxy, carboxy, SO₃H, dialkylphosphinyl or PO₃H₂, wherein at least R⁶ and/or R⁷ represent an amino group and
R⁸ represents an alkyl or aralkyl group which can be substituted by hydroxy, carboxy, SO₃H or PO₃H₂
or in which
R⁷ and R⁸ represent a saturated or unsaturated chain with 3 or 4 members of nitrogen atoms or of carbon atoms or of carbon atoms and one or several nitrogen or sulphur atoms wherein carbon atoms can be substituted by alkyl, alkoxy, alkylthio, hydroxy, aralkyl, aryl, carboxy, carboxamido, alkoxycarbonyl, cyano, halogen, amino, amino which is substituted by one or two alkyl residues which can carry one or several hydroxy, carboxy or/and alkoxycarbonyl residues and wherein nitrogen atoms that are not bound via a double bond are substituted by alkyl or aralkyl or dialkyl-phosphinyl, or two adjacent chain substituents form an alkylene group which in turn can be substituted by aryl or anellated
as well as if desired the corresponding tautomeric forms and their salts.

5. Method as claimed in claim 4, wherein a compound in which R⁷ and R⁸ form a saturated or unsaturated chain is used as the aromatic nitroso compound.

6. Method as claimed in one of the claims 1 or 2, wherein a compound of formula III - XII is used as the aromatic nitroso compound, 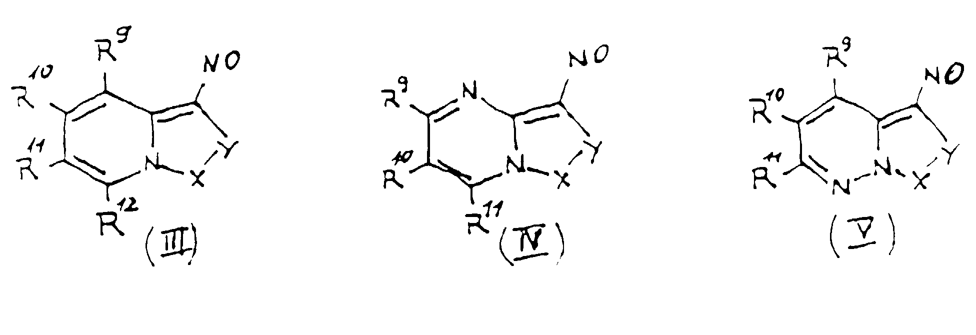 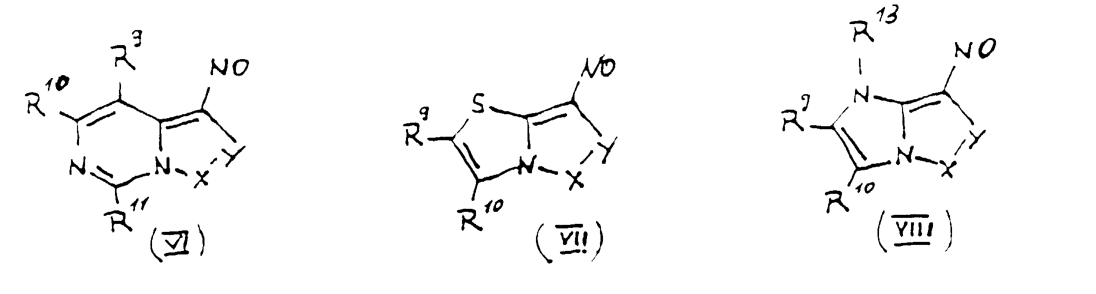 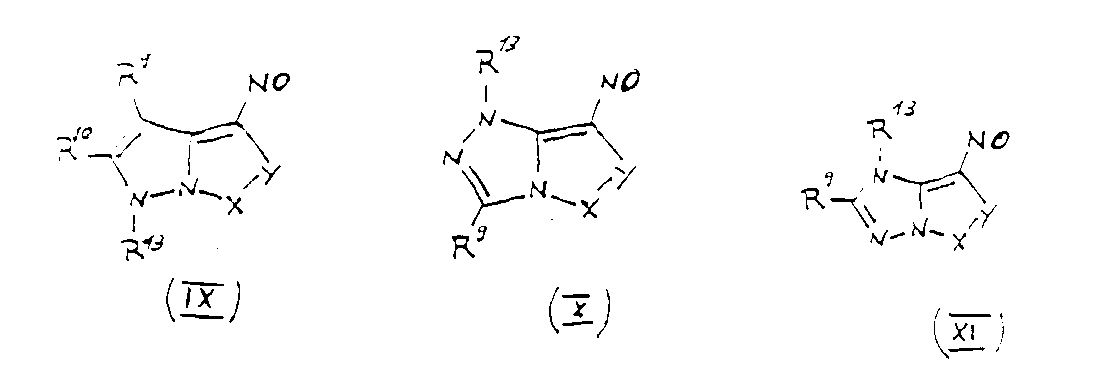 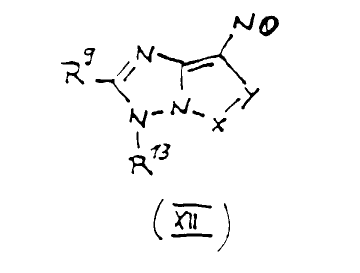 in which
R⁹, R¹⁰, R¹¹ and R¹² are the same or different and represent hydrogen, hydroxy, alkyl, alkoxy, alkylthio, aralkyl, aryl, carboxy, alkoxycarbonyl, carboxamido, cyano, amino, amino which is substituted by one or two alkyl residues that can carry one or several hydroxy, carboxy or/and alkoxycarbonyl residues or they represent halogen, wherein two adjacent residues can form an alkylene group which in turn can be substituted by aryl or anellated
and R¹³ represents alkyl or aralkyl which can be substituted by hydroxy, carboxy, SO₃H, PO₃H₂ or dialkylphosphinyl.

7. Method as claimed in claim 2, wherein the non-oxidative chromogenic detection reagent is a coupling reagent for imino compounds.

8. Method as claimed in claim 7, wherein a phenol, naphthol, aniline or naphthylamine derivative is used as the coupling reagent.

9. Method as claimed in claim 7, wherein 2,4,6-tribromo-3-hydroxybenzoic acid, 2,4-dichloro-1-naphthol or l-naphthol-4-sulfonic acid is used as the coupling reagent.

10. Method as claimed in claim 2, wherein the imino compound is detected by oxidation of a leuco dye to a dye.

11. Method as claimed in claim 10, wherein diarylimidazoles, triarylimidazoles or naphthylamines are used as the leuco dye.

12. Method as claimed in claim 1, wherein an aromatic nitrosoaniline compound of the general formula XIII is used 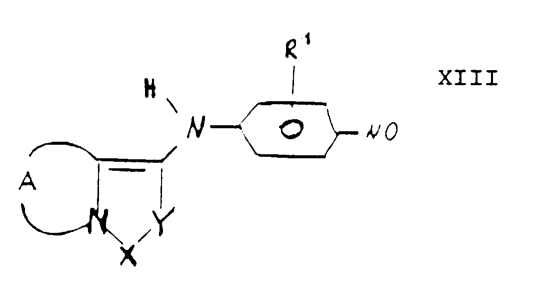 in which
R¹ denotes hydrogen, hydroxy, halogen, alkoxy, alkylthio, aryloxy or arylthiol, alkyl, alkyl substituted by hydroxy, carboxy, PO₃H₂ or SO₃H, amino, amino substituted once or several times by alkyl which in turn can be substituted by hydroxy, PO₃H₂, dialkylphosphinyl, SO₃H or carboxy and
X-Y represents NR⁵-CO or N=CR⁶, wherein
R⁵ is H, alkyl, alkyl substituted by hydroxy, carboxy, SO₃H, PO₃H₂, dialkylphosphinyl and
R⁶ is H, alkyl, alkenyl, alkoxy, alkylthio, aryl, arylthio, aralkyl, each of which can be substituted by hydroxy, carboxy, SO₃H, PO₃H₂, a salt of one of these acid residues or/and alkoxycarbonyl; or amino or
amino which is substituted by one or two alkyl residues which can carry one or several hydroxy, carboxy or/and alkoxycarbonyl residues, wherein if the amino is substituted by 2 alkyl residues, these residues can also be linked to form a ring which apart from the N atom of the amino group can also be interrupted if desired by oxygen, sulphur or a further nitrogen atom, or amino or amino which is substituted by one or two acyl groups, alkoxy or/and aralkoxycarbonyl groups, H₂N-CO, alkyl, aralkyl or/and arylcarbamoyl groups; or carboxy, alkoxycarbonyl, carboxamido or halogen and
A represents a saturated or unsaturated chain consisting of three members with a nitrogen or sulphur atom and two carbon atoms or two nitrogen atoms and one carbon atom, wherein the carbon atoms can be substituted by alkyl, alkoxy, alkylthio, hydroxy, aralkyl, aryl, carboxy, carboxamido, alkoxycarbonyl, cyano, halogen, amino, amino which is substituted by one or two alkyl residues that can carry one or several hydroxy, carboxy or/and alkoxycarbonyl residues and wherein nitrogen atoms that are not bound via a double bond are substituted by hydrogen, alkyl, alkyl substituted by SO₃H, PO₃H₂, carboxy or dialkylphosphinyl, or by aralkyl or two adjacent chain substituents can form an alkylene group which in turn can be substituted by aryl or anellated, as well as corresponding tautomeric forms and their salts,
which reacts enzymatically to form a coloured quinone diimine that is determined colorimetrically as a measure of the amount of the analyte.

13. Method as claimed in claim 12, wherein A forms an imidazole, triazole, benzimidazole, thiazole or dihydroimidazole ring with the adjacent heterocycle.

14. Method as claimed in claim 12 or 13, wherein the aromatic nitroso compound is used at a concentration of more than 10⁻³ mol/l.

15. Method as claimed in one of the claims 1-14, wherein glucose dye oxidoreductase is used as the PQQ-dependent dehydrogenase for the determination of glucose.

16. Agent for the colorimetric determination of an analyte by enzymatic oxidation of the analyte containing a PQQ-dependent dehydrogenase and an electron-rich aromatic nitroso compound, wherein it in addition contains a colour-forming, non-oxidative detection reagent for imino compounds.

17. Agent as claimed in claim 16, wherein it contains a nitroso compound of the general formula I, 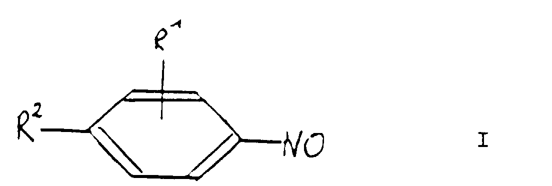
in which R¹
denotes hydrogen, hydroxy, alkyl, alkyl substituted by hydroxy, COOH, PO₃H₂ or SO₃H, alkoxy, alkylthio, aryloxy, arylthio, halogen, or amino, amino which is substituted by hydroxy, PO₃H₂, dialkylphosphinyl, SO₃H or CO₂H,
and R²
denotes a hydroxy group, alkoxy, aryloxy, arylthio or alkylthio group in which the alkyl residue in turn can be substituted by a hydroxy group, an alkoxy group, an amino group, an amino group substituted once or several times by alkyl, PO₃H₂, SO₃H or CO₂H as such or in the form of a salt as ammonium, alkali or alkaline earth salts or denotes an amino group NR³R⁴,
in which R³ and R⁴ can be the same or different and denote hydrogen, an aryl or alkyl group which can be substituted in turn by a hydroxy, alkoxy, hydroxyalkoxy, polyalkoxy group, a polyalkoxy group substituted by hydroxy, PO₃H₂, SO₃H or CO₂H as such or in the form of a salt or by an amino group or an amino group which can be substituted once or several times by alkyl, or
in which R³ and R⁴ can represent an alkylene residue which can be interrupted by oxygen, sulphur or nitrogen wherein nitrogen is substituted by an alkyl, hydroxyalkyl, hydroxyalkoxyalkyl, alkoxyhydroxyalkyl, alkoxycarbonylalkyl, dioxanylylalkyl or polyalkoxyalkyl residue each of which can in turn be substituted by a hydroxy residue in the alkyl moiety, or
if R¹ is in the ortho position in relation to NR³R⁴, R³ or R⁴ also together with R¹ can represent an alkylene residue.

18. Agent as claimed in claim 16, wherein it contains a nitroso compound of the general formula II, 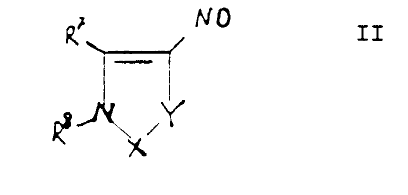 in which
X-Y denotes NR⁵-CO or N=CR⁶, wherein
R⁵ is H, alkyl, alkyl substituted by hydroxy, carboxy, SO₃H, PO₃H₂, dialkylphosphinyl
R⁶ is H, alkyl, alkenyl, alkoxy, alkylthio, aryl, aralkyl, each of which can be substituted by hydroxy, dialkylphosphinyl, carboxy, SO₃H, PO₃H₂, a salt of one of these acid residues or/and alkoxycarbonyl; or
amino or amino which is substituted by one or two alkyl residues which can carry one or several hydroxy, carboxy or/and alkoxycarbonyl residues, wherein if the amino is substituted by 2 alkyl residues, these residues can also be linked to form a ring which apart from the N atom of the amino group can also be interrupted by oxygen, sulphur or a further nitrogen atom, or amino, or amino which is substituted by one or two acyl groups, alkoxy or/and aralkyl or/and aralkoxycarbonyl groups, H₂N-CO, alkyl, arylcarbamoyl groups; or it is hydrogen, carboxy, alkoxycarbonyl, carboxamido or halogen and
R⁷ denotes alkyl, thioalkyl or aralkyl which can be substituted by hydroxy, carboxy, SO₃H or PO₃H₂ or amino,
or amino which is substituted by one or two alkyl groups which in turn can be substituted by hydroxy, carboxy, SO₃H, dialkylphosphinyl or PO₃H₂, wherein at least R⁶ and/or R⁷ represent an amino group,
R⁸ represents an alkyl or aralkyl group which can be substituted by hydroxy, carboxy, SO₃H or PO₃H₂ or in which
R⁷ and R⁸ represent a saturated or unsaturated chain with 3 or 4 members composed of nitrogen atoms or of carbon atoms or of carbon atoms and one or several nitrogen or sulphur atoms wherein carbon atoms can be substituted by alkyl, alkoxy, alkylthio, hydroxy, aralkyl, aryl, carboxy, carboxamido, alkoxycarbonyl, cyano, halogen, amino, amino which is substituted by one or two alkyl residues carrying one or several hydroxy, carboxy or/and alkoxycarbonyl residues and wherein nitrogen atoms that are not bound via a double bond can be substituted by alkyl or aralkyl which can be substituted by hydroxy, SO₃H, PO₃H₂, carboxy or dialkylphosphinyl, or two adjacent chain substituents form an alkylene group which in turn can be substituted by aryl or anellated
as well as the corresponding tautomeric forms and their salts.

19. Agent as claimed in one of the claims 16-18, wherein it contains a chromogenic coupling reagent for imino compounds or a leuco dye as the non-oxidative detection reagent for imino compounds.

20. Agent for the colorimetric determination of an analyte by enzymatic oxidation of the analyte containing a PQQ-dependent dehydrogenase and an electron-rich aromatic nitroso compound, wherein the electron-rich aromatic nitroso compound is a nitrosoaniline compound of formula XIII, 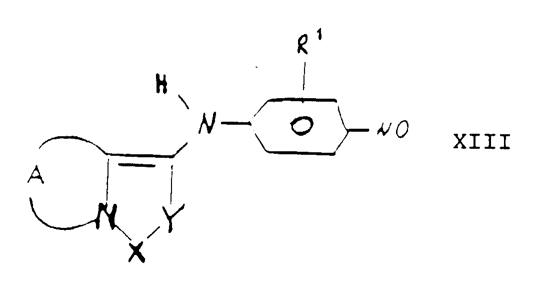 in which
R¹ denotes hydrogen, hydroxy, halogen, alkoxy or alkylthio, aryloxy, arylthio, alkyl, alkyl substituted by hydroxy, carboxy, PO₃H₂ or SO₃H, amino, amino substituted once or several times by alkyl which in turn can be substituted by hydroxy, PO₃H₂, dialkylphosphinyl, SO₃H or carboxy, and
X-Y represents NR⁵-CO or N=CR⁶, wherein
R⁵ is H, alkyl, alkyl substituted by hydroxy, carboxy, SO₃H, PO₃H₂ or dialkylphosphinyl R⁶ is H, alkyl, alkenyl, alkoxy, alkylthio, aryl, arylthio, aralkyl, each of which can be substituted by hydroxy, dialkyl-phosphinyl, carboxy, SO₃H, PO₃H₂, a salt of one of these acid residues or/and alkoxycarbonyl; or
amino, amino which is substituted by one or two alkyl residues which can carry one or several hydroxy, carboxy or/and alkoxycarbonyl residues, wherein if the amino is substituted by 2 alkyl residues, these residues can also be linked to form a ring which, apart from the N atom of the amino group, can also be interrupted by oxygen, sulphur or a further nitrogen atom, or amino, amino which is substituted by one or two acyl groups, alkoxy or/and aralkoxycarbonyl groups, H₂N-CO, alkyl, aralkyl or/and arylcarbamoyl groups; or carboxy, alkoxycarbonyl, carboxamido or halogen and
A represents a saturated or unsaturated chain consisting of three members with a nitrogen or sulphur atom and two carbon atoms or two nitrogen atoms and one carbon atom, wherein the carbon atoms can be substituted by alkyl, alkoxy, alkylthio, hydroxy, aralkyl, aryl, carboxy, carboxamido, alkoxycarbonyl, cyano, halogen, amino, amino which is substituted by one or two alkyl residues which can carry one or several hydroxy, carboxy or/and alkoxycarbonyl residues and wherein nitrogen atoms that are not bound via a double bond are substituted by hydrogen, alkyl, alkyl substituted by SO₃H, PO₃H₂, carboxy or dialkylphosphinyl, or by aralkyl or two adjacent chain substituents form an alkylene group which in turn can form an alkylene group which in turn can be substituted by aryl or anellated, as well as corresponding tautomeric forms and their salts.

21. Agent as claimed in claim 20, wherein A forms an imidazole, triazole, benzimidazole, thiazole or dihydroimidazole ring with the adjacent heterocycle.

22. Test carrier containing an agent as claimed in one of the claims 16-21.

23. Nitrosoaniline compounds of formula XIII, 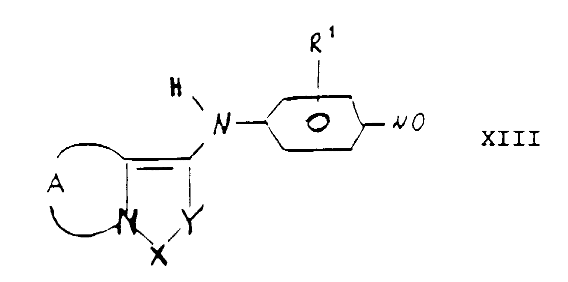 in which
R¹ denotes hydrogen, hydroxy, halogen, alkoxy or arylthio, alkyl, alkyl substituted by hydroxy, carboxy, PO₃H₂, alkyl, dialkylphosphinyl or SO₃H, amino, amino substituted once or several times by alkyl which in turn can be substituted by hydroxy, PO₃H₂, SO₃H or carboxy and
X-Y represents NR⁵-CO or N=CR⁶, in which
R⁵ is H, alkyl, alkyl substituted by hydroxy, carboxy, SO₃H, PO₃H₂ or dialkylphosphinyl
R⁶ is H, alkyl, alkenyl, alkoxy, alkylthio, aryl, arylthio, aralkyl, each of which can be substituted by hydroxy, dialkylphosphinyl, carboxy, SO₃H, PO₃H₂, a salt of one of these acid residues or/and alkoxycarbonyl; or
amino, amino which is substituted by one or two alkyl residues which can carry one or several hydroxy, carboxy or/and alkoxycarbonyl residues, wherein if amino is substituted by 2 alkyl residues, these residues can also be linked to form a ring which, apart from the N atom of the amino group, can also be interrupted by oxygen, sulphur or a further nitrogen atom, or amino, or amino which is substituted by one or two acyl groups, alkoxy or/and aralkoxycarbonyl groups, H₂N-CO, alkyl, aralkyl or/and arylcarbamoyl groups; or hydrogen, carboxy, alkoxycarbonyl, carboxamido or halogen and
A represents a saturated or unsaturated chain composed of three members with a nitrogen or sulphur atom and two carbon atoms or two nitrogen atoms and one carbon atom, wherein the carbon atoms can be substituted by alkyl, alkoxy, alkylthio, hydroxy, aralkyl, aryl, carboxy, carboxamido, alkoxycarbonyl, cyano, halogen, amino, amino which is substituted by one or two alkyl residues which can carry one or several hydroxy, carboxy or/and alkoxycarbonyl residues and wherein nitrogen atoms that are not bound via a double bond are substituted by hydrogen, alkyl, alkyl substituted by SO₃H, PO₃H₂, carboxyl, dialkylphosphinyl or aralkyl or two adjacent chain substituents form an alkylene group which in turn can be substituted by aryl or anellated, as well as corresponding tautomeric forms and their salts.

24. Nitrosoaniline compounds as claimed in claim 23, wherein A forms an imidazole, triazole, benzimidazole, thiazole or dihydroimidazole ring with the adjacent heterocycle.

25. Use of the nitrosoaniline compounds as claimed in one of the claims 23 or 24 for the colorimetric enzymatic determination of an analyte.

## Revendications

1. Procédé pour la détermination colorimétrique d'un analyte au moyen d'une oxydation enzymatique de l'analyte avec une oxydoréductase en présence d'un accepteur d'électrons directs choisi parmi le groupe des composés nitroso aromatiques riches en électrons et au moyen d'une détermination de l'accepteur d'électrons sous forme réduite par coloration comme mesure pour la quantité de l'analyte, dans lequel on soumet le composé nitroso aromatique riche en électrons, en procédant à une oxydation de l'analyte en présence d'une déshydrogénase dépendant de la PQQ, à une réduction pour obtenir un composé imino, caractérisé en ce qu'on détecte le composé imino par coloration via sa teinte propre, sans le soumettre à une réduction enzymatique ultérieure pour obtenir l'amine aromatique .

2. Procédé pour la détermination colorimétrique d'un analyte au moyen d'une oxydation enzymatique de l'analyte avec une oxydoréductase en présence d'un accepteur d'électrons directs choisi parmi le groupe des composés nitroso aromatiques riches en électrons et au moyen d'une détermination de l'accepteur d'électrons sous forme réduite par coloration comme mesure pour la quantité de l'analyte, dans lequel on soumet le composé nitroso aromatique riche en électrons, en procédant à une oxydation de l'analyte en présence d'une déshydrogénase dépendant de la PQQ, à une réduction pour obtenir un composé imino, caractérisé en ce qu'on détecte le composé imino par coloration en le faisant réagir avec un réactif de détection chromogène de type non oxydant, sans le soumettre à une réduction enzymatique ultérieure pour obtenir l'amine aromatique,.

3. Procédé selon l'une quelconque des revendications 1 ou 2, caractérisé en ce qu'on met en oeuvre, à titre de composé nitroso aromatique, un composé répondant à la formule I 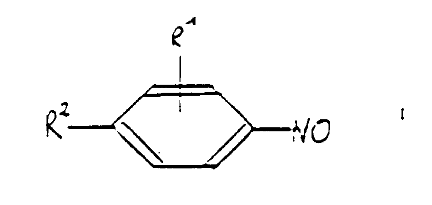 dans laquelle
R¹ représente un atome d'hydrogène, un groupe hydroxyle, un groupe alkyle, un groupe alkyle substitué par un groupe hydroxyle, par un groupe COOH, par un groupe PO₃H₂ ou par un groupe SO₃H, un groupe alcoxy, un groupe alkylthio, un groupe aryloxy, un groupe arylthio, un atome d'halogène ou un groupe amino ou encore un groupe amino substitué qui est substitué une ou plusieurs fois par un groupe alkyle, par un groupe alkyle substitué par un groupe hydroxyle, par un groupe PO₃H₂, par un groupe dialkylphosphinyle, par un groupe SO₃H ou par un groupe CO₂H, et
R² représente un groupe hydroxyle, un groupe alkylthio, un groupe alcoxy, un groupe aryloxy, un groupe arylthio ou un groupe alkylthio, le radical alkyle étant substitué pour sa part par un groupe hydroxyle, par un groupe alcoxy, par un groupe amino, par un groupe amino substitué une ou plusieurs fois par un groupe alkyle, par un groupe PO₃H₂, par un groupe SO₃H ou par un groupe CO₂H, comme tels ou sous forme de sels, tels que des sels d'ammonium, de métaux alcalins ou de métaux alcalino-terreux, ou encore représente un groupe amino NR³R⁴,
dans lequel R³ et R⁴ peuvent être identiques ou différents et représentent un atome d'hydrogène, un groupe aryle ou un groupe alkyle qui peut être, quant à lui, substitué par un groupe hydroxyle, par un groupe alcoxy, par un groupe hydroxyalcoxy, par un groupe polyalcoxy, par un groupe polyalcoxy portant un ou plusieurs substituants hydroxyle, par un groupe PO₃H₂, par un groupe SO₃H, par un groupe COOH, comme tels ou sous forme de sels, ou encore par un groupe amino ou par un groupe amino substitué une ou plusieurs fois par un groupe alkyle, ou
dans lequel R³ et R⁴ peuvent représenter un radical alkylène ou encore un radical alkylène qui est interrompu par un atome d'oxygène, par un atome de soufre ou par un atome d'azote, dans lequel l'atome d'azote est substitué par un radical alkyle, par un radical hydroxyalkyle, par un radical hydroxyalcoxyalkyle, par un radical alcoxyhydroxyalkyle, par un radical alcoxycarbonylalkyle, par un radical dioxanylylalkyle ou par un radical polyalcoxyalkyle qui peut être, quant à lui, substitué respectivement dans la fraction alkyle par un radical hydroxyle, ou
lorsque R¹ se trouve en position ortho par rapport à NR³R⁴, R³ ou R⁴ peut représenter également, de manière conjointe avec R¹, un radical alkylène.

4. Procédé selon l'une quelconque des revendications 1 ou 2, caractérisé en ce qu'on met en oeuvre, à titre de composé nitroso aromatique, un composé de formule II 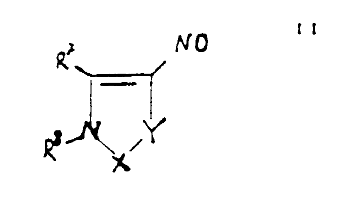 dans laquelle
X-Y représente NR⁵-CO ou N=CR⁶ où
R⁵ représente un atome d'hydrogène, un groupe alkyle, un groupe alkyle substitué par un groupe hydroxyle, un groupe carboxyle, un groupe SO₃H, un groupe PO₃H₂, un groupe dialkylphosphinyle,
R⁶ représente un atome d'hydrogène, un groupe alkyle, un groupe alcényle, un groupe alcoxy, un groupe alkylthio, un groupe aryle, un groupe arylthio, un groupe aralkyle qui peuvent être substitués respectivement par un groupe hydroxyle, par un groupe dialkylphosphinyle, par un groupe carboxyle, par un groupe SO₃H, par un groupe PO₃H₂, par un sel d'un de ces radicaux acides et/ou par un groupe alcoxycarbonyle; ou bien représente un groupe amino ou encore un groupe amino qui est substitué par un ou deux radicaux alkyle qui peuvent porter un ou plusieurs radicaux hydroxyle, un ou plusieurs radicaux carboxyle et/ou un ou plusieurs radicaux alcoxycarbonyle, dans lequel, lorsque le groupe amino est substitué par deux radicaux alkyle, ces radicaux peuvent également être fermés pour former un cycle qui peut être interrompu, outre par l'atome d'azote du groupe amino, également par un atome d'oxygène, par un atome de soufre ou par un atome d'azote supplémentaire; ou représente un groupe amino qui est substitué par un ou deux groupes acyle, par un ou deux groupes alcoxy et/ou par un ou deux groupes aralcoxycarbonyle, par un ou deux groupes H₂N-CO, par un ou deux groupes alkyle, par un ou deux groupes aralkyle et/ou par un ou deux groupes arylcarbamoyle; ou encore représente un atome d'hydrogène, un groupe carboxyle, un groupe alcoxycarbonyle, un groupe carboxamido ou un atome d'halogène, et
R⁷ représente un groupe alkyle, un groupe thioalkyle ou un groupe aralkyle qui peut être substitué par un groupe hydroxyle, par un groupe carboxyle, par un groupe SO₃H ou par un groupe PO₃H₂, ou représente un groupe amino, ou encore un groupe amino qui est substitué par un ou deux groupes alkyle qui peuvent être à nouveau substitués par un groupe hydroxyle, par un groupe carboxyle, par un groupe SO₃H, par un groupe dialkylphosphinyle ou par un groupe PO₃H₂, dans laquelle au moins R⁶ et/ou R⁷ représentent un groupe amino,
R⁸ représente un groupe alkyle ou un groupe aralkyle qui peut être substitué par un groupe hydroxyle, par un groupe carboxyle, par un groupe SO₃H ou par un groupe PO₃H₂,
ou dans laquelle
R⁷ et R⁸ représentent une chaîne saturée ou une chaîne insaturée contenant 3 ou 4 membres constitués par des atomes d'azote ou par des atomes de carbone ou encore par des atomes de carbone et par un ou plusieurs atomes d'azote ou par un ou plusieurs atomes de soufre, les atomes de carbone pouvant être substitués par un groupe alkyle, par un groupe alcoxy, par un groupe alkylthio, par un groupe hydroxyle, par un groupe aralkyle, par un groupe aryle, par un groupe carboxyle, par un groupe carboxamido, par un groupe alcoxycarbonyle, par un groupe cyano, par un atome d'halogène, par un groupe amino, par un groupe amino qui est substitué par un ou deux radicaux alkyle qui peuvent porter un ou plusieurs radicaux hydroxyle, un ou plusieurs radicaux carboxyle et/ou un ou plusieurs radicaux alcoxycarbonyle, et dans laquelle les atomes d'azote qui ne sont pas liés via une liaison double sont substitués par un groupe alkyle ou par un groupe aralkyle ou encore par un groupe dialkylphosphinyle, ou bien deux substituants de chaînes voisins forment un groupe alkylène qui peut être, quant à lui, substitué ou condensé avec un groupe aryle,
ainsi que ses formes tautomères correspondantes et ses sels.

5. Procédé selon la revendication 4, caractérisé en ce qu'on met en oeuvre, à titre de composé nitroso aromatique, un composé dans lequel R⁷ et R⁸ forment une chaîne saturée ou insaturée.

6. Procédé selon l'une quelconque des revendications 1 ou 2, caractérisé en ce qu'on met en oeuvre, à titre de composé nitroso aromatique, un composé répondant aux formules III-XII 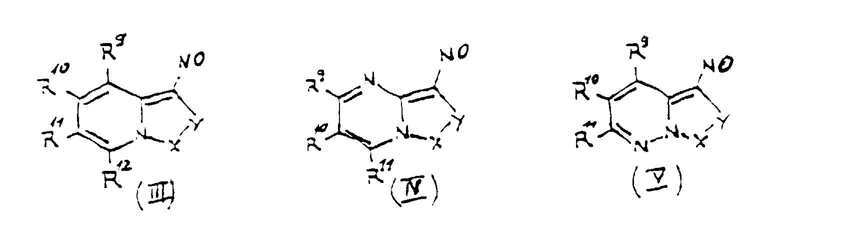 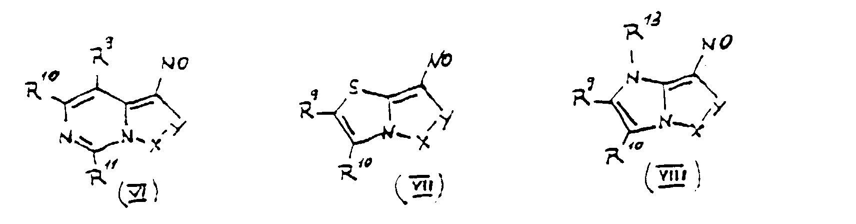 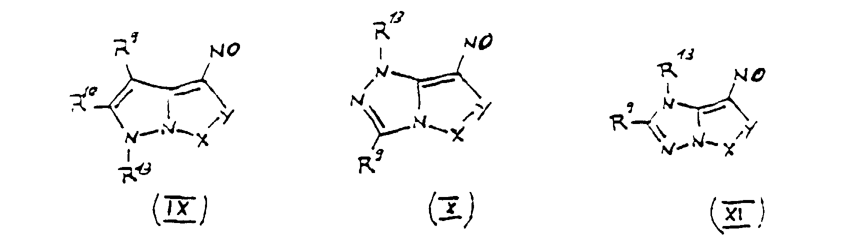 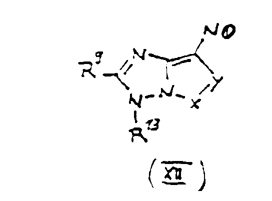
dans lesquelles R⁹, R¹⁰, R¹¹ et R¹², qui sont identiques ou différents, représentent un atome d'hydrogène, un groupe hydroxyle, un groupe alkyle, un groupe alcoxy, un groupe alkylthio, un groupe aralkyle, un groupe aryle, un groupe carboxyle, un groupe alcoxycarbonyle, un groupe carboxamido, un groupe cyano, un groupe amino, un groupe amino qui est substitué par un ou deux radicaux alkyle qui peuvent porter un ou plusieurs radicaux hydroxyle, un ou plusieurs radicaux carboxyle et/ou un ou plusieurs radicaux alcoxycarbonyle, dans lesquelles deux radicaux voisins peuvent former un groupe alkylène qui, pour sa part, peut être substitué ou condensé avec un groupe aryle, et
R¹³ représente un groupe alkyle ou un groupe aralkyle qui peut être substitué par un groupe hydroxyle, par un groupe carboxyle, par un groupe SO₃H, par un groupe PO₃H₂ ou par un groupe dialkylphosphinyle.

7. Procédé selon la revendication 2, caractérisé en ce que le réactif de détection chromogène de type non oxydant est un réactif de couplage pour des composés imino.

8. Procédé selon la revendication 7, caractérisé en ce qu'on met en oeuvre, à titre de réactif de couplage, un dérivé de phénol, un dérivé de naphtol, un dérivé d'aniline ou un dérivé de naphtylamine.

9. Procédé selon la revendication 7, caractérisé en ce qu'on met en oeuvre, à titre de réactif de couplage, l'acide 2,4,6-tribromo-3-hydroxybenzoïque, 2,4-dichloro-1-naphthol ou l'acide 1-naphthol-4-sulfonique.

10. Procédé selon la revendication 2, caractérisé en ce qu'on détecte le composé imino par oxydation d'un colorant leuco pour obtenir un colorant.

11. Procédé selon la revendication 10, caractérisé en ce qu'on utilise, à titre de colorant leuco, des diarylimidazoles, des triarylimidazoles ou des naphtylamines.

12. Procédé selon la revendication 1, caractérisé en ce qu'on met en oeuvre un composé de nitrosoaniline aromatique répondant à la formule générale XIII 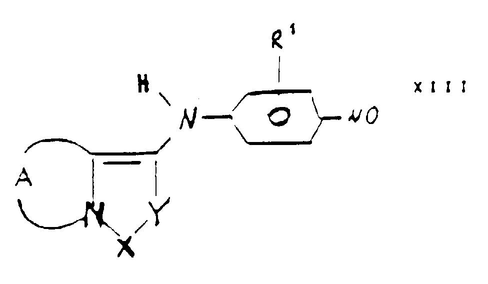 dans laquelle
R¹ représente un atome d'hydrogène, un groupe hydroxyle, un atome d'halogène, un groupe alcoxy, un groupe alkylthio, un groupe aryloxy ou un groupe arylthio, un groupe alkyle, un groupe alkyle substitué par un groupe hydroxyle, par un groupe carboxyle, un groupe PO₃H₂ ou un groupe SO₃H, un groupe amino, un groupe amino substitué une ou plusieurs fois par un groupe alkyle qui peut être à nouveau substitué par un groupe hydroxyle, par un groupe PO₃H₂, par un groupe dialkylphosphinyle, par un groupe SO₃H ou par un groupe carboxyle, et
X-Y représente NR⁵-CO ou N=CR⁶ où
R⁵ représente un atome d'hydrogène, un groupe alkyle, un groupe alkyle substitué par un groupe hydroxyle, par un groupe carboxyle, par un groupe SO₃H, par un groupe PO₃H₂, par un groupe dialkylphosphinyle,
R⁶ représente un atome d'hydrogène, un groupe alkyle, un groupe alcényle, un groupe alcoxy, un groupe alkylthio, un groupe aryle, un groupe arylthio, un groupe aralkyle qui peuvent être substitués respectivement par un groupe hydroxyle, par un groupe carboxyle, par un groupe SO₃H, par un groupe PO₃H₂, par un sel d'un de ces radicaux acides et/ou par un groupe alcoxycarbonyle; ou bien représente un groupe amino ou encore un groupe amino qui est substitué par un ou deux radicaux alkyle qui peuvent porter un ou plusieurs radicaux hydroxyle, un ou plusieurs radicaux carboxyle et/ou un ou plusieurs radicaux alcoxycarbonyle, dans lesquelles, lorsque le groupe amino est substitué par deux radicaux alkyle, ces radicaux peuvent également être fermés pour former un cycle qui peut être interrompu, outre par l'atome d'azote du groupe amino, également par un atome d'oxygène, par un atome de soufre ou par un atome d'azote supplémentaire; ou encore un groupe amino qui est substitué par un ou deux groupes acyle, par un ou deux groupes alcoxy et/ou par un ou deux groupes aralcoxycarbonyle, par un ou deux groupes H₂N-CO, par un ou deux groupes alkyle, par un ou deux groupes aralkyle et/ou par un ou deux groupes arylcarbamoyle; ou représente un groupe carboxyle, un groupe alcoxycarbonyle, un groupe carboxamido ou un atome d'halogène, et
A représente une chaîne saturée ou insaturée constituée par trois membres comprenant un atome d'azote ou un atome de soufre et deux atomes de carbone ou encore deux atomes d'azote et un atome de carbone, dans laquelle les atomes de carbone peuvent être substitués par un groupe alkyle, par un groupe alcoxy, par un groupe alkylthio, par un groupe hydroxyle, par un groupe aralkyle, par un groupe aryle, par un groupe carboxyle, par un groupe carboxamido, par un groupe alcoxycarbonyle, par un groupe cyano, par un atome d'halogène, par un groupe amino, par un groupe amino qui est substitué par un ou deux radicaux alkyle qui peuvent porter un ou plusieurs radicaux hydroxyle, un ou plusieurs radicaux carboxyle et/ou un ou plusieurs radicaux alcoxycarbonyle, et dans laquelle les atomes d'azote qui ne sont pas liés via une liaison double sont substitués par un atome d'hydrogène, par un groupe alkyle par un groupe alkyle substitué, par un groupe SO₃H, par un groupe PO₃H₂, par un groupe carboxyle ou par un groupe dialkylphosphinyle, ou encore par un groupe aralkyle; ou bien deux substituants de chaînes voisins peuvent former un groupe alkylène qui peut être, quant à lui, substitué ou condensé avec un groupe aryle, ainsi que ses formes tautomères correspondantes et ses sels,
qui réagit par voie enzymatique pour former une quinonediimine colorée qui est déterminée par voie colorimétrique comme mesure pour la quantité de l'analyte.

13. Procédé selon la revendication 12, caractérisé en ce que A forme avec le radical hétérocyclique voisin, un noyau imidazole, un noyau triazole, un noyau benzimidazole, un noyau thiazole ou un noyau dihydroimidazole.

14. Procédé selon la revendication 12 ou 13, caractérisé en ce qu'on met en oeuvre le composé nitroso aromatique en une concentration supérieure à 10⁻³ mole/litre.

15. Procédé selon l'une quelconque des revendications 1 à 14, caractérisé en ce qu'on met en oeuvre, à titre de déshydrogénase dépendant de la PQQ, la glucose-colorant-oxydoréductase pour la détermination du glucose.

16. Agent pour la détermination colorimétrique d'un analyte par oxydation enzymatique de l'analyte, contenant une déshydrogénase dépendant de la PQQ et un composé nitroso aromatique riche en électrons, caractérisé en ce qu'il contient en outre un réactif de détection chromogène de type non oxydant pour des composés imino.

17. Agent selon la revendication 16, caractérisé en ce qu'il contient un composé nitroso répondant à la formule générale I 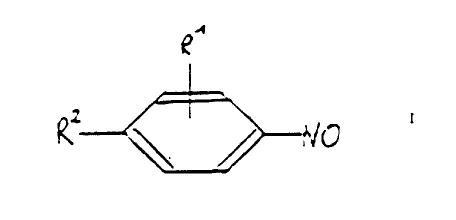 dans laquelle
R¹ représente un atome d'hydrogène, un groupe hydroxyle, un groupe alkyle, un groupe alkyle substitué par un groupe hydroxyle, par un groupe COOH, par un groupe PO₃H₂ ou par un groupe SO₃H, un groupe alcoxy, un groupe alkylthio, un groupe aryloxy, un groupe arylthio, un atome d'halogène ou encore un groupe amino ou un groupe amino qui est substitué par un groupe hydroxyle, par un groupe PO₃H₂, par un groupe dialkylphosphinyle, par un groupe SO₃H ou par un groupe CO₂H, et
R² représente un groupe hydroxyle, un groupe alcoxy, un groupe aryloxy, un groupe arylthio ou un groupe alkylthio, le radical alkyle étant substitué, pour sa part, par un groupe hydroxyle, par un groupe alcoxy, par un groupe amino, par un groupe amino substitué une ou plusieurs fois par un groupe alkyle, par un groupe PO₃H₂, par un groupe SO₃H ou par un groupe CO₂H, comme tels ou sous forme de sels, tels que des sels d'ammonium, de métaux alcalins ou de métaux alcalino-terreux, ou encore représente un groupe amino NR³R⁴,
dans lequel R³ et R⁴ peuvent être identiques ou différents et représentent un atome d'hydrogène, un groupe aryle ou un groupe alkyle qui peut être, quant à lui, substitué par un groupe hydroxyle, par un groupe alcoxy, par un groupe hydroxyalcoxy, par un groupe polyalcoxy, par un groupe polyalcoxy substitué par un groupe hydroxyle, par un groupe PO₃H₂, par un groupe SO₃H ou par un groupe CO₂H, comme tels ou sous forme de sels, ou par un groupe amino ou encore par un groupe amino substitué une ou plusieurs fois par un groupe alkyle, ou
dans lequel R³ et R⁴ peuvent représenter un radical alkylène qui peut être interrompu par un atome d'oxygène, par un atome de soufre ou par un atome d'azote, dans lequel l'atome d'azote est substitué par un radical alkyle, par un radical hydroxyalkyle, par un radical hydroxyalcoxyalkyle, par un radical alcoxyhydroxyalkyle, par un radical alcoxycarbonylalkyle, par un radical dioxanylylalkyle ou par un radical polyalcoxyalkyle qui, quant à lui, peut être substitué respectivement dans la fraction alkyle par un radical hydroxyle, ou
lorsque R¹ se trouve en position ortho par rapport à NR³R⁴, R³ ou R⁴ peut représenter également, de manière conjointe avec R¹, un radical alkylène.

18. Agent selon la revendication 16, caractérisé en ce qu'il contient un composé nitroso répondant à la formule générale II 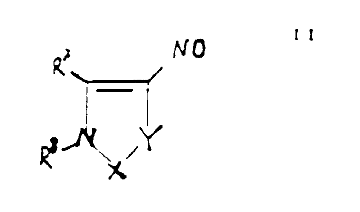 dans laquelle
X-Y représente NR⁵-CO ou N=CR⁶ où
R⁵ représente un atome d'hydrogène, un groupe alkyle, un groupe alkyle substitué par un groupe hydroxyle, par un groupe carboxyle, par un groupe SO₃H, par un groupe PO₃H₂, par un groupe dialkylphosphinyle,
R⁶ représente un atome d'hydrogène, un groupe alkyle, un groupe alcényle, un groupe alcoxy, un groupe alkylthio, un groupe aryle, un groupe aralkyle, qui peuvent être substitués respectivement par un groupe hydroxyle, par un groupe dialkylphosphinyle, par un groupe carboxyle, par un groupe SO₃H, par un groupe PO₃H₂, par un sel d'un de ces radicaux acides et/ou par un groupe alcoxycarbonyle; ou bien représente un groupe amino ou encore un groupe amino qui est substitué par un ou deux radicaux alkyle qui peuvent porter un ou plusieurs radicaux hydroxyle, un ou plusieurs radicaux carboxyle et/ou un ou plusieurs radicaux alcoxycarbonyle, dans laquelle, lorsque le groupe amino est substitué par deux radicaux alkyle, ces radicaux peuvent également être fermés pour former un cycle qui peut être interrompu, outre par l'atome d'azote du groupe amino, également par un atome d'oxygène, par un atome de soufre ou par un atome d'azote supplémentaire; ou représente un groupe amino ou un groupe amino qui est substitué par un ou deux groupes acyle, par un ou deux groupes alcoxy et/ou par un ou deux groupes aralkyle et/ou par un ou deux groupes aralcoxycarbonyle, par un ou deux groupes H₂N-CO, par un ou deux groupes alkyle, par un ou deux groupes arylcarbamoyle; ou représente un atome d'hydrogène, un groupe carboxyle, un groupe alcoxycarbonyle, un groupe carboxamido ou un atome d'halogène, et
R⁷ représente un groupe alkyle, un groupe thioalkyle ou un groupe aralkyle qui peut être substitué par un groupe hydroxyle, par un groupe carboxyle, par un groupe SO₃H ou par un groupe PO₃H₂, ou représente un groupe amino, ou encore un groupe amino qui est substitué par un ou deux groupes alkyle qui peuvent être à nouveau substitués par un groupe hydroxyle, par un groupe carboxyle, par un groupe SO₃H, par un groupe dialkylphosphinyle ou par un groupe PO₃H₂, dans laquelle au moins R⁶ et/ou R⁷ représentent un groupe amino,
R⁸ représente un groupe alkyle ou un groupe aralkyle qui peut être substitué par un groupe hydroxyle, par un groupe carboxyle, par un groupe SO₃H ou par un groupe PO₃H₂,
ou encore dans laquelle
R⁷ et R⁸ représentent une chaîne saturée ou insaturée contenant 3 ou 4 membres constitués par des atomes d'azote ou par des atomes de carbone ou encore par des atomes de carbone et par un ou plusieurs atomes d'azote ou par un ou plusieurs atomes de soufre, dans laquelle les atomes de carbone peuvent être substitués par un groupe alkyle, par un groupe alcoxy, par un groupe alkylthio, par un groupe hydroxyle, par un groupe aralkyle, par un groupe aryle, par un groupe carboxyle, par un groupe carboxamido, par un groupe alcoxycarbonyle, par un groupe cyano, par un atome d'halogène, par un groupe amino, par un groupe amino qui est substitué par un ou deux radicaux alkyle qui peuvent porter un ou plusieurs radicaux hydroxyle, un ou plusieurs radicaux carboxyle et/ou un ou plusieurs radicaux alcoxycarbonyle, et dans laquelle les atomes d'azote qui ne sont pas liés via une liaison double sont substitués par un groupe alkyle ou par un groupe aralkyle qui peuvent être substitués par un groupe hydroxyle, par un groupe SO₃H par un groupe PO₃H₂, par un groupe carboxyle ou par un groupe dialkylphosphinyle, ou bien deux substituants de chaîne voisins forment un groupe alkylène qui peut être, quant à lui, substitué ou condensé avec un groupe aryle,
ainsi que ses formes tautomères correspondantes et ses sels.

19. Agent selon l'une quelconque des revendications 16 à 18, caractérisé en ce qu'il contient, à titre de réactif de détection de type non oxydant pour des composés imino, un réactif de couplage chromogène pour des composés imino ou encore un colorant leuco.

20. Agent pour la détermination colorimétrique d'un analyte par oxydation enzymatique de l'analyte, contenant une déshydrogénase dépendant de la PQQ et un composé nitroso aromatique riche en électrons, caractérisé en ce que le composé nitroso aromatique riche en électrons est un composé de nitrosoaniline répondant à la formule XIII 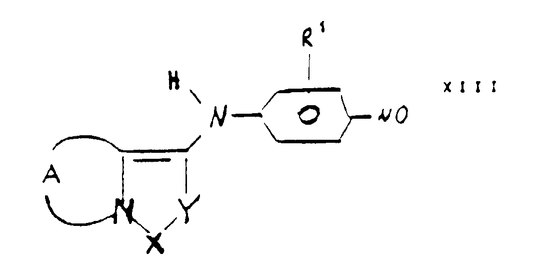 dans laquelle
R¹ représente un atome d'hydrogène, un groupe hydroxyle, un atome d'halogène, un groupe alcoxy ou un groupe alkylthio, un groupe aryloxy, un groupe arylthio, un groupe alkyle, un groupe alkyle substitué par un groupe hydroxyle, par un groupe carboxyle, par un groupe PO₃H₂ ou par un groupe SO₃H, un groupe amino, un groupe amino substitué une ou plusieurs fois par un groupe alkyle qui peut être à nouveau substitué par un groupe hydroxyle, par un groupe PO₃H₂, par un groupe dialkylphosphinyle, par un groupe SO₃H ou par un groupe carboxyle, et
X-Y représente NR⁵-CO ou N=CR⁶ où
R⁵ représente un atome d'hydrogène, un groupe alkyle, un groupe alkyle substitué par un groupe hydroxyle, par un groupe carboxyle, par un groupe SO₃H, par un groupe PO₃H₂ ou par un groupe dialkylphosphinyle,
R⁶ représente un atome d'hydrogène, un groupe alkyle, un groupe alcényle, un groupe alcoxy, un groupe alkylthio, un groupe aryle, un groupe arylthio, un groupe aralkyle qui peuvent être substitués respectivement par un groupe hydroxyle, par un groupe dialkylphosphinyle, par un groupe carboxyle, par un groupe SO₃H, par un groupe PO₃H₂, par un sel d'un de ces radicaux acides et/ou par un groupe alcoxycarbonyle, ou bien représente un groupe amino ou encore un groupe amino qui est substitué par un ou deux radicaux alkyle qui peuvent porter un ou plusieurs radicaux hydroxyle, un ou plusieurs radicaux carboxyle et/ou un ou plusieurs radicaux alcoxycarbonyle, dans laquelle, lorsque le groupe amino est substitué par deux radicaux alkyle, ces radicaux peuvent également être fermés pour former un cycle qui peut être interrompu, outre par l'atome d'azote du groupe amino, également par un atome d'oxygène, par un atome de soufre ou par un atome d'azote supplémentaire; ou encore un groupe amino qui est substitué par un ou deux groupes acyle, par un ou deux groupes alcoxy et/ou par un ou deux groupes aralcoxycarbonyle, par un ou deux groupes H₂N-CO, par un ou deux groupes alkyle, par un ou deux groupes aralkyle et/ou par un ou deux groupes arylcarbamoyle; ou représente un groupe carboxyle, un groupe alcoxycarbonyle, un groupe carboxamido ou un atome d'halogène, et
A représente une chaîne saturée ou insaturée constituée par trois membres comprenant un atome d'azote ou un atome de soufre et deux atomes de carbone ou encore deux atomes d'azote et un atome de carbone, dans laquelle les atomes de carbone peuvent être substitués par un groupe alkyle, par un groupe alcoxy, par un groupe alkylthio, par un groupe hydroxyle, par un groupe aralkyle, par un groupe aryle, par un groupe carboxyle, par un groupe carboxamido, par un groupe alcoxycarbonyle, par un groupe cyano, par un atome d'halogène, par un groupe amino, par un groupe amino qui est substitué par un ou deux radicaux alkyle qui peuvent porter un ou plusieurs radicaux hydroxyle, un ou plusieurs radicaux carboxyle et/ou un ou plusieurs radicaux alcoxycarbonyle, et dans laquelle les atomes d'azote qui ne sont pas liés via une liaison double sont substitués par un atome d'hydrogène, par un groupe alkyle, par un groupe alkyle substitué par un groupe SO₃H, par un groupe PO₃H₂, par un groupe carboxyle ou par un groupe dialkylphosphinyle, ou encore par un groupe aralkyle, ou bien deux substituants de chaîne voisins peuvent former un groupe alkylène qui peut être, quant à lui, substitué ou condensé avec un groupe aryle, ainsi que ses formes tautomères correspondantes et ses sels,

21. Agent selon la revendication 20, caractérisé en ce que A forme avec le radical hétérocyclique voisin, un noyau imidazole, un noyau triazole, un noyau benzimidazole, un noyau thiazole ou un noyau dihydroimidazole.

22. Support d'essai contenant un agent selon l'une quelconque des revendications 16 à 21.

23. Composés de nitrosoaniline répondant à la formule XIII 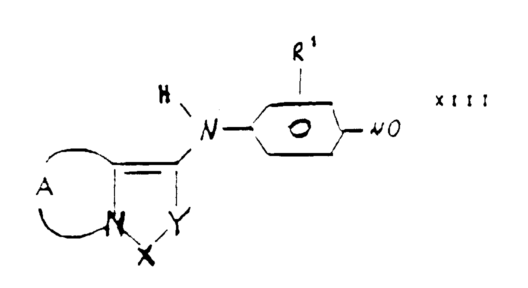 dans laquelle
R¹ représente un atome d'hydrogène, un groupe hydroxyle, un atome d'halogène, un groupe alcoxy ou un groupe arylthio, un groupe alkyle, un groupe alkyle substitué par un groupe hydroxyle, par un groupe carboxyle, par un groupe PO₃H₂, par un groupe dialkylphosphinyle ou par un groupe SO₃H, un groupe amino, un groupe amino substitué une ou plusieurs fois par un groupe alkyle qui peut être à nouveau substitué par un groupe hydroxyle, par un groupe PO₃H₂, par un groupe SO₃H ou par un groupe carboxyle, et
X-Y représente NR⁵-CO ou N=CR⁶ où
R⁵ représente un atome d'hydrogène, un groupe alkyle, un groupe alkyle substitué par un groupe hydroxyle, par un groupe carboxyle, par un groupe SO₃H, par un groupe PO₃H₂, par un groupe dialkylphosphinyle,
R⁶ représente un atome d'hydrogène, un groupe alkyle, un groupe alcényle, un groupe alcoxy, un groupe alkylthio, un groupe aryle, un groupe arylthio, un groupe aralkyle qui peuvent être substitués respectivement par un groupe hydroxyle, par un groupe dialkylphosphinyle, par un groupe carboxyle, par un groupe SO₃H, par un groupe PO₃H₂, par un sel d'un de ces radicaux acides et/ou par un groupe alcoxycarbonyle; ou bien représente un groupe amino ou encore un groupe amino qui est substitué par un ou deux radicaux alkyle qui peuvent porter un ou plusieurs radicaux hydroxyle, un ou plusieurs radicaux carboxyle et/ou un ou plusieurs radicaux alcoxycarbonyle, dans laquelle, lorsque le groupe amino est substitué par deux radicaux alkyle, ces radicaux peuvent également être fermés pour former un cycle qui peut être interrompu, outre par l'atome d'azote du groupe amino, également par un atome d'oxygène, par un atome de soufre ou par un atome d'azote supplémentaire; ou encore un groupe amino qui est substitué par un ou deux groupes acyle, par un ou deux groupes alcoxy et/ou par un ou deux groupes aralcoxycarbonyle, par un ou deux groupes H₂N-CO, par un ou deux groupes alkyle, par un ou deux groupes aralkyle et/ou par un ou deux groupes arylcarbamoyle; ou représente un atome d'hydrogène, un groupe carboxyle, un groupe alcoxycarbonyle, un groupe carboxamido ou un atome d'halogène, et
A représente une chaîne saturée ou insaturée constituée par trois membres comprenant un atome d'azote ou un atome de soufre et deux atomes de carbone ou encore deux atomes d'azote et un atome de carbone, dans laquelle les atomes de carbone peuvent être substitués par un groupe alkyle, par un groupe alcoxy, par un groupe alkylthio, par un groupe hydroxyle, par un groupe aralkyle, par un groupe aryle, par un groupe carboxyle, par un groupe carboxamido, par un groupe alcoxycarbonyle, par un groupe cyano, par un atome d'halogène, par un groupe amino, par un groupe amino qui est substitué par un ou deux radicaux alkyle qui peuvent porter un ou plusieurs radicaux hydroxyle, un ou plusieurs radicaux carboxyle et/ou un ou plusieurs radicaux alcoxycarbonyle, et dans laquelle les atomes d'azote qui ne sont pas liés via une liaison double sont substitués par un atome d'hydrogène, par un groupe alkyle, par un groupe alkyle substitué, par un groupe SO₃H, par un groupe PO₃H₂, par un groupe carboxyle, par un groupe dialkylphosphinyle ou par un groupe aralkyle; ou bien deux substituants de chaîne voisins peuvent former un groupe alkylène qui peut être, quant à lui, substitué ou condensé avec un groupe aryle, ainsi que leurs formes tautomères correspondantes et leurs sels,

24. Composés de nitrosoaniline selon la revendication 23, caractérisés en ce que A forme avec le radical hétérocyclique voisin, un noyau imidazole, un noyau triazole, un noyau benzimidazole, un noyau thiazole ou un noyau dihydroimidazole.

25. Utilisation des composés de nitrosoaniline selon l'une quelconque des revendications 23 ou 24 pour la détermination enzymatique colorimétrique d'un analyte.
